# EUROPEAN PATENT SPECIFICATION

(11) **EP 0 537 160 B1**
(45) Date of publication and mention of the grant of the patent: **21.08.2002**
(21) Application number: 91908364.2
(22) Date of filing: 15.04.1991
(51) Int. Cl.: C12N 15/12, C07K 14/00, C12N 1/19, A61K 38/00, C12P 21/02

(54) **EXPRESSION OF RECOMBINANT HEMOGLOBIN IN YEAST**
EXPRESSION VON REKOMBINANTEM HÄMOGLOBIN IN HEFE
EXPRESSION D'HEMOGLOBINE RECOMBINANTE DANS DE LA LEVURE

(30) Priority: 16.04.1990 US 509918; 14.11.1990 US 614359; 12.04.1991 US 684611
(43) Date of publication of application: 21.04.1993
(60) Divisional application: 01100881.0
(73) Proprietor: APEX BIOSCIENCE, INC., Detroit, Michigan 48207 (US)
(72) Inventor: DE ANGELO, Joseph, Hamtramck, MI 48212 (US); MOTWANI, Nalini, M., Troy, MI 48084 (US); BAJWA, Wajeeh, Canton, MI 48187 (US)
(74) Representative: Hirsch, Marc-Roger
(86) International application number: US9102568
(87) International publication number: WO91016349

(56) References cited:
- EP-A- 0 185 404
- EP-A- 0 402 300
- WO-A-88/07550
- WO-A-88/09179
- WO-A-91/13158
- US-A- 4 840 896
- NATURE vol. 283, 28 February 1980, LONDON GB pages 835 - 840 JEAN D. BEGGS ET AL. 'Abnormal expression of chromosomal rabbit beta-globin gene in Saccharomyces cerevisiae'
- BIOTECHNOLOGY vol. 9, no. 1, January 1991, NEW YORK US pages 57 - 61 MICHAEL WAGENBACH ET AL. 'Synthesis of wild type and mutant human hemoglobins in Saccharomyces cerevisiae'
- HEMOGLOBIN vol. 11, no. 5, 1987, pages 435 - 452 JUNIUS G. ADAMS ET AL. 'Hb Mississippi Äbeta44(CD3)Ser-ArgÜ: a new variant with anomalous properties'
- NATURE NEW BIOLOGY vol. 230, 28 April 1971, pages 261 - 264 J. GREER ET AL. 'Three dimensional structure of Haemoglobin Rainier'
- M. INOUYE, "Experimental Manipulation of Gene Expression", Published 1983, by ACADEMIC PRESS (N.Y.), pages 83-117.
- DNA, Volume 1, No. 4, issued 1982, M.M. COHEN-SOLAL et al., "Cloning and Nucleotide Sequence Analysis of Human Embryonic Dzeta-Globin cDNA", pages 355-363.
- CELL, Volume 21, issued October 1980, F.E. BARALLE et al., "The Primary Structure of the Human Epsilon-Globin Gene", pages 621-626.
- CELL, Volume 21, issued October 1980, J.L. SLIGHTOM et al., "Human Fetal aGamma- and aGamma-Globin Genes: Complete Nucleotide Sequences Suggest that DNA can be Exchanged Between These Duplicated Genes", pages 627-638.
- CELL, Volume 21, issued October 1980, R.A. SPRITZ et al., "Complete Nucleotide Sequence of the Human Delta-Globin Gene", pages 639-646.
- CELL, Volume 21, issued October 1980, R.M. LAWN et al., "The Nucleotide Sequence of the Human Beta-Globin Gene", pages 647-651.
- CELL, Volume 21, issued October 1980, A. EFSTRATIADIS et al., "The Structure and Evolution of the Human Beta-Globin Gene Family", pages 653-668.
- PROC. NATL. ACAD. SCI. U.S.A., Volume 82, issued November 1985, K. NAGAI et al., "Oxygen Binding Properties of Human Mutant Hemoglobins Synthesized in Escherichia Coli", pages 7252-7255.
- PROC. NATL. ACAD. SCI. U.S.A., Volume 84, issued December 1987, B.A. SPRINGER et al., "High-Level Expression of Sperm Whale Myoglobin in Escherichia Coli", pages 8961-8965.

## Description

### 1. FIELD OF THE INVENTION

The invention is directed to an expression vector which specifically comprises DNA sequences encoding at least one globin chain or heme-binding fragment thereof operably linked to a yeast promoter. The invention is also directed to methods for producing a globin chain or heme binding fragment thereof in yeast and methods for producing hemoglobin in yeast. Hemoglobin produced by methods of the present invention may be used in applications requiring physiological oxygen carriers such as in blood substitute solutions, or as in a plasma expander. The invention also provides variant hemoglobin compositions.

### 2. BACKGROUND OF THE INVENTION

### 2.1. USE OF HEMOGLOBIN AS A BLOOD SUBSTITUTE

Transfusion of a patient with donated blood has a number of disadvantages. Firstly, there may be a shortage of a patient's blood type. Secondly, there is a danger that the donated blood may be contaminated with infectious agents such as hepatitis viruses, cytomegalovirus, Epstein-Barr virus, serum parvoviruses, syphilis, malaria, filariasis, trypanosomiasis, babsiosis, pathogenic bacteria, and HIV (Bove, 1986, Progr. Hematol. 14:123-145). Thirdly, donated blood has a limited shelf life.

An alternative to transfusion involves the use of a blood substitute. A blood substitute is an oxygen carrying solution that also provides the oncotic pressure necessary to maintain blood volume. Two types of substitutes have recently been studied, fluorocarbon emulsions and hemoglobin solutions.

Hemoglobin as it exists within the red blood cell is composed of two alpha-like globin chains and two beta-like globin chains, each with a heme residue. One alpha-like globin chain and one beta-like globin chain combine to form a dimer which is very stable. Alpha-like and beta-like globin genes are each a family of related globin genes which are expressed at different stages of development and regulated by oxygen tension, pH, and the development from embryo to fetus to newborn. Two dimers then line up in antiparallel fashion to form tetramers. The binding of dimers to form the tetramers is not as strong as in the case of monomers binding to associate into dimers. The tetramers, therefore, have a tendency to fall apart to form dimers and there is always an equilibrium between tetramers, dimers, and monomers. At high concentrations of globin, the predominant form is the tetramer; with dilution, the dimer becomes the predominant form. This equilibrium is also affected by solvent, salts, pH and other factors as the forces binding the monomers together are primarily electrostatic.

The alpha-like globin genes are clustered together on chromosome 16 and include genes encoding the embryonic zeta globin chain and the adult alpha globin chain, present in both the fetus and newborn. The beta-like globin genes reside on chromosome 11 and include genes encoding the embryonic epsilon-globin chain, the fetal gamma-globin chain, and the adult delta-globin and adult beta-globin chains. Two types of gamma globin chains have been identified, ^{G}gamma and ^{A}gamma, which differ by the presence of a single glycine or alanine residue, respectively, at amino acid 135 (Schroeder et al., 1968, Proc. Natl. Acad. Sci. U.S.A. 60: 537-544). The gamma chain has been found to contain a polymorphic site at position 75, which also can be occupied either by isoleucine or threonine. A variety of hemoglobins may be formed (reviewed in Kutlar et al., 1989, Hemoglobin 13:671-683 and Honig and Adams, Human Hemoglobin Genetics, Springer Verlag, New York pp. 29-33). Examples include HbA (alpha₂beta₂), HbA₂ (alpha₂delta₂), HbF (alpha₂gamma₂), HbBarts (gamma₄), HbH (beta₄), and Hb PortlandI (zeta₂gamma₂), Hb Portland II (zeta₂beta₂), Hb Portland III (zeta₂delta₂) Hb Gower I (zeta₂epsilon₂), and Hb Gower II (alpha₂epsilon₂).

There are obstacles however to using native hemoglobin as a blood substitute.. Firstly, large dosages are required (Walder, 1988, Biotech '88, San Francisco, Nov. 14-16, 1988). A single unit (450 ml) of a 10% hemoglobin solution contains 45 g of protein. It is estimated that at least 12 million units of blood are used in the U.S. per year. Therefore the production of 450,000 kg of hemoglobin per year would be required. Secondly, it is important to obtain hemoglobin that is free from infectious agents and toxic substances. Thirdly, as mentioned, although hemoglobin is normally a tetramer of 64,000 molecular weight, it can dissociate to form alpha-beta dimers. The dimers are rapidly cleared by the kidneys and the residence time is much too short for cell-free hemoglobin to be useful as a blood substitute. Fourthly, cell-free hemoglobin has too high an oxygen affinity to effectively release oxygen to the tissues due to the absence of 2,3-diphosphoglycerate (2,3-DPG). Efforts to restore 2,3-DPG have been unsuccessful since 2,3-DPG is rapidly eliminated from the circulation.

Several approaches have been taken to circumvent these difficulties. These include the expression of hemoglobin via recombinant DNA systems, chemical modification of hemoglobin, and the production of hemoglobin variants.

### 2.1.1. EXPRESSION OF RECOMBINANT HEMOGLOBIN

Human embryonic zeta globin (Cohen-Sohal, 1982, DNA 1:355-363), human embryonic epsilon globin (Baralle et al., 1980, Cell 21:621-630), human fetal gamma globin (Slightom et al., 1980, Cell 21:627-630), human adult delta globin (Spritz et al., 1980, Cell 21:639-645), human adult alpha-globin genomic DNA (Liebhaber et al., 1980, Proc. Natl. Acad. Sci. U.S.A. 77:7054-7058) and human adult beta-globin cDNA (Marotta et al., 1977, J. Biol. Chem. 252: 5040-5053) have been cloned and sequenced.

Both human adult alpha- and beta-globins have been expressed in bacterial systems. Nagai et al. (1986, Proc. Natl. Acad. Sci. U.S.A. 82:7252-7255 and 1984, Nature (London) 309:810-812) expressed adult beta-globin in E. coli as a hybrid protein consisting of the 31 amino-terminal residues of the lambda cII protein, an Ile-Glu-Gly-Arg linker, and the complete human adult beta-globin chain. The hybrid was cleaved at the single arginine with blood coagulation factor Xa, resulting in the liberation of the beta-globin chain. PCT Application No. PCT/US88/01534 (Publication No. WO 88/091799, published December 1, 1988) discloses the expression of a DNA sequence encoding the adult alpha-globin gene and the N-terminal 20 amino acid sequence of beta-globin in which the alpha- and beta-globin sequences are separated by spacer DNA encoding a Factor Xa cleavage site.

Efforts have also been made to secrete beta-globin into the periplasm of E. coli, in which the beta-globin gene was inserted behind an OmpA secretion signal sequence (Brinigar et al., 1988, Symposium on Oxygen Binding Heme Proteins-Structure, Dynamics, Function and Genetics). However, it was found that though the fusion was correctly processed, the beta-globin was not secreted.

Nagai et al. (1986, Proc. Natl. Acad. Sci. U.S.A. 82:7252-7255) have also reported the reconstitution of adult beta-globin expressed in E. coli and adult alpha-globin obtained by conventional sources along with a heme source to obtain hemoglobin. However, it would not be possible in E. coli to produce recombinant hemoglobin that has the same functional properties as normal human hemoglobin because of E. coli's inability to remove the N-formyl-methionine by post-translational processing. The amino terminus is known to be critical in determining the oxygen binding properties of human hemoglobin as has been shown in the case of Hb Raleigh (Moo-Penn, et al., 1977, Biochemistry, 16:4872-4879). Furthermore, the hemoglobin. produced in bacteria can contain E. coli endotoxins.

Attempts have also been made to express hemoglobin in yeast. Reports from two groups indicate that yeast cells were unable to excise the intervening sequences in both alpha- and beta-globin precursor mRNA (Langford et al., 1983, Proc. Natl. Acad. Sci. U.S.A. 80:1496-1500 and Beggs et al., 1980,-Nature (London) 283:835-840). An attempt was also made to secrete beta-globin in Streptomyces by constructing a plasmid having a GalK-FX-beta-globin sequence behind a beta-galactosidase secretion signal sequence (Brinigar et al., 1988, Symposium on Oxygen Binding Heme Proteins Structure, Dynamics, Function and Genetics). GalK-FX-beta-globin however remained within the cells under conditions where galactokinase was secreted.

Recently the construction of two yeast plasmids containing adult beta-globin was-reported (Brinigar et al., 1988, Symposium on Oxygen Binding Heme Proteins Structure, Dynamics, Function and Genetics). One contained a constitutive promoter, glyceraldehyde-3-phosphate dehydrogenase and ubiquitin fused directly to adult beta-globin, and the other contained metallothionein, an inducible promoter, and ubiquitin fused directly to beta-globin. It was reported that in both instances, both intracellular soluble and intracellular insoluble adult beta-globin was obtained. No further details were disclosed regarding the construction of the plasmids or the quantity of adult beta-globin obtained.

The expression of globin in mammalian cells has also been reported. The construction of recombinant herpes simplex virus, adenovirus, SV-40, and retrovirus vectors containing a DNA sequence encoding the human adult beta-globin gene has been disclosed (Dobson et al., 1989, J. Virol. 63:3844-3851; Yanagi et al., 1989, Gene 76:19-26; Miller et al., 1988, J. Virol. 62:4337-4345; and Karlsson et al., 1985, EMBO J 5: 2377-2386). The expression of human adult alpha-globin genes in Chinese hamster ovary cells which involved introducing a recombinant DNA molecule containing the normal human adult alpha-globin gene and a hybrid gene containing the 5' promoter-regulator region of the mouse metallothionein gene linked to a SV2-cDNA dihydrofolate reductase gene has also been disclosed (Lau et al., 1984, Mol. Cell Biol. 4:1469-1475). However, the expression of the globin genes was found to be rather low due to low efficiency of gene transfer.

### 2.1.2. CHEMICAL MODIFICATION OF HEMOGLOBIN

One approach that has been taken to circumvent the problem of dissociation of the hemoglobin tetramer to a dimer has been to chemically modify the hemoglobin by either intramolecular or intermolecular crosslinking. Examples of such modification include crosslinking with polyalkylene glycol (Iwashita, U.S. Patent NO. 4,412, 989 and 4,301,144), with polyalkylene oxide (Iwasake, U.S. Patent No. 4,670,417); with a polysaccharide (Nicolau, U.S. Patent Nos. 4,321,259 and 4,473,563); with inositol phosphate (Wong, U.S. Patent Nos. 4,710,488 and 4,650,786); with a bifunctional crosslinking agent (Morris et al., U.S. Patent No. 4,061, 736); with insulin (Ajisaka, U.S. Patent No. 4,377,512); and with a crosslinking agent so that the hemoglobin composition is intramolecularly crosslinked between lys 99 alpha₁ and lys 99 alpha₂ (Walder, U.S. Patent No. 4,598,064).

Hemoglobin has also been chemically modified to decrease the oxygen affinity of isolated hemoglobin. One approach has involved polymerization with pyridoxal phosphate (Sehgal et al., 1984, Surgery, 95:433-438). Another approach has involved the use of reagents that mimic 2,3-DPG (Bucci et al., U.S. Patent No. 4,584,130). Although these compounds do lower the oxygen affinity of hemoglobin, the affinity is still relatively high.

### 2.1.3. HEMOGLOBIN VARIANTS

Categories of naturally occuring hemoglobin variants include: variants which autopolymerize; variants which prevent the dissociation of the tetramer, variants with lowered intrinsic oxygen affinity, and variants that are stable in alkali. Examples of autopolymerizing hemoglobin variants include Hb Porto Alegre, Hb Mississippi, and Hb Ta-Li.

Hb Porto Alegre is a beta chain variant first reported by Tondo et al. (1974, Biochem. Biophys. Acta 342: 15-20; 1963, Am. J. Human Genet. 15:265-279). The beta-9 serine is replaced by cysteine which is able to form disulfide bonds with other cysteine residues. Through these crosslinks, Hb Porto Alegre forms poly-tetramers. These polymers however do not form in the blood of Hb Porto Alegre carriers. It has been shown that Hb Porto Alegre carriers have a two-fold elevated level of glutathione and three-fold elevated level of glutathione reductase which prevents the polymerization of the Hb Porto Alegre within the red blood cells (Tondo et al., 1982, Biochem. Biophys. Res. Commun. 105:1381-1388). The exact structure of these polymers is not known.

Hb Mississippi is a recently isolated polymerizing variant of hemoglobin. The new variant was first reported by Adams et al. (1987, Hemoglobin 11:435-452). The beta-44 serine is replaced by cysteine in this variant resulting in inter-tetramer disulfide bonds. This variant is believed to form polymers with as many as ten tetramers.

Hb Ta-Li is another known polymerizing beta variant. The beta-83 glycine is replaced by cysteine. This variant was first reported in 1971 (Blackwell et al., 1971, Biochem. Biophys. Acta 243:467-474). This variant also forms inter-tetramer crosslinks.

Another group of variants include those with nondissociating tetramers. One example is Hb Rainier, a well characterized variant of the beta chain (Greer and Perutz, 1971, Nature New Biology 230:261 and Statoyannopoulos et al., 1968, Science 159:741). The beta-145 tyrosine is replaced by cysteine. This cysteine is able to form disulfide crosslinks with. beta-93 cysteine which is present in natural beta-globin. This disulfide bond is intra-tetramer, i.e. it is formed between the two beta subunits within a tetramer. This covalent disulfide bond stabilizes the tetramer form and prevents the dissociation of the tetramer into its constituent dimers. Hb Rainier has also been found to have a high affinity for oxygen, a reduced Hill coefficient, and only half the alkaline Bohr effect of normal hemoglobin.

Another group of variants includes those that are stable in alkali. Hb Motown/Hacettepe is a variant reported to be stable in alkali (Gibb and Rucknagel, 1981, Clinical Research 29:795A and Altay et al., 1976, Biochem. Biophys. Acta 434:1-3). The beta-127 glutamine is replaced by glutamic acid in this variant. This portion of the beta chain is involved in the alpha₁beta₁ interface between the monomers forming a dimer. The substituted glutamic acid forms an ionic bond with alpha-31 arginine. This is a stronger bond than that formed between the alpha-31 arginine and the normal beta-127 qlutamine and is believed to be responsible for the increased stability of Hb Motown/Hacettepe. HbF (fetal hemoglobin) and bovine hemoglobin are also in this group of alkali stable variants (Perutz, 1974, Nature 247:341).

There are also over 30 naturally occurring hemoglobin variants which exhibit lowered oxygen affinity. Several examples of such variants are disclosed in PCT Application No. PCT/US88/01534 (Publication No. WO 88/091799, published December 1, 1988), Bonaventura and Bonaventura, 1980, In: Abnormal Human Hemoglobins and Red Cell Enzymes, Huisman, T., Ed., Marcel Dekker, NY, Hemoglobin 4 (3 & 4):275-289 and Bonaventura and Bonaventura, 1978,in Biochemical and Clinical Aspects of Hemoglobin Abnormalities, Caughey, W.S., Ed., Academic Press, NY, pp. 647-663. There seems in a group of these low oxygen affinity mutants to be a generalizable relationship between the intrinsic oxygen affinity of an alpha₂beta₂ hemoglobin and the cluster of positively charged residues that are involved in the binding of 2,3-DPG and other anionic allosteric cofactors of hemoglobin function (Bonaventura and Bonaventura, 1980, Amer. Zool. 20:131-138).

One example of a low oxygen affinity mutant is Hb Chico where the beta-66 lysine is replaced by threonine (Shih et al., 1987, Hemoglobin 11: 453-464). The P50 of Hb Chico's red blood cells is 38 mm Hg compared with normal red blood cell controls with P50 of 27 mm Hg. All other properties, i.e. Hill coefficient and alkaline Bohr effect are normal.

Another low oxygen affinity variant is Hb Raleigh, a beta chain variant in which beta-1 valine is replaced by alanine (Moo-Penn et al., 1977, Biochemistry 16:4873). A post-translational modification of the amino-terminal alanine results in the formation of acetylalanine. Because the positively charged amino group of valine is involved in 2,3-DPG binding, the acetvlation results in a decreased charge cluster in the DPG binding site. This charge difference acts to decrease the oxygen affinity of Hb Raleigh and to lessen the effect of DPG which lowers the oxygen affinity of normal HbA. The Hill coefficient (cooperativity) and alkaline Bohr effect (pH dependent oxygen binding) are unaffected by this change.

Hb Titusville (alpha-94 aspartate to asparagine) is one of a group of low affinity hemoglobin variants with altered alpha₁beta₂ contacts (Schneider et al., 1975, Biochem. Biophys. Acta 400:365). The alpha₁beta₂ interface is stabilized by two different sets of hydrogen bonds between the alpha and beta subunits. One set stabilizes the T-structure which is the low-affinity form and the other stabilizes the R-state which is the high affinity form. It is the shifting back and forth between these two sets of bonds and alternating between the T- and R-states which is responsible for the positive cooperativity. The deoxyhemoglobin is primarily in the T-state. For hemoglobin with one oxygen bound, the amount of R-state molecules increases and therefore binds oxygen with a higher affinity. In hemoglobin with two oxygens bound, there is an even higher proportion of R state molecules. In Hb Titusville, the R-state bonds are disrupted. The alpha-94 aspartate would normally form a non-covalent bond with beta-102 asparagine. Because this bond is disrupted, the equilibrium is pushed in the direction of the T-state and Hb Titusville's oxygen affinity is very low.

Hb Beth Israel is another variant affecting the alpha₁beta₂ interface which destabilizes the high oxygen affinity R-state (Nagel et al., 1976, New Eng. J. Med. 295:125-130). The beta-102 asparagine is replaced by serine. The whole blood of an Hb Beth Israel patient has a P50 of 88 mm Hg as compared with the normal value of 27. The Hill coefficient is biphasic with a value of 1.0 at the high end and 1.0 at the low end. The Bohr effect is normal. A hemolysate of Hb Beth Israel has a P50 of 17 mm Hg and a Hill coefficient of 1.65 at the bottom and 1.29 at the top of the curve as compared to a P50 of 5.6 and a Hill coefficient of 2.72 for normal hemoglobin.

Another example of a low affinity human hemoglobin mutant is Hb Kansas (PCT Application No. PCT/US88/01534, Publication No. WO 88/091799, published December 1, 1988 and Bonaventura and Riggs, 1968, J. Biol. Chem. 243: 980-991). The beta-102 asparagine is replaced by threonine. It has been shown that isolated Hb Kansas' heme-containing beta-globin chains have lowered oxygen affinity (Riggs and Gibson, 1973, Proc. Natl. Acad. Sci. U.S.A. 70:1718-1720).

### 2.2. EXPRESSION OF HETEROLOGOUS DNA IN YEAST

With the advent of recombinant DNA technology; efforts have been made to express heterologous DNA in a variety of prokaryotic and eukaryotic systems. One such system is yeast.

Yeast has a number of advantages over bacteria and other eukaryotes as a system for the production of polypeptides or proteins encoded by recombinant DNA. Yeast has been used in large scale fermentations for centuries, so the technology for fermenting yeast is well known and a number of yeast hosts are commercially available. Additionally, yeast can be grown to higher densities than bacteria and many other types of eukaryotic cells, and is readily adaptable to continuous fermentation processing. Since yeast is a eukaryotic organism, yeast may be capable of glycosylating expression products, may exhibit the same codon preferences as higher organisms, and may remove the amino terminal methionine during post-translational processing.

A number of heterologous proteins have been expressed in yeast. Examples include interferon (Hitzeman and Leung, U.S. Patent No. 4,775,622, issued October 4, 1988; Hitzeman et al., Canadian Patent No. 1,205,026, issued May 27, 1986; Hitzeman et al., 1981, Nature (London) 293: 717); platelet derived growth factor (Murray et al., U.S. Patent No. 4,801,542, issued January 31, 1989); glucagon (Norris et al., U.S. Patent No. 4,826,763, issued May 2, 1989).

Heterologous proteins expressed in yeast have been linked to a wide variety of promoters. Examples include operably linking heterologous proteins to SV40 and RSV promoters (Gelfand et al., U.S. Patent No. 4,8710,013, issued September 26, 1989). Additionally, DNA sequences encoding heterologous proteins have been linked to yeast promoters, which are inducible. European Patent Application Publication No. 132, 309, published January 30, 1985 discloses the construction of a plasmid containing the yeast galactose-induced promoters for galactokinase (GAL1) and UDP-galactose epimerase (GAL10), hereinafter referred to as the GAL1-10 promoter, which is bidirectional. Another example of a bidirectional yeast promoter is the YPT1/TUB2 intergene sequence which contains overlapping binding sites for the transcription factor BAF1 (Halfter et al., 1989, EMBO J. 8:3029-3037). Broach et al. (Manipulation of Gene Expression, ed. Inouye, 1983) disclose a plasmid containing a GAL10 upstream activator sequence which promotes transcription and an alcohol dehydrogenase transcription (ADH1) terminator sequence to prevent run through transcription derived from YEp51. Kingsman et al., U.S. Patent No. 4,615,974, issued October 7, 1986 disclose the use of the 5' regions of the yeast phosphoglycerate kinase genes as a promoter of the transcription of interferon. Hitzeman et al., Canadian Patent No. 1,205,026, issued May 27, 1986 disclose the use of the 5' flanking sequence of the ADH1 structural gene to promote the transcription of interferon. Burke et al., U.S. Patent No. 4,876,197, issued October 24, 1989 disclose a DNA construct comprising a first transcription regulatory region obtained from the yeast lcohol dehydrogenase II gene (ADH2), the regulatory region of acid phosphatase (PHO5) or the regions regulated by GAL4, which provides for inducible transcriptional regulation and a second transcriptional initiation region from the yeast glyceraldehyde-3-phosphate dehydrogenase gene (TDH3) and a terminator region.

### 3. SUMMARY OF THE INVENTION

The invention thus provides a composition comprising a variant hemoglobin, in which the variant hemoglobin comprises:
(a) a variant globin chain or heme-binding fragment thereof expressed in a yeast, which is substantially homologous to:
   i) a mammalian fetal gamma-globin chain or heme-binding fragment thereof,
(b) an alpha-like globin chain or heme-binding fragment thereof expressed in a yeast, wherein the alpha-like globin chain is a mammalian embryonic zeta-globin chain; and
(c) heme; and wherein
   the variant hemoglobin
(d) has the ability to bind to oxygen at a low oxygen affinity;
(e) has the ability to autopolymerize;
(f) is stable in alkali; or
(g) does not dissociate under physiological conditions;
   and which composition
   is free of erythrocyte membrane components and E. coli endotoxins,
wherein the substantially homologous variant globin chain or heme-binding fragment thereof is a variant in which a cDNA sequence encoding the variant globin chain or heme-binding fragment thereof has the ability to hybridize to a cDNA sequence encoding the mammalian fetal gamma-globin chain or heme-binding fragment thereof, under stringent conditions comprising 0.1X SSC and a temperature of about 65°C.

The invention thus also provides a composition comprising a variant hemoglobin, in which the variant hemoglobin comprises:
(a) a variant globin chain or heme-binding fragment thereof expressed in a yeast, which is substantially homologous to:
   i) a mammalian embryonic zeta-globin chain or heme-binding fragment thereof,
(b) a beta-like globin chain or heme-binding fragment thereof expressed in a yeast, wherein the beta-like globin chain is a mammalian fetal gamma-globin chain; and
(c) heme; and wherein
   the variant hemoglobin
(d) has the ability to bind to oxygen at a low oxygen affinity; or
(e) is stable in alkali;
   and which composition is free of erythrocyte membrane components and E. coli endotoxins,
wherein the substantially homologous variant globin chain or heme-binding fragment thereof is a variant in which a cDNA sequence encoding the variant globin chain or heme-binding fragment thereof has the ability to hybridize, to a cDNA sequence encoding the mammalian embryonic zeta-globin chain or heme-binding fragment thereof under stringent conditions comprising 0.1X SSC and a temperature of about 65°C.

Hemoglobin consisting essentially of a zeta and gamma globin chain may be obtained by mixing zeta globin and gamma globin or variants thereof with a source of heme. Hemoglobin consisting essentially of zeta and gamma globin chain or variant thereof may be used in applications requiring physiological oxygen carriers such as in blood substitute solutions, or as in a plasma expander.

In the following, alpha-like refers to zeta while beta-like refers to gamma.

The globin chains or heme-binding fragments thereof of the present invention may be obtained by expressing a recombinant DNA vector comprising a DNA sequence encoding at least one globin chain or heme binding fragment thereof in yeast. The invention is therefore directed to a recombinant DNA vector capable of expressing a globin chain or heme-binding fragment thereof in a yeast cell comprising:
(a) a DNA sequence encoding a globin chain or heme-binding fragment thereof;
(b) a yeast transcriptional promoter which promotes the transcription of the DNA sequence encoding the globin chain or heme-binding fragment thereof;
(c) a DNA sequence encoding a yeast selectable marker or functionally active portion thereof; and
(d) a yeast replication origin.

The invention also provides a recombinant DNA vector capable of expressing in a yeast cell a globin chain I and globin chain II, which recombinant DNA vector comprises:
(a) a cDNA sequence I encoding the globin chain I;
(b) a yeast transcriptional promoter upstream of the sequence I which regulates the transcription of the sequence I;
(c) a cDNA sequence encoding a yeast selectable marker or functionally active portion thereof; and
(d) a yeast replication origin or functionally active portion thereof;
wherein the globin chain I is
(i) a mammalian embryonic zeta-globin chain or heme-binding fragment thereof; or a variant zeta globin chain or heme-binding fragment thereof which is substantially homologous to said zeta globin chain or heme-binding fragment thereof, wherein the substantially homologous variant zeta globin chain or heme-binding fragment thereof is a variant in which a DNA sequence encoding the variant zeta globin chain or heme-binding fragment thereof has the ability to hybridize to a DNA sequence encoding said zeta globin chain or heme-binding fragment thereof under stringent conditions comprising 0.1X SSC and a temperature of about 65°C; and
wherein the globin chain II is
(ii) mammalian fetal gamma-globin chain, or heme-binding fragment thereof, or a variant gamma globin chain or heme-binding fragment thereof which is substantially homologous to said gamma globin chain or heme-binding fragment thereof, wherein the substantially homologous variant gamma globin chain or heme-binding fragment thereof is a variant in which a DNA sequence encoding the variant gamma globin chain or heme-binding fragment thereof has the ability to hybridize to a DNA sequence encoding said gamma globin chain or heme-binding fragment thereof under stringent conditions comprising 0.1X SSC and a temperature of about 65°C.

The invention also provides a yeast cell containing the recombinant DNA vector of the invention.

A yeast transcriptional promoter may be selected from the group including but not limited to a yeast inducible transcriptional promoter, a yeast constitutive transcriptional promoter, a yeast hybrid promoter, and a yeast bidirectional promoter.

The recombinant DNA vector may be capable of expressing zeta globin chain and gamma globin chain or variant thereof. In one embodiment, the vector may be capable of expressing a zeta globin chain and a gamma globin chain or variants thereof. In specific embodiments, the vector may be capable of expressing a non-variant zeta globin chain and a variant gamma globin chain; alternatively, the recombinant vector may be capable of expressing a variant zeta globin chain and a non-variant gamma globin chain; or the recombinant vector may be capable of expressing a variant zeta globin chain and a variant gamma globin chain.

The invention is further directed to a method for producing at least one globin chain or heme-binding fragment thereof in a yeast cell comprising:
(a) introducing into a yeast cell the above recombinant DNA vector; and
(b) growing the yeast cell in an appropriate medium such that the globin chain or heme-binding fragment thereof is expressed.

The invention also provides a yeast cell containing a recombinant DNA vector I capable of expressing a globin chain I in the yeast cell, and a recombinant DNA vector II capable of expressing a globin chain II in the yeast cell, wherein globin chains I and II are as defined above and wherein
(a) the recombinant DNA vector I expresses the globin chain I in the yeast cell and comprises:
   (i) a yeast transcription promoter I:
   (ii) a cDNA sequence I which encodes the globin chain I and is located downstream from the promoter I, wherein the promoter I regulates the transcription of the sequence I;
   (iii) a yeast selectable marker I or functionally active portion thereof;
   (iv) a yeast replication origin I or functionally active portion thereof; and
   (v) a transcription termination sequence I located downstream from the sequence I; and
(b) the recombinant DNA vector II expresses the globin II chain in the yeast cell and comprises:
   (i) a yeast transcription promoter II;
   (ii) a cDNA sequence II which encodes the globin chain II, and is located downstream from the promoter II, wherein the promoter II regulates the transcription of the sequence II;
   (iii) a yeast selectable marker II or functionally active portion thereof;
   (iv) a yeast replication origin II or functionally active portion thereof; and
   (v) a transcription termination sequence II located downstream from the sequence II.

The invention is also directed to a method for producing hemoglobin comprising an alpha-like globin chain and a beta-like globin chain comprising the steps of:
(a) introducing into a yeast cell a recombinant DNA vector comprising: (i) a DNA sequence encoding an alpha-like globin chain or variant thereof; (ii) a DNA sequence encoding a beta-like globin chain or variant thereof; (iii) a yeast transcriptional promoter which promotes the transcription of the DNA sequence encoding the alpha-like globin chain and the beta-like globin chain; (iv) a DNA sequence encoding a yeast selectable marker or functionally active portion thereof; and (v) a yeast replication origin;
(b) growing the yeast cell in an appropriate medium such that the alpha-like globin chain or heme-binding fragment thereof and the beta-like globin chain or heme-binding fragment thereof is expressed;
(c) isolating the alpha-like globin chain and the beta-like globin chain from the yeast cell; and
(d) mixing the alpha-like globin chain and the beta-like globin chain with a source of heme.

The invention is further directed to a method for producing hemoglobin comprising an alpha-like globin chain and a beta-like globin chain in a heme-producing yeast cell comprising:
(a) introducing into a yeast cell a recombinant DNA vector comprising: (i) a DNA sequence encoding an alpha-like globin chain or variant thereof; (ii) a DNA sequence encoding a beta-like globin chain or variant thereof; (iii) a yeast transcriptional promoter which promotes the transcription of the DNA sequence encoding the alpha-like globin chain and the beta-like globin chain; (iv) a DNA sequence encoding a yeast selectable marker or functionally active portion thereof; and (v) a yeast replication origin;
(b) growing the yeast cell in an appropriate medium such that the alpha-like globin chain or variant thereof and the beta-like globin chain or variant thereof are expressed and assembled together with heme in the heme-producing yeast cell to form hemoglobin comprising an alpha-like globin chain and a beta-like globin chain.

The invention is also directed to a method for producing hemoglobin comprising an alpha like-globin chain or variant thereof and a beta-like globin chain or variant thereof comprising the steps of:
(a) introducing into a yeast cell two recombinant DNA vectors in which the first recombinant DNA vector comprises: (i) a DNA sequence encoding an alpha like-globin chain or variant thereof; (ii) a yeast transcriptional promoter which promotes the transcription of the DNA sequence encoding the alpha like-globin chain or variant thereof; and (iii) a DNA sequence encoding at least one yeast selectable marker or functionally active portion thereof; and (iv) a yeast replication origin and in which the second recombinant DNA vector comprises (i) a DNA sequence encoding a beta like-globin chain or variant thereof; (ii) a yeast transcriptional promoter which promotes the transcription of the DNA sequence encoding the beta like-globin chain or variant thereof; and (iii) a DNA sequence encoding at least one yeast selectable marker or functionally active portion thereof; and (iv) a yeast replication origin;
(b) growing the yeast cell such that the alpha and beta like-globin chains or heme-binding fragments thereof are expressed;
(c) isolating the alpha and beta like-globin chains from the yeast cell; and
(d) combining the alpha and beta like-globin chains with a source of heme.

The invention is also directed to a method for producing hemoglobin comprising an alpha like-globin chain or variant thereof and a beta like-globin chain or variant thereof in a heme-producing yeast cell comprising the steps of:
(a) introducing into a yeast cell two recombinant DNA vectors in which the first recombinant DNA vector comprises: (i) a DNA sequence encoding an alpha like-globin chain or variant thereof; (ii) a yeast transcriptional promoter which promotes the transcription of the DNA sequence encoding the alpha like-globin chain or variant thereof; and (iii) a DNA sequence encoding at least one yeast selectable marker or functionally active portion thereof; and (iv) a yeast replication origin and in which the second recombinant DNA vector comprises (i) a DNA sequence encoding a beta like-globin chain or variant thereof; (ii) a yeast transcriptional promoter which promotes the transcription of the DNA sequence encoding the beta like-globin chain or variant thereof; and (iii) a DNA sequence encoding at least one yeast selectable marker or functionally active portion thereof; and (iv) a yeast replication origin; and
(b) growing the yeast cell in an appropriate medium such that the alpha and beta like-globin chains or variants thereof are expressed and assembled together with heme in the yeast cell to form hemoglobin.

### 4. BRIEF DESCRIPTION OF THE FIGURES

Figure 1 shows the nucleotide sequences of the embryonic zeta (1A), embryonic epsilon (1B), fetal gamma (1C), adult delta (1D), adult alpha (1E) and adult beta (1F) chains of human hemoglobin. The deduced amino acid sequences are shown underneath. The AUG start codon and the corresponding amino-terminal methionine which is removed by methionine aminopeptidase in a post-translational modification are not shown in the figures.
Figure 2 shows a partial restriction map of the plasmid pSPβC. The complete insert of the beta-globin gene is shown by the double line and the plasmid sequences are shown by a single line. Restriction sites shown above the line are A=AccI; E=EcoRI; F=SfaNI; H=HindIII; N=NcoI; and B=BamHI. The line above the restriction sites represents the coding region of the beta-globin gene. Numbers below the lines represent length in base pairs from an EcoRI site present in the vector.
Figure 3A shows the strategy used to clone the Porto Alegre beta-globin gene into YEp51. Figure 3B shows the strategy used to clone the adult beta-globin gene into YEp51.
Figure 4 shows the restriction map of the plasmid YEpWB51/Port.

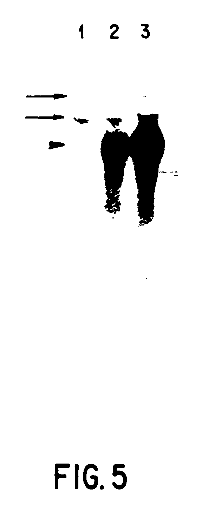

Figure 5 shows an autoradiograph of total RNA extracted from yeast strain Sc340 transformed with YEp51 (340g2C), YEp51T/Nat (340g2B) and YEpWB51/Port (340g2P). Total RNA was subjected to electrophoresis on a 1.1% agarose gel, transferred to the Hybond paper and probed with an ApaLI-HindIII fragment (600 bp) of the beta-globin gene from plasmid mp18bHS. The level of a control RNA (CYH2) was determined with the plasmid mp19CYH22 (9.0 kb) which carries the coding region of the CYH2 gene. 20 µg of the total RNA was loaded into each lane. Sample in each lane is as follows: Lane 1: 340g2C, Lane 2: 340g2B and Lane 3: 340g2P. β marks the beta-globin mRNA. The CYH2 mRNA is marked with C1 (precursor form) and C2 (mature mRNA). Numbers on the side indicate the length in nucleotides.

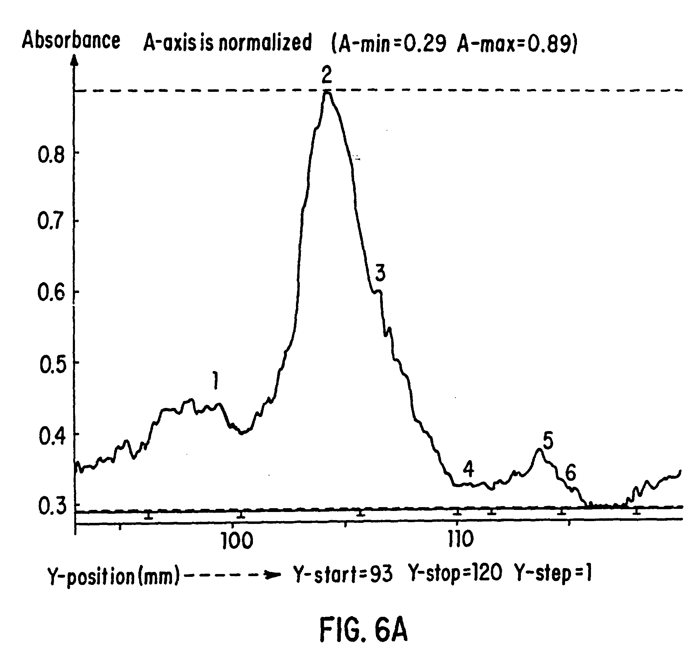

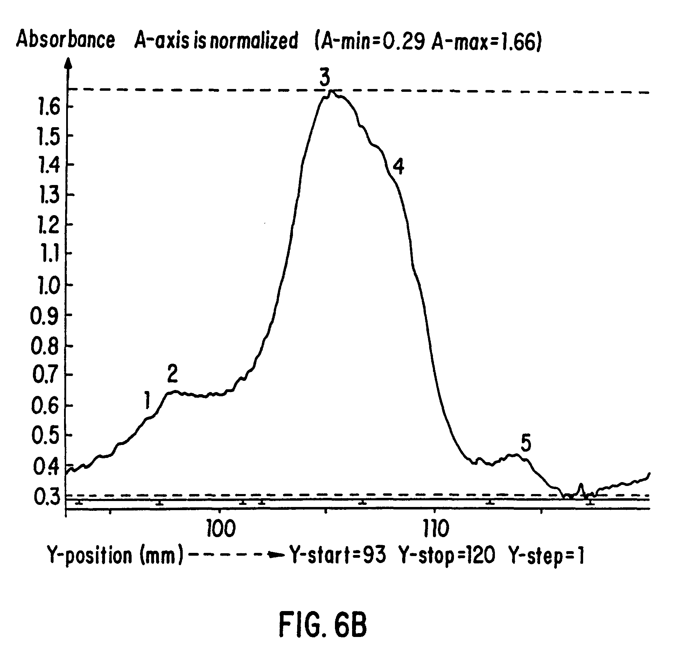

Figure 6A shows the results of scanning an autoradiograph containing both beta-globin and CYH2 mRNA obtained from a Northern Blot using an LKB gel scanner. The large peak in A (340g2B) represents the beta-globin mRNA and two small peaks at either side of the large peak represent the CYH2 mRNA. Figure 6B shows the results of scanning an autoradiograph containing both Porto Alegre beta-globin mRNA and CYH2 mRNA obtained from a Northern blot using an LKB gel scanner. The large peak in B (340g2P) represents the Porto Alegre beta-globin mRNA and the two small peaks at either side of the large peak represent the CYH2 mRNA.
Figure 7 shows a map of mp18βHS.
Figure 8 shows a map of AAH5.
Figure 9 shows a map of L19βAt.
Figure 10 shows the restriction map of plasmid pUC19-HβAt.
Figure 11 shows part of the GAL1-10 promoter sequence.
Figure 12 shows the restriction map of plasmid pUC19-GHβAt.
Figure 13 shows the restriction map of plasmid pNML-V-G-1.
Figure 14 shows the restriction map of the plasmid YEpWB51/Nat.
Figure 15 shows the strategy for cloning ADH1-terminator into YEpWB51/NAT.
Figure 16 shows the restriction map of the plasmid YEp51T/NAT.
Figure 17 shows the construction of YEp51T/G.
Figure 18 shows the DNA sequence of the gamma globin gene.
Figure 19 shows the sequences of and restriction sites present on GAM-5-S (5'-end primer) GAM-3-H (3'-end primer). These primers were used to synthesize gamma-globin DNA.
Figure 20 shows the restriction map of plasmid YEp51T/G.
Figure 21 shows the sequences of and the restriction sites present on the 5' primer, 5TG-ApaL and the 3' primer, ADHSBS-3' used to synthesize gamma(val) globin DNA.
Figure 22 shows the restriction map of plasmid pNM-5-G-gammaᵥₐₗ1.
Figure 23 shows the sequences of and restriction sites present on primers 51-A-1 and 519-A-3.
Figure 24 shows the sequences of and restriction sites present on primers G10T-5B and G10T3ESS.
Figure 25 shows the sequences of and restriction sites present on the 5' and 3' primers used to synthesize the hybrid promoter-first 36 bases of alpha-globin.
Figure 26 shows the sequences of and restriction sites present on the 5' and 3' primers used to synthesize the 3' alpha globin gene fragment.
Figure 27 shows the restriction map of plasmid pUC19-GHA.
Figure 28 shows the restriction map of plasmid pUC19-GHαGt.
Figure 29 shows the sequences and restriction sites of primers used for synthesizing the HαGt DNA fragment.
Figure 30 shows the restriction map of pUC19-AHαGt.
Figure 31 shows the restriction map of pPM40.
Figure 32 shows the restriction map of pNM-R-A-α1.
Figure 33 shows the restriction map of plasmid pNM-R-G-α1.
Figure 34 shows the strategy used for cloning the GHαGt cassette into the yeast vector carrying GHβAt expression cassette.

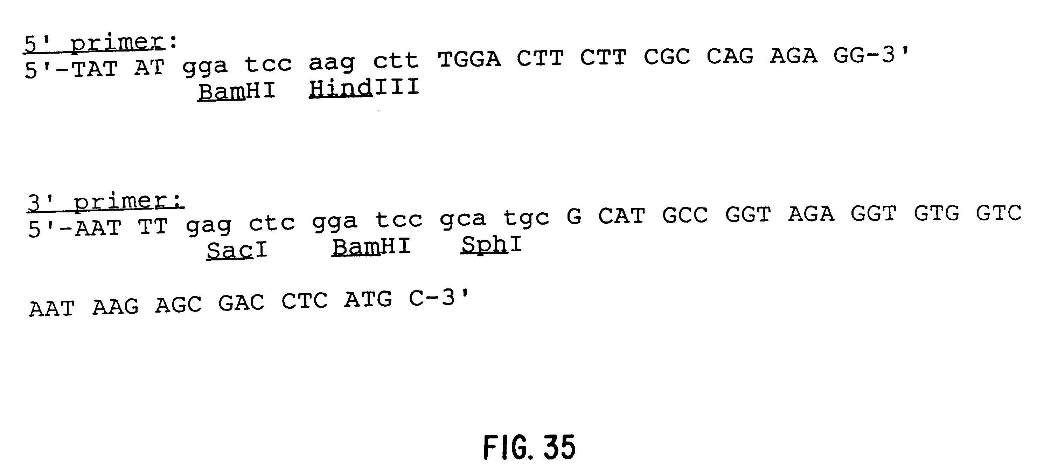

Figure 35 shows the sequences of and the restriction sites present on the 5' and 3' primers used for synthesizing the ADH1 terminator sequence in plasmid pUC19-ADHt.
Figure 36 shows the strategy used for cloning AHγᵥₐₗAt into pNM-R-A-α1.
Figure 37 shows the sequences of and restriction sites present on the 5' and 3' primers used for synthesizing the ADH2-UAS DNA fragment.
Figure 38 shows the map of plasmid pUC19-ADH2-UAS.
Figure 39 shows the sequences of and restriction sites present on the 5' primer, 5TDH3-3X and the 3' primer, ADH-t-SB.
Figure 40 shows the map of plasmid pUC19-AHγᵥₐₗAt.

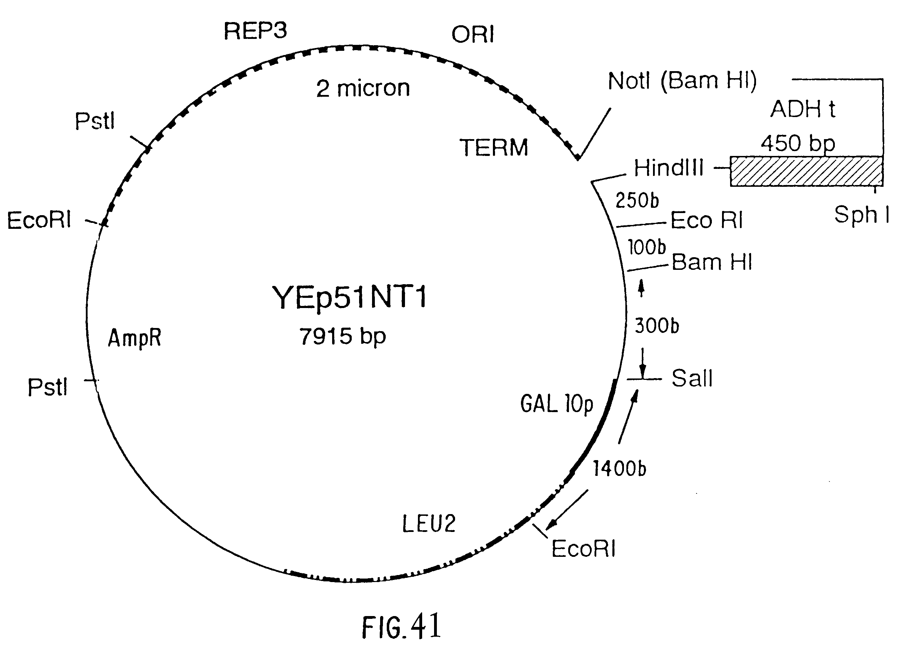

Figure 41 shows a restriction map of YEp51NT1.
Figure 42 shows the sequences of and the restriction sites on 5'-end primer, GAM-5-S and the 3'-end primer, G66T-3.

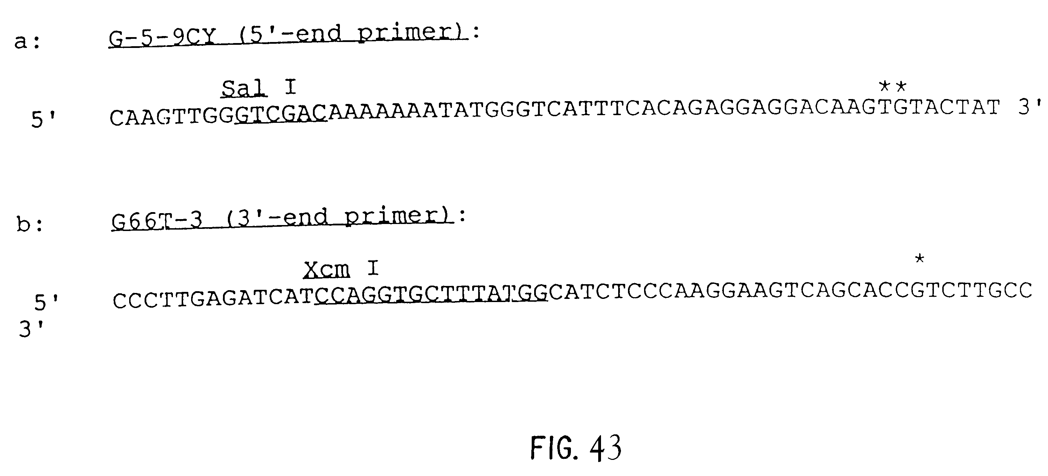

Figure 43 shows the sequences of and the restriction sites on 5'-end primer, G-5-9CY and the 3'-end primer, G66T-3.

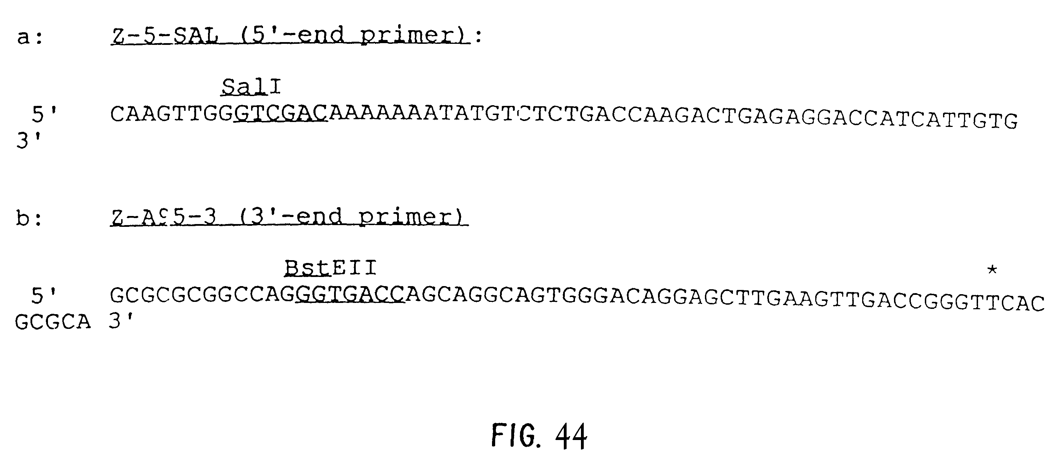

Figure 44 shows the sequences of and the restriction sites on 5'-end primer, Z-5-SAL and the 3'-end primer, Z-A95-3.

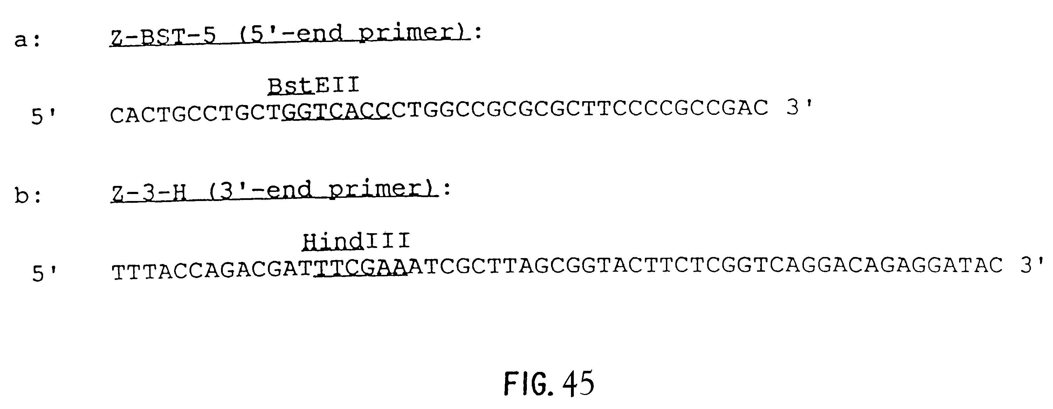

Figure 45 shows the sequences of and the restriction sites on 5'-end primer, Z-BST-5 and the 3'-end primer, Z-3-H.

### 5. DETAILED DESCRIPTION OF THE INVENTION

The globin chain is a zeta globin chain or variant thereof or gamma globin chain or variant thereof. Zeta globin and gamma globin may be mixed with a source of heme to obtain hemoglobin comprising zeta globin and gamma globin. Hemoglobin produced by methods of the present invention may be used in applications requiring physiological oxygen carriers such as in blood substitute solutions, or in a plasma expander.

The invention is also directed to recombinant vectors capable of expressing a globin chain or heme binding fragment thereof in yeast. The recombinant vector may be capable of expressing two globin chains or heme-binding fragments thereof. The globin chains comprise a zeta globin and a gamma globin chain or variants thereof. The invention also relates to methods for expressing at least one globin chain in yeast. Expressed zeta-globin and gamma-globin chains or variants thereof may be combined with a source of heme to produce hemoglobin or a variant thereof. The invention also relates to methods for expressing hemoglobin in yeast where the heme which is produced by the yeast or obtained from an exogenous source is ligated to the globin to form functional hemoglobins in vivo.

### 5.1. ISOLATION AND CLONING OF GLOBIN

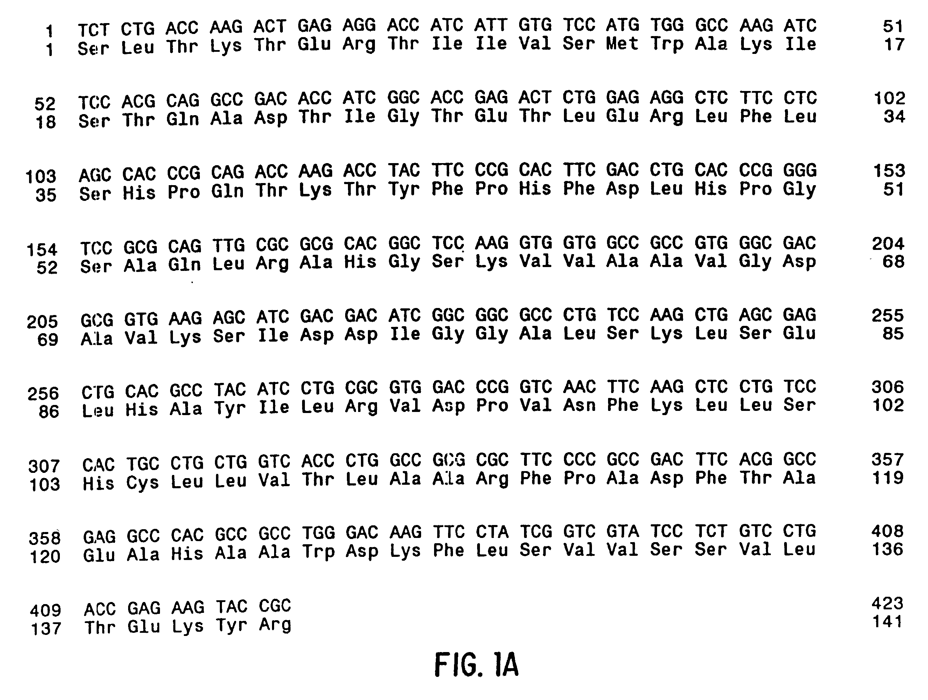

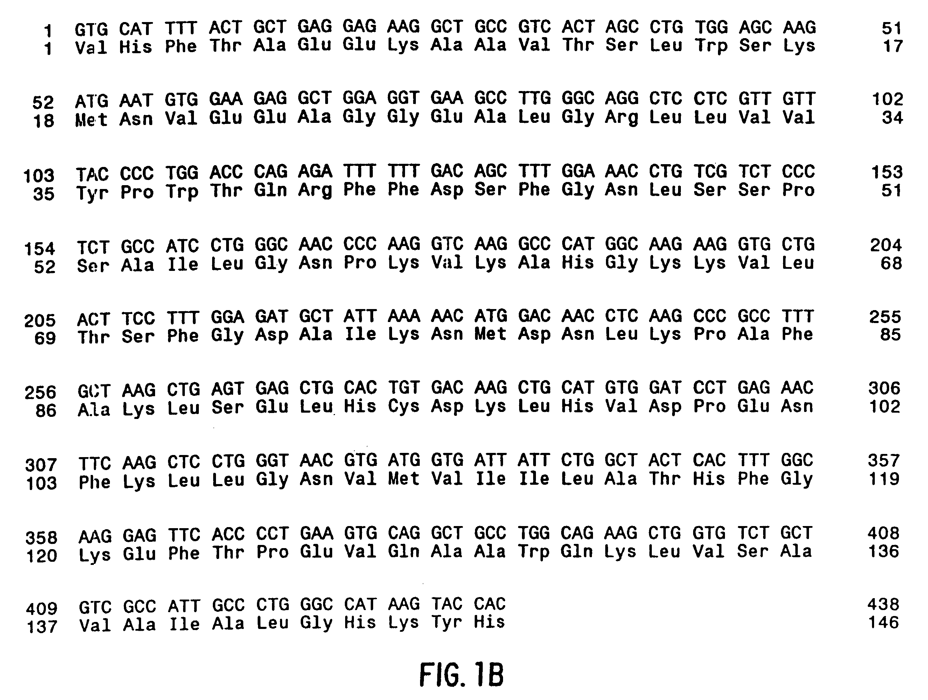

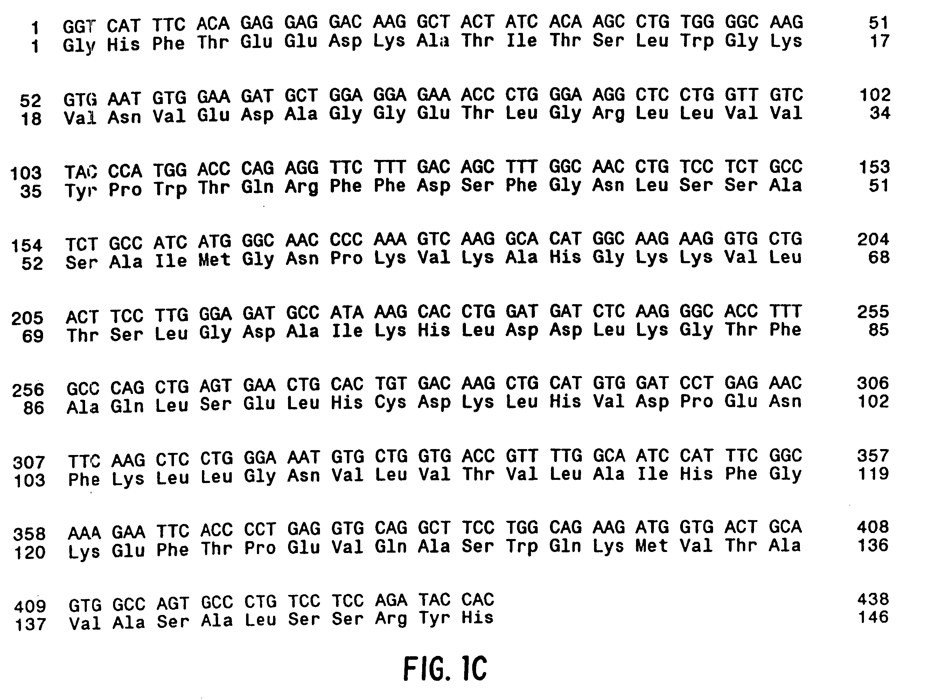

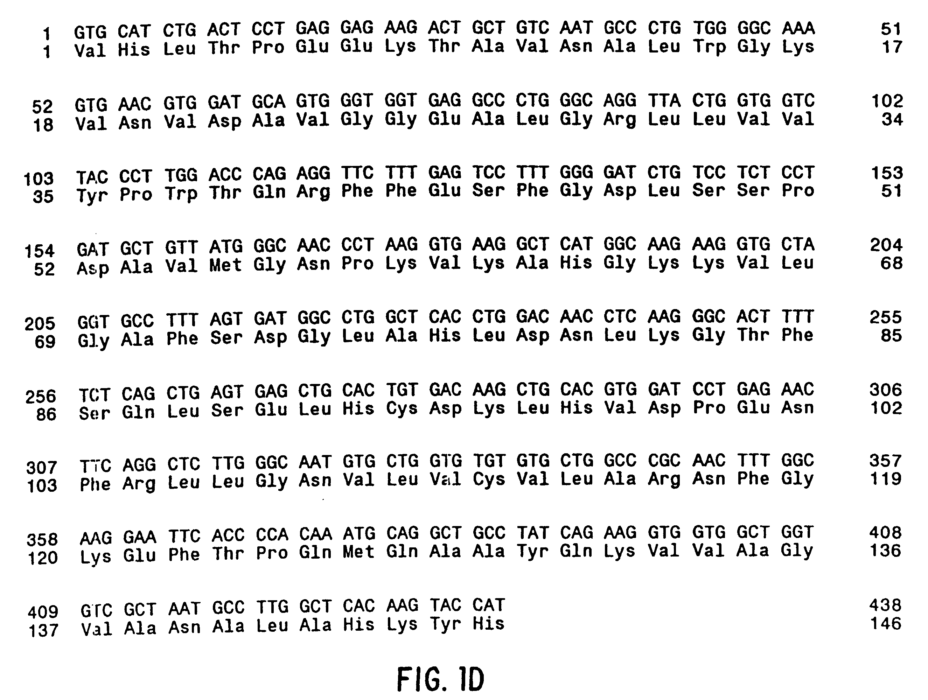

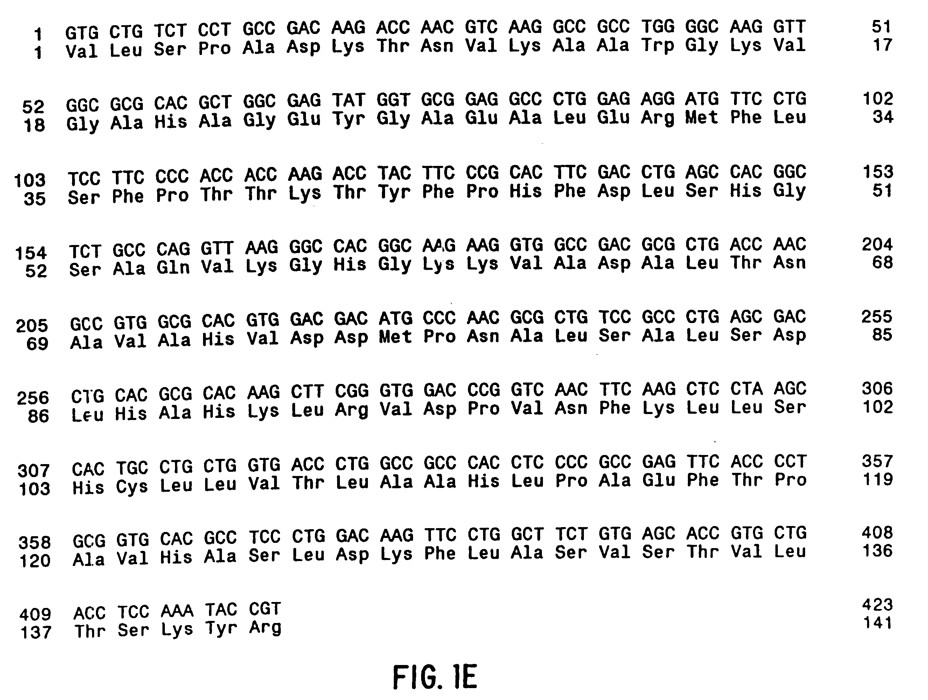

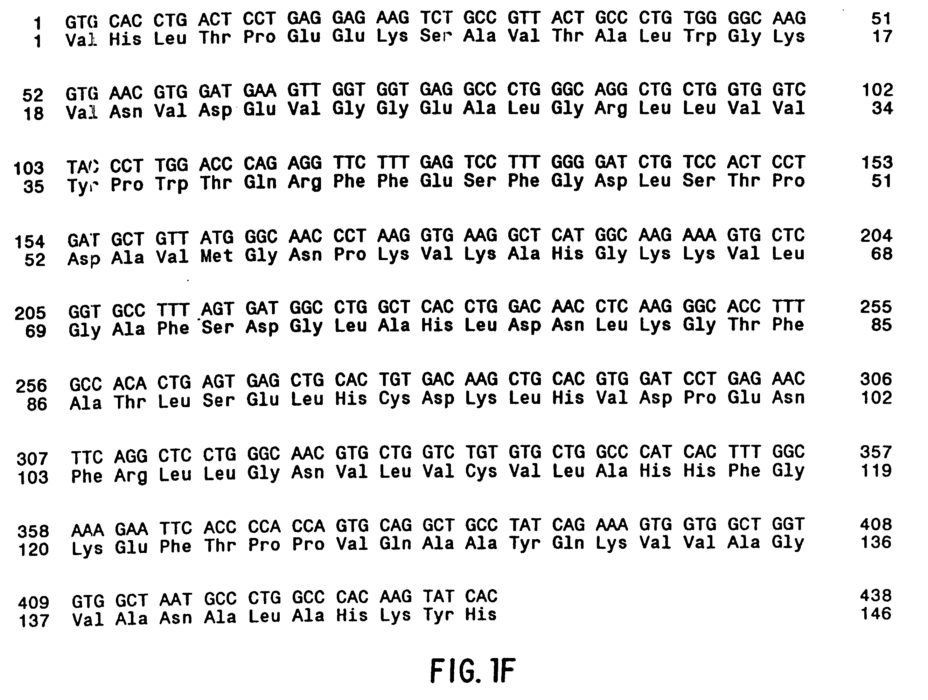

The nucleotide sequence of the genes encoding the human embryonic zeta globin, the human embryonic epsilon globin, the human fetal gamma globin, the human adult delta globin, the human adult alpha-globin and the human adult beta-globin chains and their derived amino acid sequences are depicted in Figures 1A-F respectively. These include but are not limited to nucleotide sequences comprising all or portions of the nucleotide sequence depicted in Figures 1A-F which are altered by the substitution of different codons that encode the same or a functionally equivalent amino acid residue thus producing a silent change as well as amino acid sequences comprising all or portions of the amino sequence depicted in Figures 1A, 1B, 1C, 1D, 1E, or 1F which are altered by the substitution of functionally equivalent amino acid residues within the sequence thus producing a silent change and derivatives thereof which are modified or processed.

The genes encoding alpha-like globin and beta-like globin chains may be isolated from hemoglobin containing cells using procedures known in the art. The DNA encoding alpha-like globin and/or beta-like globin may be obtained by standard procedures known in the art from cloned DNA (e.g. a DNA "library"), by chemical synthesis, by cDNA cloning or by the cloning of genomic DNA, or fragments thereof, purified from for example human reticulocytes (see for example, Maniatis et al., 1989, Molecular Cloning: A Laboratory Manual, Cold Spring Harbor Laboratory, Cold Spring Harbor, New York). DNA encoding alpha-like or beta-like globin DNA may also be obtained using polymerase chain reaction (PCR) technology (see for example Mullis et al., U.S. Patent No. 4,800,159, 1989). Clones derived from genomic DNA may contain regulatory and intron DNA regions, in addition to coding regions; clones derived from cDNA will contain only exon sequences. Whatever the source, a globin gene should be molecularly cloned into a suitable vector for propagation of the gene.

In the molecular cloning of the gene from genomic DNA, DNA fragments are generated, some of which will encode the desired globin gene. The DNA may be cleaved at specific sites using various restriction enzymes. Alternatively, one may use DNAse in the presence of manganese to fragment the DNA, or the DNA can be physically sheared, as for example, by sonication. The linear DNA fragments can then be separated according to size by standard techniques, including but not limited to, agarose and polyacrylamide gel electrophoresis and column chromatography.

Once the DNA fragments are generated, identification of the specific DNA fragment containing the globin may be accomplished in a number of ways. For example if an amount of a globin gene or its specific RNA, or a fragment thereof, is available and can be purified and labeled, the generated DNA fragments may be screened by nucleic acid hybridization to the labelled probe (Benton and Davis, 1977, Science 196:180 and Grunstein and Hogness, 1975, Proc. Natl. Acad. Sci. U.S.A. 72:3961). Those DNA fragments with substantial homology to the probe will hybridize. If a purified globin-specific probe is unavailable, nucleic acid fractions enriched in globin sequences may be used as a probe, as an initial selection procedure. It is also possible to identify the appropriate fragment by restriction enzyme digestion(s) and comparison of fragment sizes with those expected according to a known restriction map if such is available. Further selection on the basis of the properties of the gene, or the physical or chemical properties of its expressed product, as described infra, can be employed after the initial selection.

The globin gene can also be identified by mRNA selection by nucleic acid hybridization followed by in vitro translation. In this procedure, fragments are used to isolate complementary mRNAs by hybridization. In vitro translation products of the isolated mRNAs identifies the mRNA, and therefore the complementary DNA fragments that contain the globin sequences.

Alternatives to isolating the globin genomic DNA include, but are not limited to, chemically synthesizing the gene sequence itself from the known sequence or making cDNA to the mRNA which encodes the globin gene.

The identified and isolated gene or cDNA can then be inserted into an appropriate cloning vector. A large number of vector-host systems known in the art may be used. Possible vectors include, but are not limited to, cosmids, plasmids or modified viruses, but the vector system must be compatible with the host cell used. Such vectors include, but are not limited to, bacteriophages such as lambda derivatives, or plasmids such as pBR322, pUC, pGEM1®, or Bluescript® plasmid derivatives. Recombinant molecules can be introduced into host cells via transformation, transfection, infection, electroporation, etc.

In an alternative embodiment, the gene may be identified and isolated after insertion into a suitable cloning vector, in a "shot gun" approach. Enrichment for a globin gene, for example, by size fractionation or subfractionation of cDNA, can be done before insertion into the cloning vector.

The globin gene is inserted into a cloning vector which can be used to transform, or infect appropriate host cells so that many copies of the gene sequences are generated. This can be accomplished by ligating the DNA fragment into a cloning vector which has complementary cohesive termini. However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. In an alternative method, the cleaved vector and globin gene may be modified by homopolymeric tailing.

In specific embodiments, transformation of host cells with recombinant DNA molecules that incorporate an isolated globin gene, cDNA, or synthesized DNA sequence enables generation of multiple copies of the gene. Thus, the gene may be obtained in large quantities by growing transformants, isolating the recombinant DNA molecules from the transformants and, when necessary, retrieving the inserted gene from the isolated recombinant DNA.

After the globin-containing clone has been identified, grown, and harvested, its DNA insert may be characterized using procedures known in the art. The cloned DNA or cDNA corresponding to the globin gene can be analyzed by methods including but not limited to Southern hybridization (Southern, 1975, J. Mol. Biol. 98:503-517), restriction endonuclease mapping (Maniatis et al., 1989, Molecular Cloning, A Laboratory Manual, Cold Spring Harbor Laboratory, Cold Spring Harbor, New York), and DNA sequence analysis. DNA sequence analysis can be performed by any techniques known in the art, including but not limited to chemical methods (Maxam and Gilbert, 1980, Meth. Enzymol. 65:499-560), enzymatic methods (see e.g. Innes, 1988, Proc. Natl. Acad. Sci. U.S.A. 85:9436; Tabor and Richardson, 1987, Proc. Natl. Acad. Sci. U.S.A. 84:4767; and Sanger et al., 1977, Proc. Natl. Acad. Sci. U.S.A. 74:5463), or the use of an automated DNA sequenator (see for example Martin et al., 1985, Biotechnology 3:911-915).

### 5.2. GLOBIN VARIANTS

The production and use of globin variants are also envisioned and within the scope of the present invention. The term "globin variant" as defined herein refers to a globin whose nucleotide sequence has been altered in such a fashion so as to result in the alteration of the structure or function of the globin, but so that the globin still remains functionally active as defined by the ability to reversibly bind to oxygen. The variant may be naturally occurring or non-naturally occurring. The invention is directed to the following categories of hemoglobin variants: variants which autopolymerize; variants in which the tetramer does not dissociate under physiological conditions in vivo; variants with lowered intrinsic oxygen affinity, i.e. an oxygen affinity having a P50 of at least about 10 mm Hg under physiological conditions; and variants that are stable in alkali. In a specific embodiment, poly alpha-like globin or poly beta-like globin may result.

As discussed in Section 2.1.3. supra, globin variants which autopolymerize include but are not limited to Porto Alegre beta-globin or Hb Porto Alegre (beta-9 serine is replaced with cysteine), Hb Mississippi (beta-44 serine is replaced with cysteine), or Hb Ta-Li (beta-83 glycine is replaced with cysteine).

As also disclosed in Section 2.1.3., supra, an example of variant in which the tetramer does not dissociate includes but is not limited to Hb Rainier (beta-145 tyrosine is replaced by cysteine) and HbFᵥₐₗ (gamma-1 glycine is replaced by valine).

Alkali stable hemoglobin variants are those in which the dimers do not dissociate into monomers in the presence of alkali. One example is Motown/Hacettepe (beta-127 glutamine is replaced with glutamic acid). Another example is a variant in which serine replaces the alpha-104 cysteine (Perutz, 1974, Nature 247:371).

Examples of variants which have a lowered oxygen affinity include but are not limited to HbA Chico (beta-66 lysine is replaced by threonine); HbF Chico (gamma-66 lysine is replaced by threonine); HbA Titusville (alpha-94 aspartate to asparagine);Hb Portland Titusville (zeta-94 aspartate to asparagine); HbF Beth Israel (gamma-102 asparagine is replaced with serine); and HbF Kansas (gamma-102 asparagine to threonine).

In a specific embodiment, the globin variant is substantially homologous to an adult beta-qlobin chain and the variant comprises a cysteine at the beta-9 position. In a most specific embodiment, the globin variant is substantially homologous to Porto Alegre beta-globin and the variant comprises a cysteine at the beta-9 position. The term "substantially homologous" as used herein refers to the ability of a DNA sequence encoding a first globin chain to hybridize to a DNA sequence encoding a second globin chain under stringent conditions, for example, at about 0.1X SSC at a temperature of about 65°C. For example, if a globin variant is substantially homologous to an adult beta-globin chain, a DNA sequence encoding the globin variant is capable of hybridizing to a DNA sequence encoding the adult beta-globin chain under stringent conditions.

The globin variants may be produced by various methods known in the art. The manipulations which result in their production can occur at the gene or protein level. The globin may be altered at the gene level by site-specific mutagenesis using procedures known in the art. One approach which may be taken involves the use of synthetic oligonucleotides to construct variant globins with base substitutions. In one embodiment, a short oligonucleotide containing the mutation is synthesized and annealed to the single stranded form of the wild-type globin gene (Zoller and Smith, 1984, DNA 3:479-488). The resulting short heteroduplex can serve as primer for second strand synthesis by DNA polymerase. At the 5' end, a single stranded nick is formed which is closed by DNA ligase. In another embodiment, two complementary oligonucleotides are synthesized, each containing the mutant sequence. The duplex that forms after annealing these complementary oligonucleotides, can be joined to a larger DNA molecule by DNA ligase provided that the ends of both molecules have complementary single-stranded "sticky" ends. Another approach which may be taken involves introducing a small single-stranded gap in the DNA molecule followed by mis-repair DNA synthesis i.e., the misincorporation of a non-complementary nucleotide in the gap (Botstein and Shortle, 1985, Science 229:1193). The incorporation of a thiol nucleotide into the gap may minimize the excision of the non-complementary nucleotide. Alternatively, a globin variant may be prepared by chemically synthesizing the DNA encoding the globin variant using procedures known in the art (see for example Froehler, 1986, Nucl. Acids Res.14:5399-5407 and Caruthers et al., 1982, Genetic Engineering, J.K. Setlow and A. Hollaender eds., Plenum Press New York, vol. 4, pp. 1-17). In a preferred embodiment, fragments of the variant globin are chemically synthesized and these fragments are subsequently ligated together. The resulting variant globin strands may be amplified using procedures known in the art, e.g. PCR technology and subsequently inserted into a cloning vector as described in Section 5.1., supra. In a specific embodiment, site specific mutants may be created by introducing mismatches into the oligonucleotides used to prime the PCR amplification (Jones and Howard, 1990, Biotechniques 8:178-180).

Manipulations of the globin sequence may be carried out at the protein level. Any of numerous chemical modifications may be carried out by known techniques including but not limited to specific chemical cleavage by cyanogen bromide, trypsin, chymotrypsin, papain, V8 protease, NaBH₄; acetylation, formylation, oxidation, reduction; etc. Alternatively, the variant globin protein may be chemically synthesized using procedures known in the art, such as commercially available peptide synthesizers and the like. Such standard techniques of polypeptide synthesis can be found described in such publications as Merrifield, 1963, J. Chem. Soc. 85:2149-2154 and Hunkapillar et al., 1984, Nature (London) 310:105-111).

### 5.3. EXPRESSION OF HEMOGLOBIN

The nucleotide sequence coding for a globin chain is inserted into an appropriate expression vector, i.e. a vector which contains the necessary elements for the transcription and translation of the inserted protein-coding sequence. A variety of host-vector systems may be utilized to express the DNA sequence encoding the globin chain. These include but are not limited to mammalian cell systems infected with virus (e.g. vaccinia virus, adenovirus, etc.); insect cell systems infected with virus (e.g. baculovirus); yeast containing yeast vectors, and bacteria transformed with plasmid DNA, cosmid DNA, or bacteriophage DNA. In a preferred aspect, the host cell is a yeast cell.

### 5.3.1. EXPRESSION OF HEMOGLOBIN IN YEAST

Special considerations however have to be taken into account when expressing globin chains in yeast. Different signals regulating the expression of sequences encoding globin chains in yeast are required than when expressing such sequences in procaryotic systems or mammalian systems. For example, a yeast replication origin is required in a recombinant DNA vector capable of expressing a globin sequence in order for there to be replication of such a vector and thus significant expression. The nucleotide sequence coding for the alpha-like and/or beta-like chain of globin is inserted into a vector which may be expressed in yeast. In one embodiment, one DNA sequence encoding one globin chain or variant thereof is inserted into the recombinant DNA vector. In another embodiment, one DNA sequence encoding two globin chains or variants thereof may be inserted into the recombinant DNA vector. In yet another embodiment two DNA sequences, each encoding one glcbin chain or variant thereof is inserted into the recombinant DNA vector. In a specific embodiment, one DNA sequence encodes an alpha-like globin chain or variant thereof and the second DNA sequence encodes a beta-like globin chain or variant thereof.

In a further embodiment, the yeast cell is a member of the species Saccharomyces cerevisiae. Such a vector comprises in addition to the DNA sequence encoding the globin: (a) a yeast transcriptional promoter which promotes the transcription of the DNA sequence encoding the globin chain; (b) a DNA sequence encoding a yeast selectable marker or functionally active portion thereof; and (c) a yeast replication origin.

The first component of the vector, a yeast transcriptional promoter comprises two components: (a) a transcriptional regulatory region which contains a structural gene distal region, or activator sequence which provides for regulated (inducible) or constitutive transcription and (b) the transcriptional initiation region which includes the transcription initiation site, the "TATA" sequence, capping sequence as appropriate, and an RNA polymerase binding sequence, which includes nucleotides upstream from the initiation site for directing the initiation of synthesis of the messenger RNA. In a preferred embodiment, the activator sequence is an upstream activator sequence. The transcriptional regulatory region will preferably be at least 100 base pairs (bp) and will not exceed 3000 base pairs. The regulatory region may begin at least about 200 bp from the initiation codon, usually at least about 300 bp and may begin at 400 bp or farther upstream from the initiation codon. The transcriptional initiation region will be at least about 150 bp, more usually at least about 200 bp, usually not more than about 600 bp, and preferably about 400 bp. The sequence may extend in the downstream direction of transcription from about bp -10 to about bp -25 (relative to transcription initiation at +1).

In one embodiment, the yeast transcriptional promoter is an inducible promoter. Inducible promoters may be unidirectional or bidirectional. Unidirectional inducible promoters in a preferred embodiment are located upstream from the DNA sequence encoding the globin chain. Unidirectional inducible promoters may include but are not limited to promoters which are regulated by galactose (e.g. UDP-galactose epimerase (GAL10), galactokinase (GAL1)), glucose (e.g. alcohol dehydrogenase II (ADH2)), and phosphate (e.g. acid phosphatase (PHO5)). In another embodiment, the inducible promoter may be a bidirectional promoter. A bidirectional promoter may be located upstream (5' of the ATG start codon) from the DNA sequence encoding a globin chain on the plus strand at one of its ends and upstream from the DNA sequence encoding a globin chain on the minus strand at its other end; and thereby, control the transcription of both. In a specific embodiment, such a bidirectional promoter is GAL1-10.

The promoter may also be a constitutive promoter. In a specific embodiment, the constitutive promoter is a promoter of glyceraldehyde-3-phosphate dehydrogenase III (TDH3) transcription and is herein after referred to as the TDH3 promoter. Other constitutive promoters include but are not limited to glyceraldehyde-3-phosphate dehydrogenase II (TDH2), glyceraldehyde-3-phosphate dehydrogenase I (TDH1), alcohol dehydrogenase I (ADH1), phosphoglycerate kinase (PGK), pyruvate kinase (PYK), enolase (ENO), and triose phosphate isomerase (TPI). Such promoter sequences will be at least about 200 bp and will not exceed about 5000 base pairs.

In another embodiment, the promoter can be a hybrid promoter, in which the sequence containing the transcriptional regulatory region is obtained from one source and the sequence containing the transcription initiation region is obtained from a second source. In one embodiment, the sequence containing the transcriptional regulatory region is an upstream activating sequence of a yeast inducible promoter. The inducible promoter can be a unidirectional or a bidirectional promoter. The sequence containing the transcriptional initiation region may be obtained from the transcriptional initiation region of a constitutive promoter. In a specific embodiment, the hybrid promoter comprises a transcriptional regulatory region which is the upstream activation sequence of the GAL10 promoter and a transcription initiation region which contains the transcription initiation region of the TDH3 promoter. In another specific embodiment, the hybrid promoter can regulate the expression of two separate DNA sequences in opposite orientations if the hybrid promoter comprises an upstream activating sequence with transcription initiation sites located on both sides, thereby forming a bidirectional promoter. In a very specific embodiment, a GAL1-10 upstream activating sequence may be flanked on either side by the initiation region of the TDH3 promoter. DNA encoding a globin chain is located downstream from each TDH3 sequence. In another embodiment, the ADH2 UAS may be used in place of the GAL1-10 UAS. In still other embodiments, the transcriptional initiation region of the TDH3 promoter can be substituted by TDH1, TDH2, PGK, ENO, TPI, CYC1, or PYK.

Another component of the recombinant DNA vector is a sequence encoding a yeast selectable marker. The recombinant DNA vector in one embodiment may contain more than one such sequence. A yeast selectable marker provides for selective pressure for survival of yeast cells expressing the marker. In a preferred aspect, the selectable marker complements a genetic defect in the host strain. For example, URA3 can be used as a selectable marker in a yeast strain which is deficient in the URA3 gene product. Such sequences may include but are not limited to the LEU2 gene, the URA3 gene, the HIS3 gene, the LYS2 gene, the HIS4 gene, the ADE8 gene, the CUP1 gene, and the TRP1 gene. Another example of such a sequence includes the leu2d gene which is a promoter defective LEU2 gene. In a preferred embodiment, the leu2d gene is inserted into a multicopy recombinant DNA vector. A yeast cell transformed by a vector comprising the LEU2 or leu2d gene may grow in leucine free media; a yeast cell transformed by a vector comprising the URA3 gene may grow in uracil free media; a yeast cell transformed by a vector comprising the LYS3 gene may grow in lysine free media, a yeast cell transformed by a vector comprising the HIS3 gene may grow in histidine free media, a yeast cell transformed by a vector comprising the ADE8 gene may grow in adenine free media, a yeast cell transformed by a vector comprising the HIS4 gene may grow in histidine free media, a yeast cell transformed by a vector comprising the CUP1 gene may grow in media containing levels of copper inhibitory to the host strain without plasmid; and a yeast cell transformed by a vector comprising the TRP1 gene may grow in tryptophan free media. The recombinant. DNA vector may also comprise a DNA sequence encoding a functionally active portion of a yeast selectable marker. The term "functionally active portion"-as defined herein is a portion of the sequence that encodes a portion of the marker which provides an effective amount of selective pressure for the survival of yeast cells expressing the portion of the marker.

The recombinant vector also comprises a yeast replication origin or functionally active portion of the replication origin which effects replication of the vector. Any replication origin useful in yeast may be employed which provides for efficient replication and maintenance (reviewed for example in Kingsman and Kingsman, U.S. Patent No. 4,615,974, issued October 7, 1986). Examples of such replication origins include but are not limited to the 2µ plasmid replication system, or a functionally active portion thereof and autonomous replicating sequences (ARS). Examples of ARS include but are not limited to ARS1 or ARS3. The replication origins may be of high or low copy number, depending on the effect of the construct on the viability of the host. The vector may further comprise centromeric sequences (CEN) which may provide meiotic and mitotic stability. Examples of CEN sequences include but are not limited to CEN3, CEN4, and CEN11.

The expression vector may further comprise but does not always require a transcription termination sequence. A transcription termination sequence may include the necessary transcription signals for termination and polyadenylation and may be derived from any yeast sequence. In a specific embodiment, the transcription termination sequence is the alcohol dehydrogenase I (ADH1) termination sequence. Other termination sequences suitable for use include but are not limited to those of iso-1-cytochrome c (CYC1), UDP-glucose-4-epimerase (GAL10), phosphoglycerate kinase (PGK), acid phosphatase (PHO5), enolase (ENO), and triose phosphate isomerase (TPI). The transcription termination sequence is at least about. 100 bp and should not exceed about 1500 bp. In a preferred embodiment, the transcription termination sequence ranges from about 150 bp to about 1200 bp.

The expression vectors of the present invention may be constructed using recombinant DNA procedures known in the art. Such procedures were disclosed in detail in Section 5.1., supra. Specific examples of yeast expression vectors and their construction, comprising sequences encoding adult beta-globin under the control of the hybrid promoter containing the GAL1-10 promoter, and the TDH3 promoter, are disclosed in Section 7. A specific example of a yeast expression vector and its construction, comprising sequences encoding the Porto Alegre beta-globin chain under the control of the GAL10 inducible promoter is disclosed in Section 6. A specific example of a yeast expression vector and its construction, comprising sequences encoding the adult alpha-globin chain under the control of a hybrid promoter are disclosed in Sections 10 and 11. A specific example of a yeast expression vector and its construction, comprising sequences encoding the gamma-globin chain under the control of the GAL10 inducible promoter is disclosed in Section 8. A specific example of a yeast vector and its construction, comprising sequences encoding a gamma-globin chain variant is disclosed in Section 9. Specific examples of other globin variants are disclosed in Section 16. Specific examples of a yeast expression vector and its construction, comprising sequences encoding an alpha like-globin chain and a beta like-globin chain, are disclosed in Sections 13 and 15. A specific example of a yeast expression vector and its construction, comprising sequences encoding the zeta-globin chain under the control of a hybrid promoter is disclosed in Section 14. Specific examples of the expression of hemoglobin by coexpression of plasmids comprising sequences encoding alpha like- and beta like-globin are disclosed in Sections 12, 17, 18, and 19.

The expression vectors of the present invention may be propagated in yeast using procedures known in the art. The expression vectors may be propagated in yeast which may or may not be capable of producing heme. The yeast can be transformed with one or more of the expression vectors using procedures known in the art (e.g. the spheroplast method, (Hinnen et al., 1978, Proc. Natl. Acad. Sci. U.S.A. 75:1929-1933) or the lithium acetate method (Ito et al., 1983, J. Bact. 153:163-168)). Transformants may be selected by the presence of the marker (selectable) gene function in the transformant. For example, a leu2-yeast cell transformed with an expression vector comprising a LEU2 marker gene is selected by virtue of its ability to grow in leucine free media. The transformed yeast cells may be grown in media comprising a nitrogen and carbon source as well as essential vitamins, minerals, and trace elements (Hinnen et al., 1978, Proc. Natl. Acad. Sci. U.S.A. 75:1929-1933). If the vector comprises an inducible promoter, the media should also comprise the inducer.

If the expression vector comprises DNA sequences encoding both an alpha-like globin chain and beta-like globin chain or a beta-like globin chain (e.g. gamma-globin chain), hemoglobin may be expressed in the yeast cell transformed with the vector. In one embodiment, the heme is produced by the yeast and ligated to the globin to form functional hemoglobins in vivo. In another embodiment, the yeast cell may be deficient in components required for heme production, for example 5-aminolevulinic acid. Hemoglobin may still be expressed in such a cell if the required component is added.

The protein product of the expressed globin gene may be isolated and purified using standard methods including but not limited to chromatography (e.g., ion exchange, affinity, and sizing column chromatography), centrifugation, differential solubility, or by any other standard technique for the purification of proteins. If one globin chain is expressed, the expressed globin chain may be combined with another globin chain and a source of heme to form hemoglobin. If hemoglobin is expressed in the yeast cell, no further steps are necessary.

The expressed gene and its product may be analyzed at the genomic level or the protein level using procedures known in the art. For example, hemoglobin gene expression may be analyzed by Southern or Northern hybridization. The expressed hemoglobin protein may for example be analyzed by Western Blot procedures known in the art and also described herein in Section 6.6., infra.

### 5.4. USES FOR EXPRESSED RECOMBINANT HEMOGLOBINS

Hemoglobin of large quantity and high purity may be obtained using the methods of the present invention. Examples of hemoglobin which may be obtained include but are not limited to Hb Portland I (zeta₂gamma₂). The hemoglobin will be free of cellular material and other contaminants. Such hemoglobins and especially hemoglobin variants which autopolymerize; variants which prevent the dissociation of the tetramer, variants with lowered intrinsic oxygen affinity, variants that are stable in alkali, variants that are stable in acid, variants which do not autooxidize, and/or variants which do not bind to haptoglobin through the use of variant alpha and/or beta-globin genes described in Section 5.2, supra are of value for use in blood substitutes.

In another embodiment, alpha-like and/or beta-like globin may be chemically modified using procedures known in the art to increase tetramer stability and/or lower oxygen affinity (see Section 2.1.2., supra for examples of such procedures). A wild-type or variant alpha-like or beta-like globin may be modified. Such chemically modified hemoglobins may also be used in blood substitutes.

The hemoglobin compositions, in addition to being used in blood substitutes, may be used in a blood plasma expander, in a pharmaceutical composition with an acceptable carrier, and with other plasma expanders, or in any application where a physiological oxygen carrier is needed. The pharmaceutical carriers may be such physiologically compatible buffers as Hank's or Ringer's solution, physiological saline, a mixture consisting of saline and glucose, and heparinized sodium-citrate-citric acid-dextrose solution. The hemoglobin produced by the methods of the present invention can be mixed with colloidal-like plasma substitutes and plasma expanders such as linear polysaccharides (e.g. dextran), hydroxyethyl starch, balanced fluid gelatin, and other plasma proteins. Additionally, the hemoglobin may be mixed with water soluble, physiologically acceptable, polymeric plasma substitutes, examples of which include polyvinyl alcohol, poly(ethylene oxide), polyvinylpyrrolidone, and ethylene oxide-polypropylene glycol condensates. Techniques and formulations for administering the compositions comprising the hemoglobin generally may be found in Remington's Pharmaceutical Sciences, Meade Publishing Col., Easton, PA, latest edition.

The following examples are presented by way of illustration not by way of limitation.

### 6.0. EXAMPLE 1: EXPRESSION OF THE PORTO ALEGRE BETA-GLOBIN IN A YEAST EXPRESSION VECTOR CONTAINING THE GAL10 PROMOTER

As detailed herein, the natural beta-globin was modified to obtain a Porto Alegre beta-globin gene by replacing a 104 bp AccI-NcoI fragment from the natural beta-globin gene with a synthetic oligonucleotide containing a cysteine as amino acid 9 (instead of a serine). The Porto Alegre beta-globin gene was subsequently cloned into the yeast expression vector YEp51 to obtain plasmid YEpWB51/PORT. YEpWB51/PORT was transformed into yeast strain Sc340, a hem1 strain. Quantitation of RNA by scanning the autoradiograph showed that mRNA for the Porto Alegre beta-globin was around 6.0% of total yeast RNA. Western blot analysis indicated that Porto Alegre beta-globin was expressed.

### 6.1. MATERIALS

The restriction enzymes, Klenow enzyme and T4-DNA ligase were obtained from New England Biolabs (Biolabs), Bethesda Research Laboratories (BRL) or Boehringer Mannheim (BM). All enzymes were used according to the suppliers specifications. Plasmid DNA was isolated from a one liter culture of the transformed E. coli cells and purified by CsCl gradient centrifugation.

### 6.2. CLONING OF THE PORTO ALEGRE BETA-GLOBIN GENE INTO THE YEAST EXPRESSION VECTOR YEp51

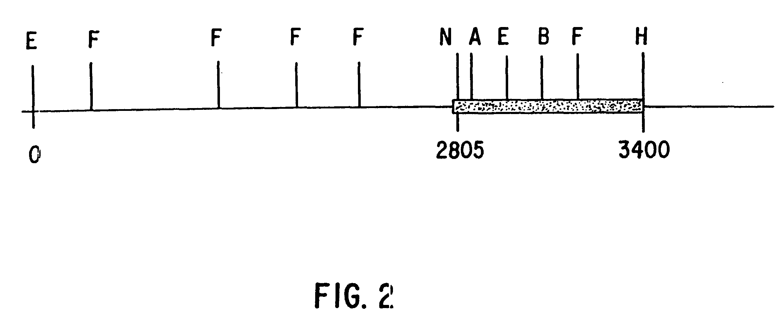

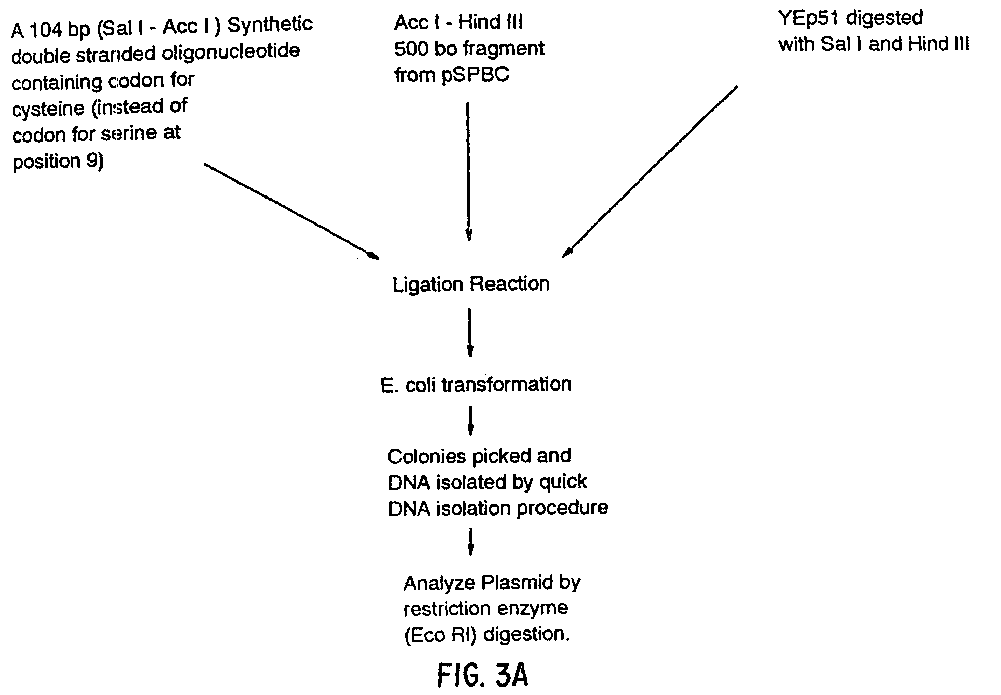

The general procedure used to clone the Porto Alegre beta-globin gene into the yeast expression vector YEp51 is shown in Figure 3A. The plasmid pSPβC (see Figure 2 for the partial restriction map of pSBβC) was digested withAccI and HindIII. Digestion with this combination of enzymes generated two fragments. A 500 base pair (bp) DNA containing the beta-globin gene and a 2800 bp fragment from the plasmid. The 500 bp fragment was isolated from a 0.6% agarose gel. After the band was excised from the gel, the DNA was electroeluted, and ethanol precipitated. The precipitated DNA was spun in an Eppendorf Centrifuge, the supernatant was removed and the DNA pellet was dried under vacuum.

The 500 bp DNA fragment carrying the natural beta-globin gene fragment isolated from pSPβC was AccI compatible at the 5'-end while the 3'-end was HindIII compatible. To modify the 5'-end of the isolated fragment, a synthetic oligonucleotide was used. This double stranded oligonucleotide (104 bp) contained a codon for cysteine as amino acid 9 instead of a codon for serine and had a AccI compatible end at its 3'-end and a SalI compatible end at it 5'-end (see Figure 3A). The 3'-end of the isolated fragment did not receive any adapter as the HindIII site was compatible with the HindIII site introduced into the YEp51.

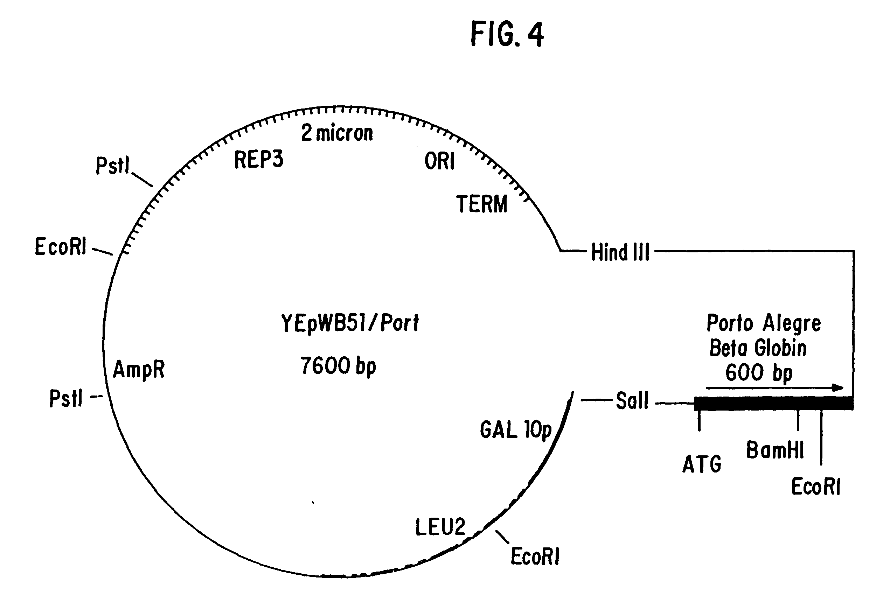

The recipient plasmid YEp51 was cleaved with SalI and HindIII restriction enzymes. To insert the isolated fragment containing the beta-globin gene, a three-way ligation was set up (see Figure 3A). The ligation reaction was carried out using the standard ligation procedures (Maniatis et al., 1982, Molecular Cloning, A Laboratory Manual, Cold Spring Harbor Laboratory, Cold Spring Harbor, New York). The ligation mixture was transformed into the E. coil HB101 cells using standard transformation procedure. Cells were spread on plates containing LB-media with 100 mg/L ampicillin. Plates were incubated overnight at 37°C. Forty eight colonies from the ampicillin plates were picked and a 5 ml culture was inoculated with individual transformant. Cultures were grown overnight at 37°C with vigorous shaking. The plasmid DNA was isolated from 1.5 ml of the overnight culture using the quick alkaline plasmid isolation procedure (Maniatis et al., 1982, Molecular Cloning, A Laboratory Manual, Cold Spring Harbor Laboratory, Cold Spring Harbor, New York). The plasmid from each transformant was digested with EcoRI to confirm the presence of a DNA fragment containing the Porto Alegre beta-globin gene. The plasmid carrying the Porto Alegre beta-globin gene was called YEpWB51/Port. The map of plasmid YEpWB51/Port is shown in Figure 4.

### 6.3. TRANSFORMATION OF Sc340 CELLS WITH YEpWB51/Port

The yeast strain Sc340 was obtained from Dr. J.E. Hopper of Hershey Medical Center. The genotype of this strain is:
MATa ura3-52, leu2, ade1, his3::GAL10^{uas}-GAL4-URA3⁺, MEL⁺.

Sc340 cells were transformed with the plasmids YEpWB51/Port and YEp51 (control). The spheroplast method of transformation was performed according to the published procedure (Hinnen et al., 1978, Proc. Natl. Acad. Sci. U.S.A. 75:1929-1933). The transformants were selected by plating out on minimal media containing 0.67% Bacto yeast nitrogen base without amino acids, 2% glucose, 20 mg/L adenine sulfate, 20 mg/L histidine, and 20 mg/L uracil. The plates were incubated at 28°C for three days and were examined for colony formation.

Colonies were picked from these plates following incubation and were precultured in yeast minimal media (0.67% yeast nitrogen base without amino acids) containing 0.5% glucose plus 20 mg/L each of adenine, uracil, and histidine. The overnight culture was then used to inoculate 1000 ml of the yeast minimal media containing 2% lactic acid, 3% glycerol and appropriate amino acids. The cultures were inoculated to OD₆₀₀ of 0.02. Cultures were grown at 30°C until they reached OD₆₀₀ of 0.20 (usually after 48 hours). Induction was initiated by the addition of galactose to a final concentration of 2% in the media. After four hours, cultures were harvested by centrifugation and the pellet was washed with 150 mM NaCl. The pellet was divided into two parts. One part was used for RNA isolation and the other was kept at -70°C for Western blot analysis.

### 6.4. QUANTITATION OF RNA FROM SC340 CELLS TRANSFORMED WITH PLASMIDS YEp51 AND YEp51WB/Port

RNA was isolated using published procedures (Meyhack et al., 1982, The EMBO Journal 1:675-680 or Carlson and Botstein, 1982, Cell 28:145). Yeast cells were washed with 150 mM NaCl and the pellet was resuspended in RNA buffer (0.5 M NaCl, 0.2 M Tris-HCl, pH 7.6, 0.1 M EDTA and 1% SDS). Approximately 0.5 g of glass beads (0.45-0.5 mm) were added to the tubes. An equal volume of phenol mixture (phenol: chloroform:isoamyl alcohol 25:24:1, equilibrated with RNA buffer without SDS) was added. Yeast cells were broken by vortexing at maximum speed for 2.5 minutes and the sample was placed on ice for 3 minutes. The above step was repeated twice more. Equal volumes of RNA buffer and phenol mixture were added to the cells and tubes were centrifuged. Aqueous phase was transferred to a clean Corex tube and 2.5 volumes of ethanol were added to each tube. RNA was allowed to precipitate at -20°C for 4 to 6 hours. RNA was pelleted by centrifugation and dried under vacuum. RNA pellet was suspended in sterile water.

Total RNA was denatured using the glyoxal method (Thomas, P., 1983, in "Methods in Enzymology", Colowhich, S. P. and Kaplan, N. O. eds. Vol. 100: pp. 255-266, Academic Press, New York). RNA was electrophoresed on 1.1% agarose gel in 10 mM NaPO₄ for approximately 4 hours at 75 volts (constant). After the electrophoresis was complete, RNA was transferred to Amersham Hybond-N paper (Thomas, P., 1983, in "Methods in Enzymology" Colowhich, S. P. and Kaplan, N. O. eds. Vol. 100: pp 255-266, Academic Press, New York).

Total yeast RNA bound to the filter paper was hybridized to the radioactive labelled beta-globin DNA. Hybridizations were carried out at 42°C overnight in 50% (v/v) formamide with 5X SSC (SSC: 3.0 M NaCl, 0.3 M Na citrate, pH 7.5); 50 mM NaPO₄, pH 6.5; 250 µg/ml Salmon sperm DNA; and 1X Denhardt's solution; (Denhardt's solution: 0.02% Ficoll, 0.02% polyvinylcarbonate, and 0.02% BSA, fraction V). The CYH2 mRNA which codes for yeast ribosomal protein L19 was used as control. The probe was plasmid mp10CYH22 which carries the yeast CYH2 gene. After the hybridizations, filters were washed three times at room temperature in 2X SSC and 0.1% SDS and four times at 50°C in 0.1X SSC and 0.1% SDS. Filters were exposed to X-ray films for 1 hour to overnight depending on the radioactivity. X-ray films were developed in a Konica automated film developer.

The results from these RNA blot hybridizations are shown in Figure 5, which show results from RNA isolated from yeast transformed with a control plasmid containing no globin sequences (lane 1), a plasmid containing sequences encoding beta-globin (lane 2), and a plasmid containing sequences encoding Porto Alegre beta-globin (lane 3). The results indicate that the mRNA samples from all sources were intact and no degradation was detected. It was also observed that no beta-globin mRNA could be detected in lane 1, which contains the parent plasmid only. These results indicate that nonspecific hybridization of the beta-globin probe is minimal.

Autoradiographs containing bands corresponding to both beta-globin and CYH2 mRNA were scanned using the LKB gel scanner. Results obtained from the scanner are shown in Figure 6. It can be clearly seen that the abundance of CYH2 mRNA in all three lanes is approximately the same while the abundance of the Porto Alegre beta-globin mRNA in 340g2P was high.

### 6.5. WESTERN BLOT ANALYSIS OF EXPRESSED PORTO ALEGRE BETA-GLOBIN

Four major steps were involved in the analysis of the expressed Porto Alegre beta-globin:
(1) Sample preparation via yeast cell disruption using glass beads followed by protein solubilization using SDS-containing buffer.
(2) Extracted protein separation via polyacrylamide gel electrophoresis.
(3) Protein transfer to nitrocellulose paper by application of a transverse electrical field.
(4) Globin protein detection via a two-stage antibody procedure. The primary antibody is specific for hemoglobin. The secondary antibody is a conjugate of ¹²⁵I, and antibody against IgG of the animal in which the first antibody was raised. ¹²⁵I was then detected by autoradiography.

Phosphate-buffered saline (PBS, 0.9 M NaCl, 0.01 M phosphate, pH 7.6) solution (2 ml) was added to thawed yeast samples (0.02 g wet weight). The samples were centrifuged at 4°C for 10 min. and decanted. Cold disruption buffer (50 mM Tris, 5 mM EDTA, 0.5 mM PMSF, pH 8.0) prepared immediately before use (0.2 ml) was added, followed by enough ice-cold glass beads to just reach the top surface of the liquid. After vortexing for 30 seconds at maximum speed the samples were placed on ice for 5 min.; this step was repeated twice more. Ice-cold disruption buffer (1 ml) was added to each sample and the homogenate was transferred to an Eppendorf tube. In another Eppendorf tube, 200 µl of homogenate was combined with 200 µl of freshly prepared standard discontinuous 2X sample buffer (Laemli, 1970, Nature 227:680-685) and the sample was boiled for 10 min.

After centrifugation for 10 min., the samples were loaded onto a discontinuous denaturing gel in which the stacking gel was 3.75% acrylamide and the separating gel was 12%. The stacking gel was run at a constant current of 25 mA/cm² and the separating gel was run at a current of 33 mA/cm².

After the electrophoresis was complete and the dye band had reached the bottom of the separating gel, the gels were removed from the electrophoresis unit and the plates were pried apart under running deionized water. The stacking gel was discarded and the lower gel was separated from the plate. The transfer unit was filled with transfer buffer (2L methanol, 30.3 g Tris base, 144 g glycine, pH 8.30, in 10L distilled water), 2L of the transfer buffer was put into a shallow pan. The transfer sandwich consisting in sequence of large pore gauze, 3 M blotting paper, the gel, a piece of nitrocellulose paper precut to just cover the gel, 3M blotting paper, and another piece of large pore gauze was assembled under the buffer in the shallow pan.

Protein was then transferred from the gel to the nitrocellulose paper by applying a voltage of 40V for 1.5 hrs. After transfer was complete, the nitrocellulose sheet was removed and placed into a small, covered shallow pan with 50 ml blocking solution (20 g dried milk, 0.5 ml Nonidet-P40 (Sigma) in 1L PBS). The filter was agitated at 70 rpm for 45 min. The solution was replaced with 50 ml of fresh blocking solution and agitated for another 45 min. The blocking solution was discarded and 25 ml of fresh blocking solution was added. After adding 25 ml of primary antibody, the sample was agitated for 1.5 hrs. The solution was discarded and the filter was washed with 50 µl of fresh blocking solution (4 x 15 min). Afterwards, 25 ml blocking solution and 25 µl of secondary antibody was added. The solution was agitated and subsequently washed with 50 ml fresh blocking solution (6 x 10 min).

The following procedure was used to quantitate the amount of beta-globin present. Firstly, the globin present was detected by autoradiography. The autoradiograph was scanned using a laser densitometer and the quantity contained in each sample was estimated using the hemoglobin standard regression line. The standard used was apo-beta-globin purified by reverse phase HPLC from red blood cell lysate. The detection limit for the autoradiograph is about 2 ng. Approximately 0.09 mg Porto Alegre beta-globin per 100 mg yeast protein (0.09%) was detected.

### 7. EXAMPLE 2: EXPRESSION OF BETA-GLOBIN IN A YEAST EXPRESSION VECTOR CONTAINING A HYBRID PROMOTER AND ADH1 TRANSCRIPTION TERMINATION SEOUENCE

A hybrid promoter was constructed by the fusion of the upstream activating sequence of GAL1-10 promoter with the downstream promoter elements of the TDH3 promoter (referred to hereafter as the 3' end of the TDH3 promoter or TDH3-3'). The cassette containing the hybrid promoter + beta-globin gene + ADH1 terminator were excised and cloned into the yeast shuttle vector, YEp13. Yeast strain Sc340 was transformed with the resulting plasmid, pNML-VG-1 and the proteins expressed were analyzed by Western Blot Analysis.

### 7.1. MATERIALS

The restriction enzymes, Klenow enzyme and T4-DNA ligase were obtained from New England Biolabs (Biolabs), Bethesda Research Laboratories (BRL) or Boehringer Mannheim (BM). All enzymes were used according to the suppliers specifications. Plasmid DNA was isolated from a one liter culture of the transformed cells and purified by CsCl gradient centrifugation..

### 7.2. CONSTRUCTION OF PLASMID L19βt CONTAINING BETA-GLOBIN GENE AND THE ADH1 TERMINATOR

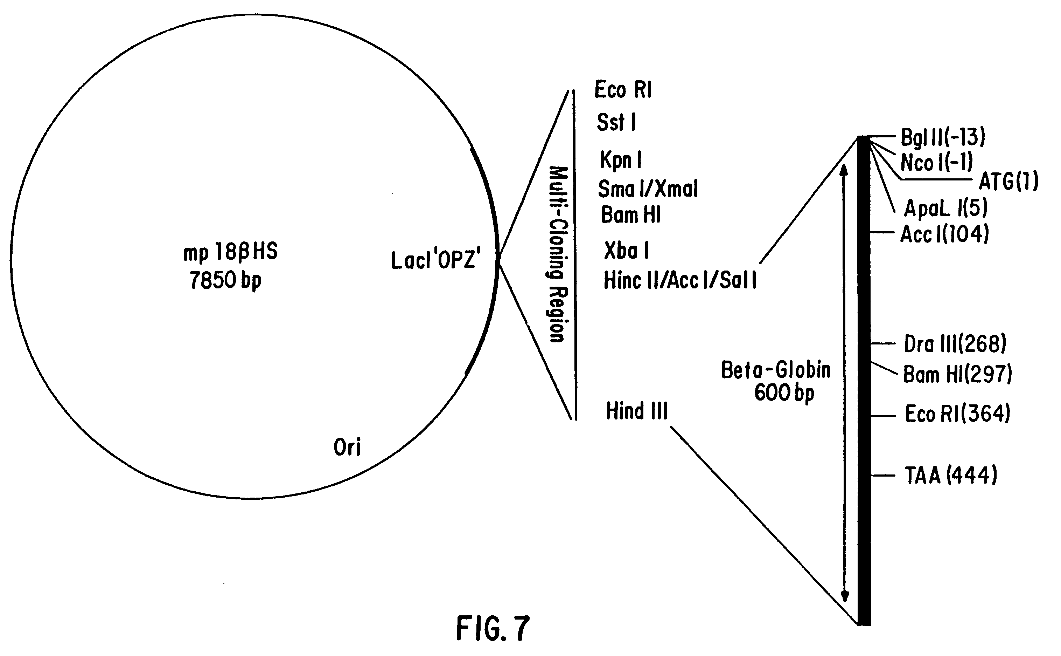

The beta-globin gene was obtained by digestion of plasmid mp18βHS with SalI and HindIII (see Figure 7 for map of mp18βHS). The 600 bp fragment was isolated by electroelution.

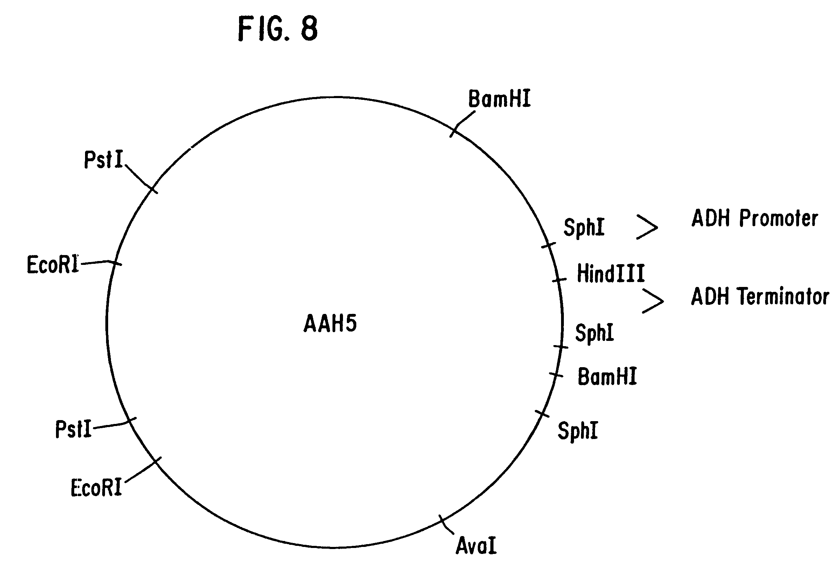

Plasmid AAH5 was digested with HindIII and BamH1 (Ammerer, G., Methods in Enzymology, 101, pp. 192-201, 1983.) AAH5 was obtained from Dr. Ben Hall at the University of Washington, Seattle (see Figure 8). The resulting 450 bp fragment was isolated by gel electrophoresis. Subsequently, the band containing the 450 bp fragment was precipitated with ethanol and digested with SphI. The 320 bp fragment (HindIII-SphI) containing ADH1 transcription termination sequences was isolated by electroelution.

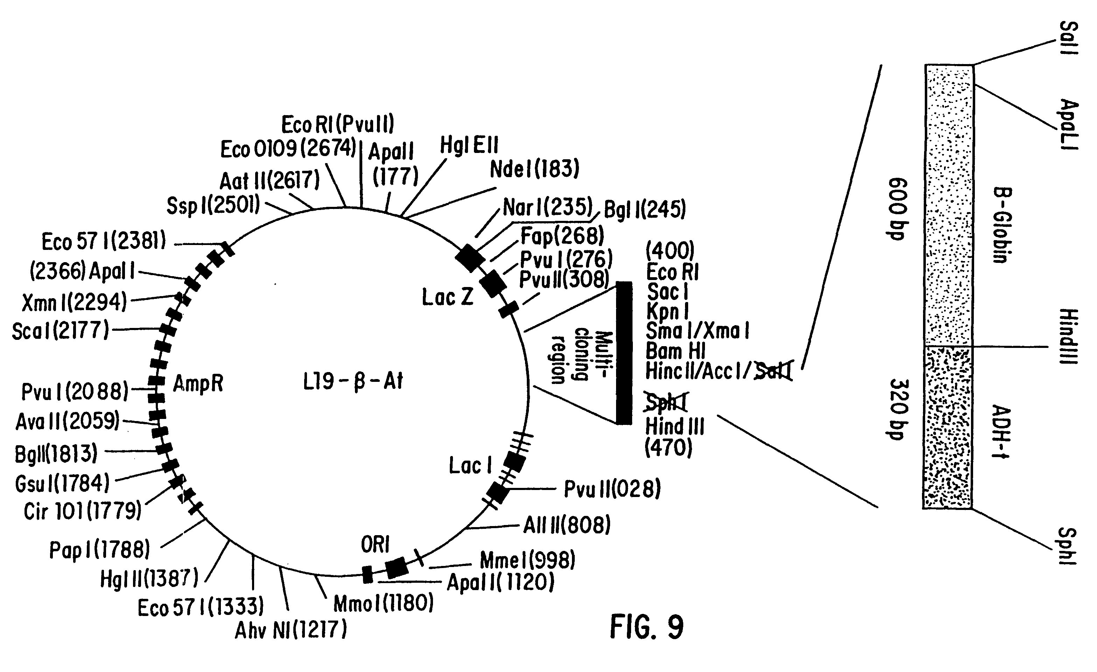

Plasmid pUC19 was cut with SalI and SphI. A. three way ligation reaction mixture was set up between the pUC19 fragment, the SalI-HindIII beta-globin fragment, and the HindIII-SphI ADH1 terminator fragment. The ligation was used for transforming competent E. coli cells (DH5α). The transformants were selected on ampicillin plates (100 mg/L). Plasmid DNA was isolated from twenty transformants (clones) and analyzed by restriction digestion with SalI-HindIII. The resulting plasmid containing the above two inserts in pUC19 was called L19βAt, and is shown in Figure 9. The DNA was digested with SphI and ApaLI and a 920 bp fragment containing the beta-globin gene and the ADH1 terminator was isolated by electroelution and used for the construction of the plasmid containing the TDH3 promoter, the beta-globin gene and the ADH1 terminator.

### 7.3. CONSTRUCTION OF PLASMID pUC19-HβAt

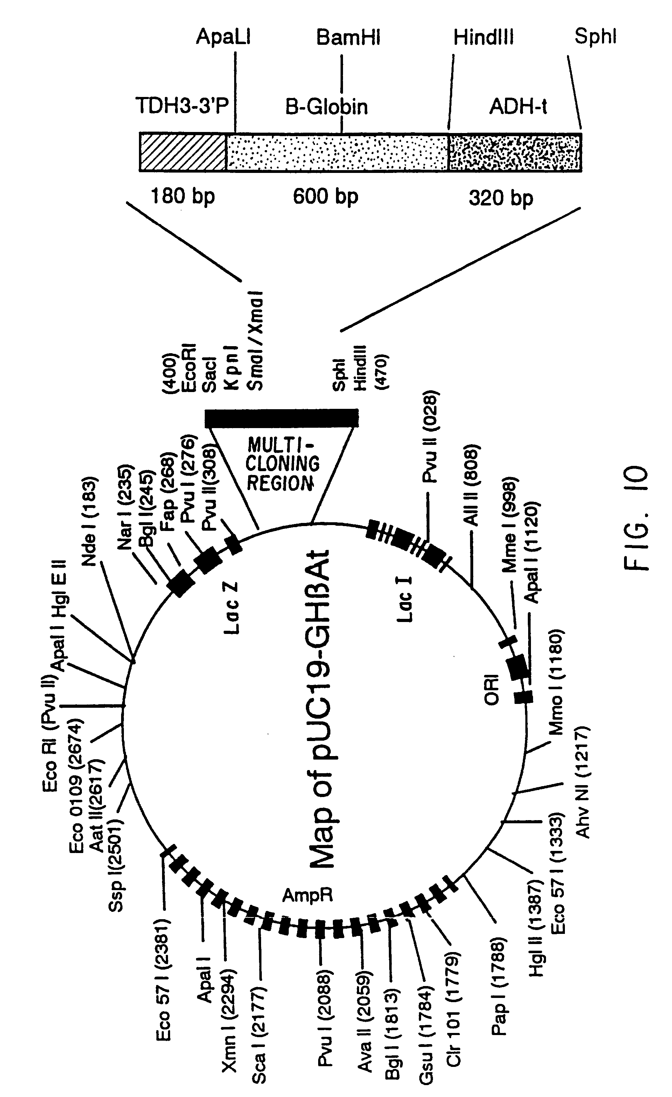

The TDH3-3' promoter fragment was synthesized by PCR using appropriate primers and template DNA from plasmid gp491. The following sequences were used as primers: 
-141                    -121
TDH3-3' 5' primer:  5'----ATcccgggAAGGTTGAAACCAGTTCCCTG---3'
SmaI
 
+28 +48
TDH3-3' 3' primer: 3'---GTGTGTATTTATTTGTTTTACcacgtgCGC---5'
ApaLI
 The 180 bp promoter fragment (TDH3-3') synthesized by PCR was digested with ApaLI and SmaI. The plasmid pUC19 was cut with SmaI and SphI. The DNA from plasmid L19βAt was cut with ApaLI and SphI and 920 bp fragment was isolated. Three way ligation was set between these three fragments. The transformation of E. coli DH5α cells was carried out as described earlier. The DNA isolated from the transformants were screened by restriction enzyme analysis with PvuII, ApaLI, and PvuII-HindII to check for the correct insert. The map of the resulting plasmid, pUC19-HβAt, is shown in Figure 10.

### 7.4. CLONING OF GAL1-10 UAS INTO pUC19-HβAt

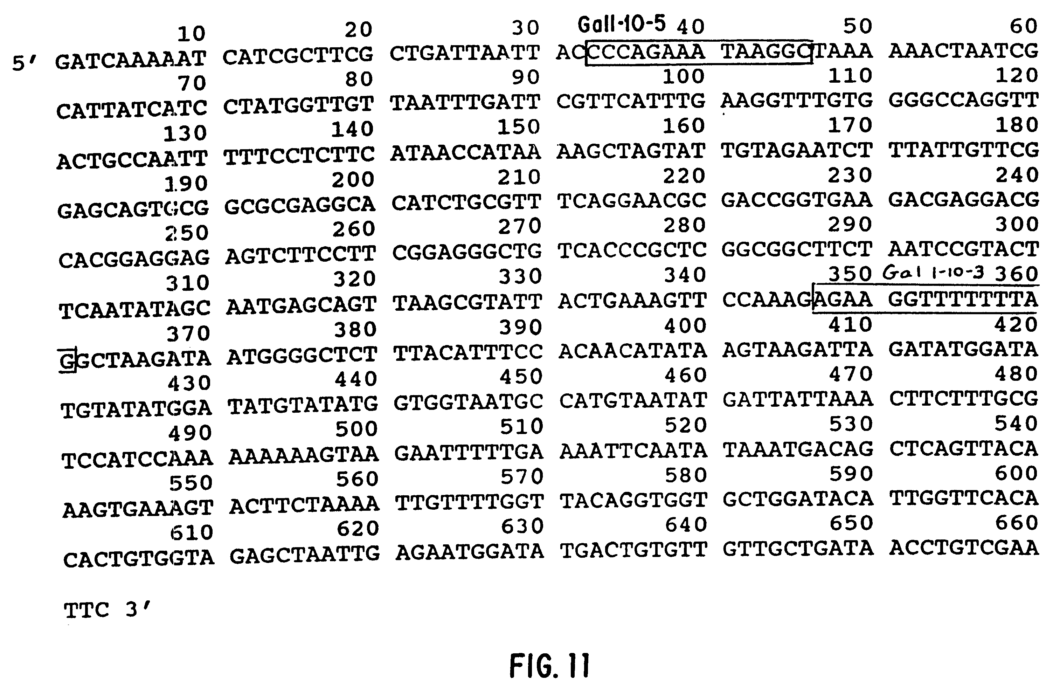

GAL1-10 upstream activator sequence (UAS), which is shown in Figure 11 was synthesized by polymerase chain reaction using GAL1-10-5' and GAL1-10-3' primers and DNA from YEp51 as a template. The sequences of these primers are shown below. The restriction sites SacI and SmaI were added to facilitate cloning.

+33 +46
Primer GAL1-10-5': 5'-TTgagctcCCCAGAAATAAGGC-3'
SacI

+347          +361
Primer GAL1-10-3':  3'-TCTTCCAAAAAAATCgggcccGT-5'
                                        SmaI

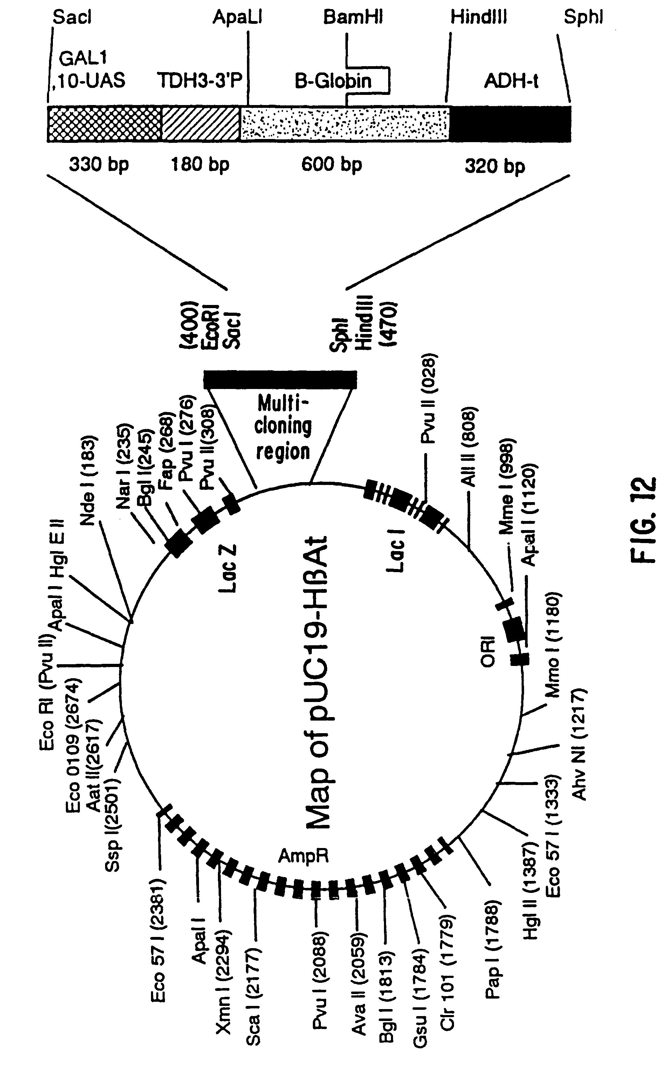

The GAL1-10 UAS PCR product was digested with SacI, blunt ended and cut with SmaI. It was cloned by blunt end ligation into SmaI digested pUC19-HβAt which contains the 3' end of the TDH3 promoter with the beta-globin gene and ADH1 terminator. The structure of the resulting plasmid, pUC19-GHβAt is shown in Figure 12. Transformation was carried out using E. coli DH5α cells. The DNA isolated from the transformants were screened by restriction enzyme analysis with PvuII, EcoRI, and HindIII to check for the correct insert.

### 7.5. CLONING OF THE HYBRID PROMOTER-BETA-GLOBIN GENE CASSETTE IN SHUTTLE VECTOR, YEp13

pUC19-GHβAt was digested with SacI-SphI to excise the GAL10-UAS + TDH3-3'+ beta-globin gene + ADH1-terminator cassette from pUC19 which was subsequently blunt-ended. The resulting 1.43 kb fragment was isolated by electroelution.

Plasmid YEp13 (obtained from Fred Winston, Harvard Medical School) which contains LEU2 (yeast) and Amp^{R} (E. coli) markers, was digested with BamHI and blunt-ended; the resulting linear DNA was isolated by electroelution.

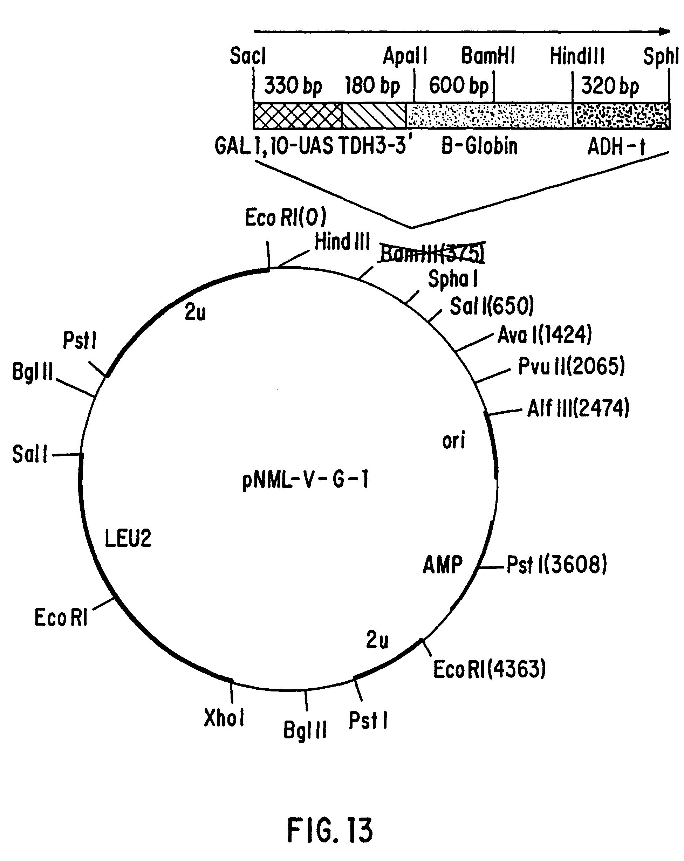

Ligation was set between the insert and the vector and the ligation mixture was used for transforming competent E. coli cells (DH5α). The transformants were selected on ampicillin plates (100 mg/L). The plasmid DNA was isolated from 24 transformants and analyzed by restriction digestion with HindIII, EcoRI, EcoRI/SalI. A map of the resulting plasmid, pNML-V-G-1 is shown in Figure 13.

### 7.6. TRANSFORMATION OF YEAST STRAIN Sc340 CELLS WITH pNML-V-G-1

Strain Sc340 has the following genotype: MATa, ura3-52, leu2, ade1, MEL+, [his3::GAL10 (UAS+P) + GAL4 + URA3]. Yeast strain Sc340 was transformed with plasmid pNML-V-G-1 using the spheroplast procedure (Rose, M. et al., 1989, Methods in Yeast Genetics, Cold Spring Harbor Laboratory, Cold Spring Harbor, N.Y., pp 112-115). To minimize background in the control plates and increase efficiency of transformation, the regeneration media contained 1 M sorbitol, 10 mM CaCl₂, 0.1% yeast nitrogen base, and 2% glucose. The medium was filter sterilized. The plating media was prepared by mixing 182 g sorbitol, 20 g agar, 6.7 g Difco YNB without amino acids, glucose, required amino acids except leucine in 1 L distilled water. The top agar was made by mixing 18.2 g sorbitol, 2 g agar, 0.67 g Difco YNB without amino acids, 2 g glucose and required amino acids in 100 ml distilled water.

For the starter culture, cells were grown overnight in minimal media containing 0.67% yeast nitrogen base, 0.5% glucose, and supplemented with uracil, adenine, and histidine. 500 ml of SD media supplemented with 200 µM ferric citrate and 20 mg/L each of adenine, uracil, and histidine was inoculated with the starter culture to an OD₆₀₀ of 0.02. The culture was incubated with shaking (300 rpm) at 30°C, and was induced with 2% galactose for a period of 4 hours before sampling for analysis.

### 7.7. WESTERN BLOT ANALYSIS OF EXPRESSED BETA-GLOBIN

The expressed beta-globin was quantitated by Western Blot analysis using procedures described in Section 6.6. supra. The results indicated that up to 5.4% of the total yeast protein expressed in transformed Sc340 cells was beta-globin.

### 8. EXAMPLE 3: EXPRESSION OF NATURAL GAMMA-GLOBIN IN A YEAST EXPRESSION VECTOR CONTAINING GAL10 PROMOTER AND ADH1 TERMINATOR

The gamma-globin gene was obtained from plasmid pJW151 using PCR. The gamma-globin gene was modified by PCR to have a SalI site at the 5'-end and a HindIII site at the 3'-end. The modified gamma-globin gene was cloned into the yeast expression vector YEp51T/NAT, which contains the ADH1 transcription termination sequence, the GAL10 promoter, and the DNA sequence encoding the beta-globin gene. YEp51T/NAT had been cut with SalI and HindIII to remove the beta-globin gene. The plasmid containing the gamma-globin gene was called YEp51T/G. Yeast strain Sc340 was transformed with YEp51T/G and the transformant was called 340g2G. Following growth of 340g2G and induction by galactose, expressed proteins were analyzed by Western blot analysis. The results from Western blot analysis indicated that gamma-globin was expressed.

### 8.1. MATERIALS

Restriction and DNA modifying enzymes were obtained from Boehringer-Mannheim, Bethesda Research Laboratories, Perkin-Elmer or New England Biolabs. All enzymes were used according to the suppliers' specifications.

The E. coli strain used for all bacterial transformations was DH5α.

Oligonucleotides were synthesized on the Applied Biosystem Inc.'s DNA synthesizer 380B using Cyanoethyl chemistry. Polymerase Chain Reaction (PCR or PC reaction) was carried out in a DNA thermal cycler obtained from Cetus, Inc.

### 8.2. CLONING OF THE BETA-GLOBIN GENE INTO THE YEAST EXPRESSION-VECTOR YEp51

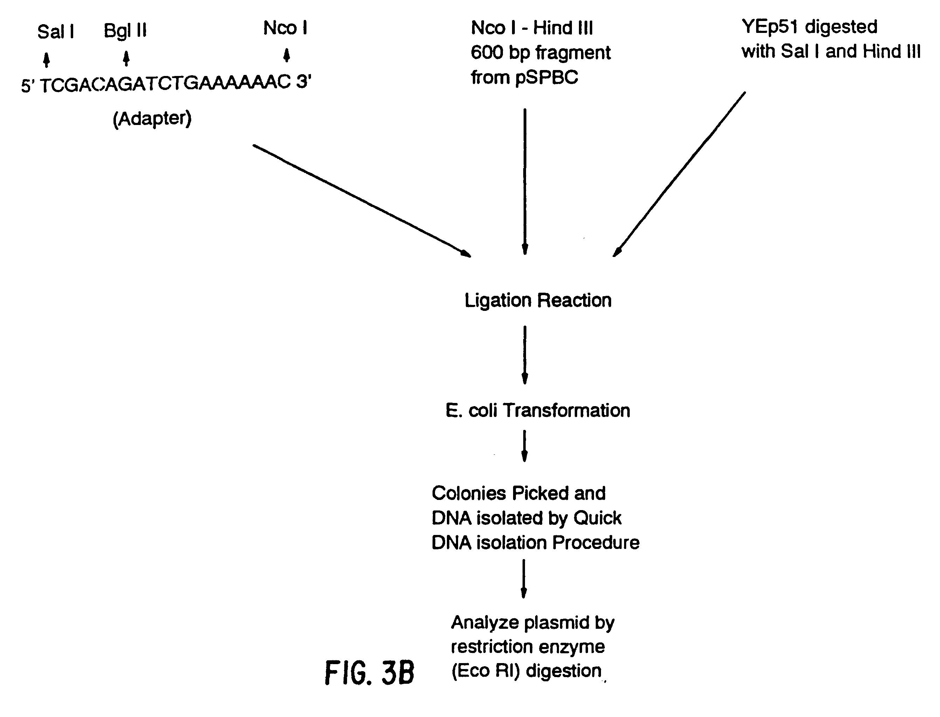

The general procedure used to clone the beta-globin gene into the yeast expression vector YEp51 is shown in Figure 3B. The plasmid pSPβC (see Figure 2 for restriction map of pSBβC) was digested with NcoI and HindIII. Digestion with this combination of enzymes generated two fragments, a 600 base pair DNA containing the beta-globin gene and a 2700 bp fragment from the plasmid. The 600 bp fragment was isolated from a 0.6% agarose gel. After the band was excised from the gel, the DNA was electroeluted, and ethanol precipitated. The precipitated DNA was spun in an Eppendorf Centrifuge, the supernatant was removed and the DNA pellet was dried under vacuum.

The 600 bp fragment was modified by adapter addition before cloning into the plasmid YEp51. The DNA fragment carrying the beta-globin gene isolated from pSBβC was NcoI compatible at the 5'-end while the 3'-end was HindIII compatible. These ends had to be modified so that they could be compatible with the restriction sites present in YEp51. To modify the 5'-end of the isolated fragment, a synthetic adapter was used. This adapter had a NcoI compatible end at its 3'-end and a SalI compatible end at its 5'-end (see Figure 2). The 3'-end of the isolated fragment did not receive any adapter as the HindIII site was compatible with the HindIII site introduced into the YEp51.

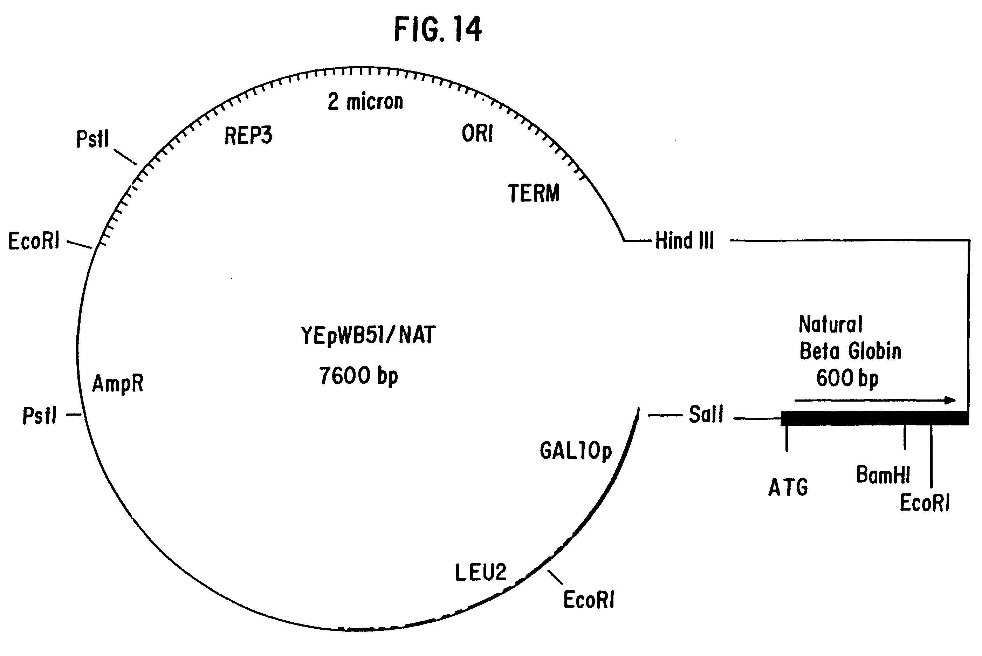

The recipient plasmid YEp51 was cleaved with SalI and HindIII restriction enzymes. To insert the isolated fragment containing the beta-globin gene, a three-way ligation was set up (see Figure 3B). The ligation reaction was carried out according to the standard ligation procedures (Maniatis et al., 1982, Molecular Cloning, A Laboratory Manual, Cold Spring Harbor Laboratory, Cold Spring Harbor, New York). The ligation mixture was transformed into the E. coli HB101 cells using standard transformation procedure. Cells were spread on plates containing LB-media with 100 mg/L ampicillin. Plates were incubated overnight at 37°C. Forty eight colonies from the ampicillin plates were picked and a 5 ml culture was inoculated with individual transformants. Cultures were grown overnight at 37°C with vigorous shaking. The plasmid DNA was isolated from 1.5 ml of the overnight culture using quick plasmid isolation procedure. The plasmid from each transformant was digested with EcoRI to confirm the presence of a DNA fragment containing natural beta-globin gene. The plasmid carrying the natural beta-globin gene was called YEpWB51/Nat. The map of the plasmid YEpWB51/Nat is shown in Figure 14.

### 8.3. CLONING OF THE ADH1-TERMINATOR SEQUENCES INTO YEpWB51/NAT

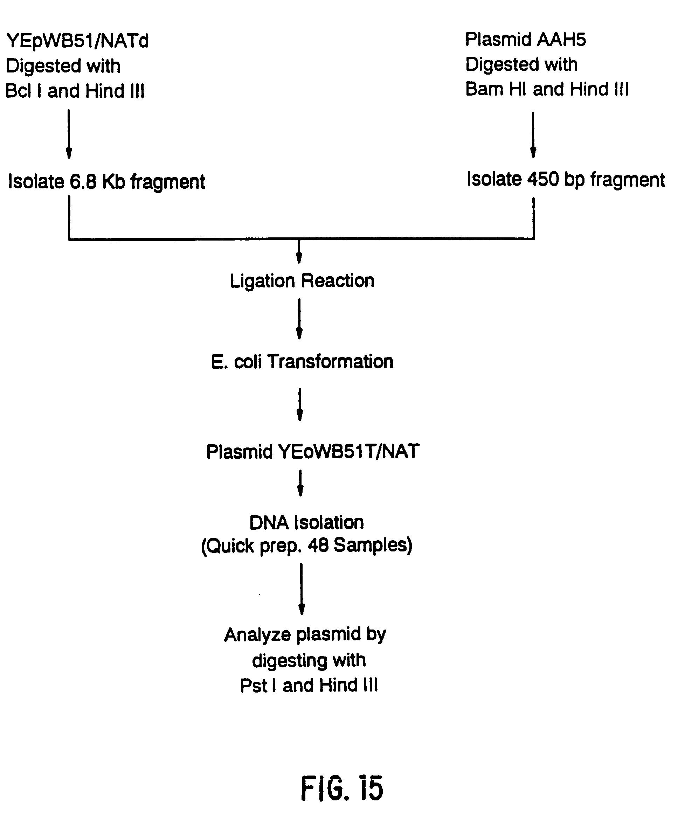

The strategy used to insert ADH1 terminator sequences into YEpWB51/NAT is shown in Figure 15. The plasmid YEpWB51/NATd⁻ (d⁻=dam⁻ and dcm⁻, i.e. methylation minus) was digested with restriction enzymes BclI and HindIII. After the digestion, a 6.8 kb DNA fragment containing the beta-globin gene and vector was isolated from a 0.6% agarose gel (in 1X TBE, 0.1 M Tris, pH 8.0, 0.09 M boric acid, 1. mM EDTA). DNA was electroeluted from the gel slice and precipitated with ethanol at -20°C. The precipitated DNA was spun in an Eppendorf Centrifuge for 15 min and the pellet was dried under vacuum. The DNA was suspended in 20 ml H₂O.

The ADH1-transcription termination sequences were isolated from plasmid AAH5 (Ammerer, G., 1983, Methods in Enzymology, 101, pp 192-201). AAH5 was obtained from Dr. Ben Hall at the University of Washington, Seattle. The plasmid AAH5 was digested with BamHI and HindIII (see Figure 8 for a map of plasmid AAH5). Digestion with this combination of enzymes generated three fragments. A 450 base pair DNA fragment containing the ADH1-transcription termination sequence was isolated from the 0.6% agarose gel. DNA was electroeluted from the gel slice and precipitated with ethanol at -20°C. The precipitated DNA was spun in an Eppendorf Centrifuge for 15 min and the pellet was dried under vacuum. The DNA was suspended in 20 µl H₂O.

The DNA fragment carrying the ADH1-transcription terminator isolated from AAH5 was BamHI compatible at the 3'-end while the 5'-end was HindIII compatible. These ends were compatible with the restriction sites present in YEpWB51/NAT.

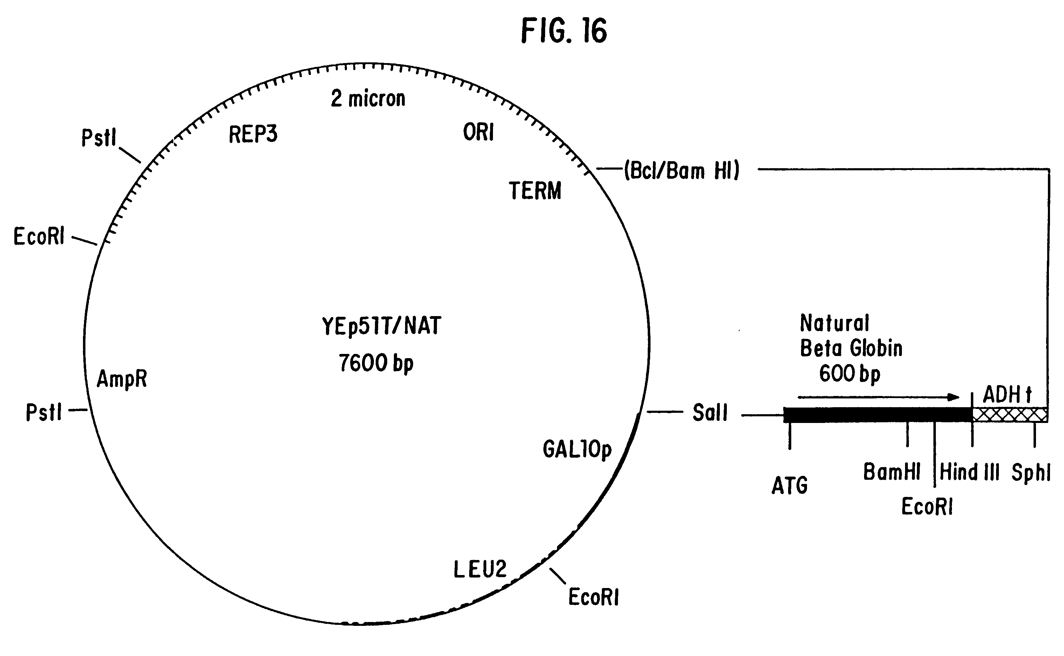

The recipient plasmid YEpWB51/NATd⁻ was cleaved with BclI and HindIII restriction enzymes. As shown in Figure 15, a two-way ligation was set up to insert the isolated fragment. The ligation mixture was transformed into E. coli HB101 cells using standard transformation procedures. Cells were spread on plates containing LB-media with 100 mg/L ampicillin. Plates were incubated overnight at 37°C. Twenty four colonies from the ampicillin plates were picked and a 5 ml culture was inoculated with individual transformants. Cultures were grown overnight at 37°C with vigorous shaking. The plasmid DNA was isolated from 1.5 ml of the overnight culture using standard alkaline miniprep procedures (Maniatis et al., 1982, Molecular Cloning, A Laboratory Manual, Cold Spring Harbor Laboratory, Cold Spring Harbor, New York). The plasmid from each transformant was digested with PstI and HindIII restriction enzyme to confirm the presence of a DNA fragment containing the ADH1-terminator. The plasmid carrying the natural beta-globin gene with the ADH1-terminator was called YEp51T/NAT and is shown in Figure 16.

### 8.4. CLONING OF THE GAMMA-GLOBIN GENE INTO THE YEAST EXPRESSION VECTOR YEp51T/NAT

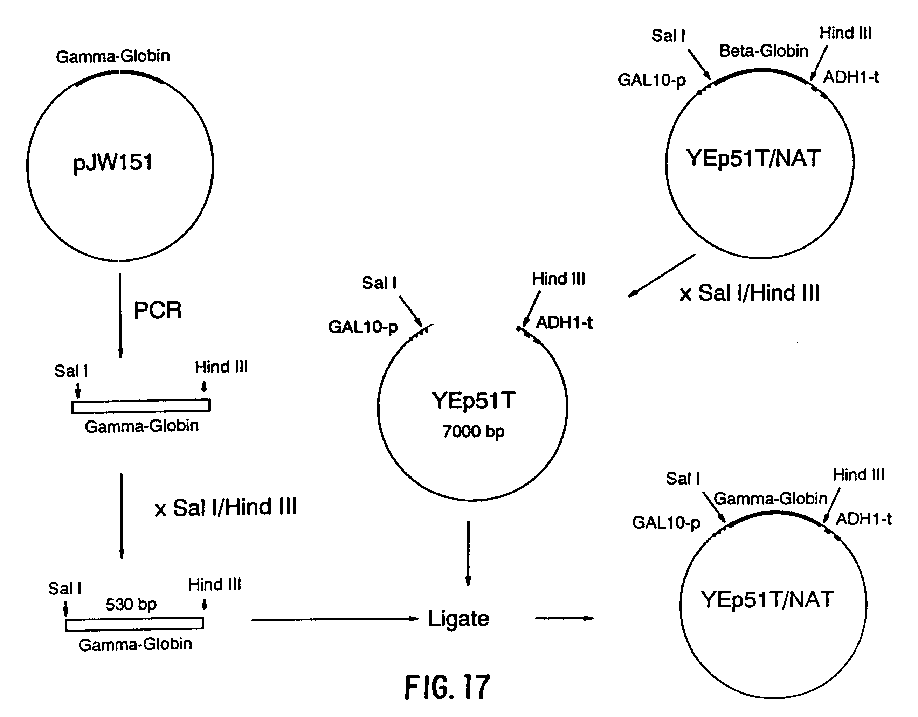

The general procedure used to clone the gamma-globin gene into the yeast expression vector YEp51T/NAT resulting in the construction of YEp51T/G is shown in Figure 17.

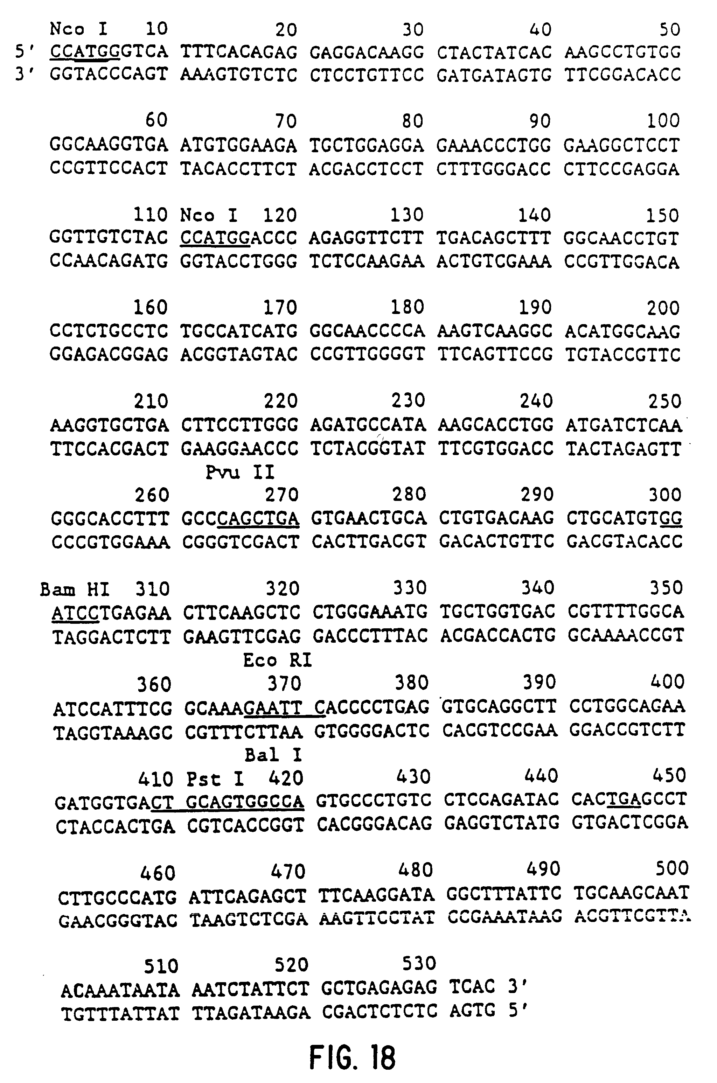

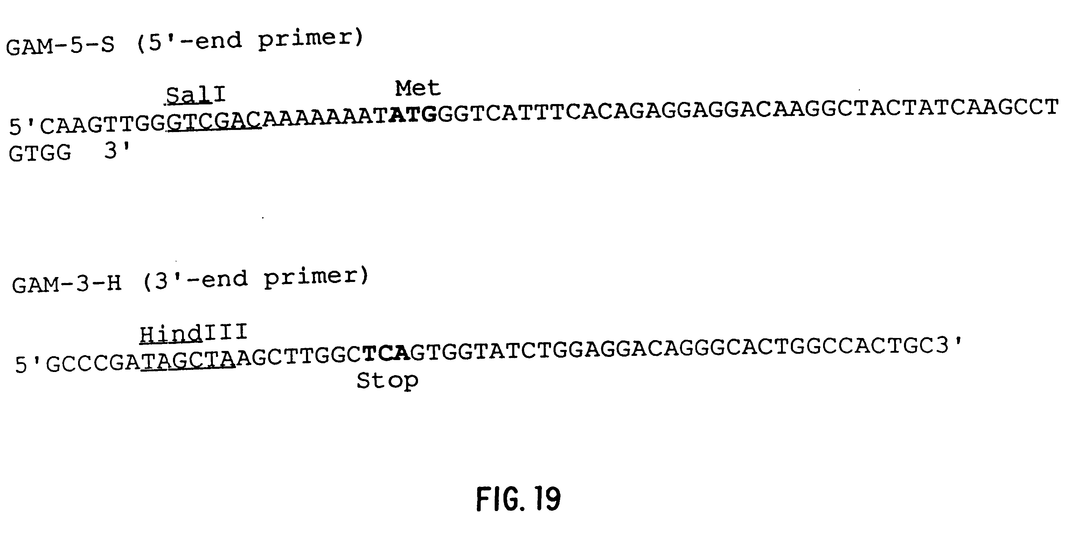

The gamma-globin gene was synthesized by PCR using appropriate primers (shown below) and plasmid pJW151 DNA (Wilson, J.T., et al., Nucleic Acid Research, 5:563-581, 1978) as template. The sequence of gamma-globin DNA is shown in Figure 18. The 5' and 3' primers used for synthesizing the gene are shown in Figure 19. The PCR product was analyzed by electrophoresis in a 1.5% agarose gel (in 1X TBE). The 530 bp PCR product was removed from the gel by electroelution and the DNA was precipitated with ethanol. The purified PCR product was then digested with restriction enzymes SalI (5'-end) and HindIII (3'-end). The digested PCR product was phenol extracted and ethanol precipitated.

Plasmid YEp51T/NAT which contains the human beta-globin gene was digested with SalI and HindIII to remove the beta-globin gene. The digested plasmid was electrophoresed in 0.6% agarose gel (in 1X TBE). A 7000 bp fragment was electro-eluted and ethanol precipitated.

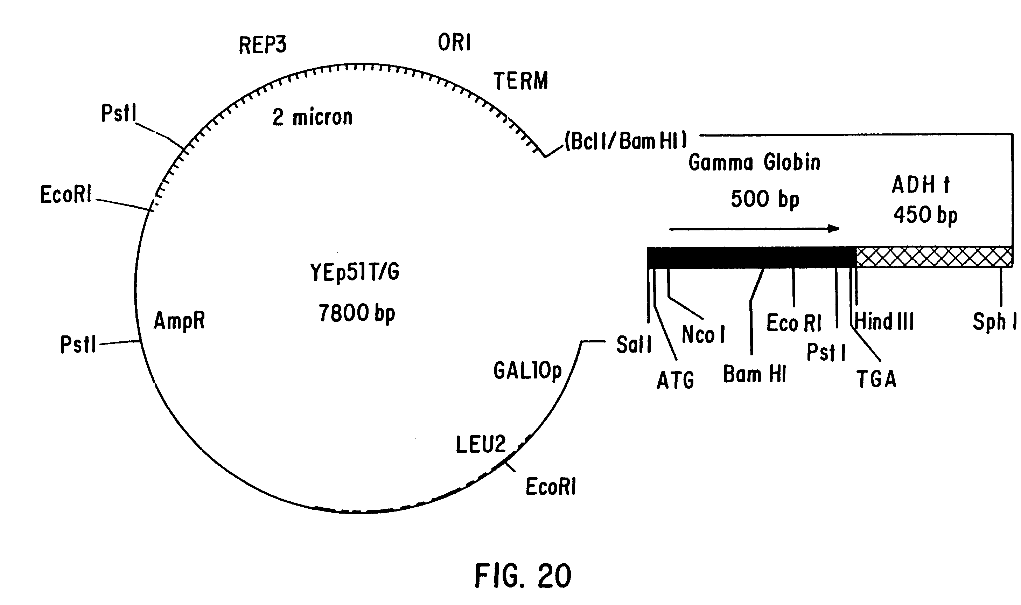

A ligation reaction mixture was set up between the gamma-globin obtained by digestion of the PCR product described above and YEp51T/NAT cut with SalI and HindIII (7000 bp). The ligation mixture was used to transform E. coli DH5α cells using standard transformation procedure and plated on LB plates containing Ampicillin (100 mg/L). Plasmid DNA was isolated from 20 clones and digested with restriction enzyme PstI. The resulting plasmid was called YEp51T/G (Figure 20).

### 8.5. TRANSFORMATION AND GROWTH OF YEAST STRAIN Sc340 CELLS WITH PLASMID YEp51T/G

Yeast strain Sc340 cells were transformed with plasmid YEp51T/G (Rose, et al., 1989, Methods in Yeast Genetics, Cold Spring Harbor Laboratory, Cold Spring Harbor, N.Y., pp. 112-115). The starter culture was grown in SD supplemented with adenine and histidine, and 3% glycerol and 2% lactate as carbon source. The preculture was used to inoculate 2 L of the above media in a Braun Biostat E fermentor. The pH was maintained at 5.5 using a 5% ammonium hydroxide solution. The pO₂ was maintained at 80% until the culture was induced with galactose at which point it was lowered to 10%. The stirrer speed was set at 500 rpm and then reduced to 100 rpm at galactose induction. The culture was incubated at 30°C and grown to an O.D. 600 of 30.4 at which time it was induced with galactose added at the rate of 5g/L/hour. Samples were collected from 0 to 74 hours after induction for globin analysis.

### 8.6. WESTERN BLOT ANALYSTS OF EXPRESSED GAMMA-GLOBIN

The expressed gamma-globin was quantitated by Western Blot analysis using procedures described in Section 6.6., supra. The results indicated that up to 0.05% of the total yeast protein in yeast cell line 340g2G was gamma-globin.

### 9. EXAMPLE 4: EXPRESSION OF GAMMA-GLOBIN VARIANT IN A YEAST EXPRESSION VECTOR CONTAINING A HYBRID PROMOTER AND TRANSCRIPTION TERMINATION SEOUENCE

A hybrid promoter was constructed by the fusion of the upstream activating sequence of GAL10 promoter with the downstream promoter elements of the TDH3 promoter (referred to hereafter as the 3' end of the TDH3 promoter or TDH3-3'). A gamma globin variant gene was constructed by changing the sequence of the 5'-end primer for PCR and replacing the penultimate glycine codon with one for valine, therebv creating an ApaLI site. The cassette containing the hybrid promoter+gamma-globin gene+ADH1 terminator was constructed in pUC19 and then excised and cloned into the yeast shuttle vector, YEp51. Yeast Sc340 cells were transformed with the resulting plasmid, pNM-5Gγᵥₐₗ1 and the proteins expressed by the transformants following galactose induction were analyzed by Western Blot.

### 9.1. MATERIALS

The restriction enzymes, Klenow enzyme and T4-DNA ligase were obtained from New England Biolabs (Biolabs), Bethesda Research Laboratories (BRL) or Boehringer Mannheim (BM). All enzymes were used according to the suppliers specifications. Plasmid DNA was isolated from a one liter culture of the transformed cells and purified by CsCl gradient centrifugation.

### 9.2. SYNTHESIS OF GAMMA(val)At

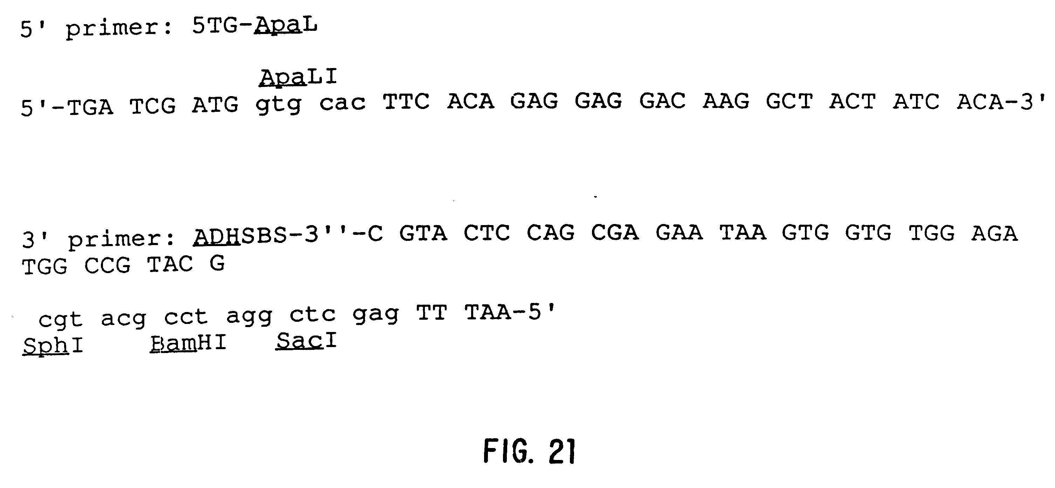

Using appropriate primers and DNA from plasmid YEp51T/G (Section 8.4., supra) as template, a DNA fragment containing γ-globin upstream of the ADH1 terminator was synthesized by PCR. The ApaLI site was added to the γ-globin gene by altering the sequences ggtcat in the gene to gtgcac (ApaLI site). The 5' and 3' primers used for synthesizing the gene are shown in Figure 21. Using the 5' γ-globin primer and a primer complementary to the 3' ADH1 terminator sequences, and DNA from the plasmid YE51T/G as template, a 780 bp fragment containing γᵥₐₗ-globin gene and ADH1 terminator was synthesized by PCR.

### 9.3. CONSTRUCTION OF pUC19-GHγ(val)At

The gamma (val) PCR fragment was cut with ApaLI and SphI (fragment #1). The DNA from pUC19 plasmid was cut with SacI/SphI and linear DNA fragment was isolated (fragment #2). The DNA from pUC19-GHβAt (Section 8.3., supra) was cut with ApaLI and SacI, and the 510bp fragment containing GAL-UAS-TDH3-3' hybrid promoter was isolated by electroelution (fragment #3).

A three way ligation was set between the above three fragments. E. coli DH5α cells were used for transformation. The DNA from 24 transformants was isolated by alkaline digestions and analyzed by restriction digestions. The resulting plasmid containing all three fragments in correct orientation was labeled pUC19-GHγᵥₐₗAt.

### 9.4. CLONING OF GHγᵥₐₗAt CASSETTE INTO YEAST SHUTTLE VECTOR YEp51

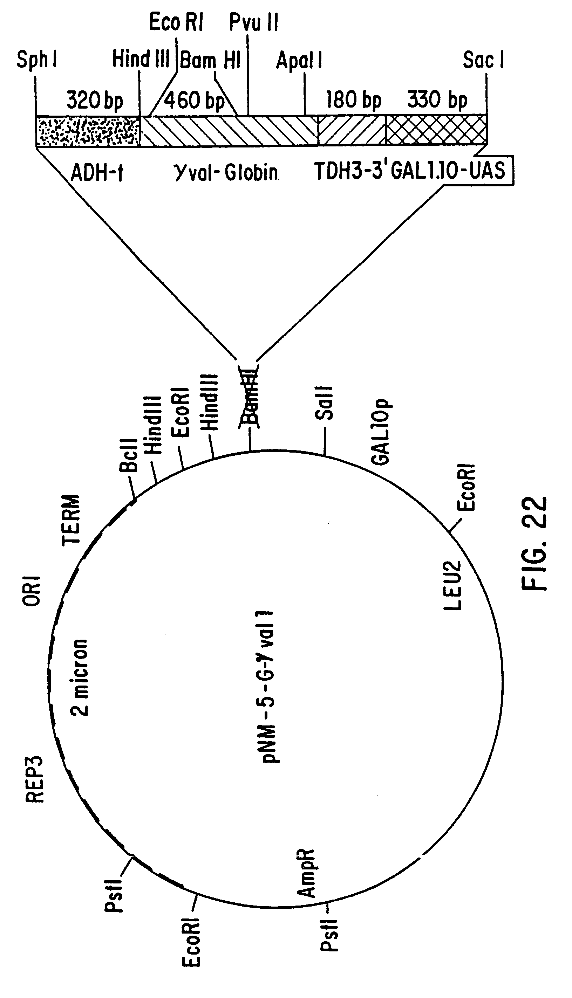

The expression cassette GHγᵥₐₗAt (1.29 kb) was isolated by digestion of pUC19-GHγᵥₐₗAt with SacI/SphI. The cassette was blunt ended and cloned into BamHI-cut YEp51 by blunt end ligation. DNA samples from forty eight clones from each transformation were screened with ApaLI digestion. The positive clones showed four fragments and the negative clones showed three fragments. The positive clones were further characterized by digestion with different enzymes. The positive clone was labeled pNM-5-G-γᵥₐₗ1. Figure 22 shows the map of plasmid pNM-5-G-γᵥₐₗ1.

### 9.5. TRANSFORMATION AND GROWTH OF YEAST STRAIN Sc340 WITH PLASMID pNM-5-G-γᵥₐₗ1

Plasmid pNM-5-G-γᵥₐₗ1 DNA was used to transform yeast Sc340 cells (Rose et al., 1989, Methods in Yeast Genetics, Cold Spring Harbor Laboratory, Cold Spring Harbor, N.Y., pp. 112-115). The transformant was named 340-5GgVAL. The starter culture was grown at 30°C overnight in medium containing 0.5% raffinose, 0.67% yeast nitrogen base without amino acids (Difco), and supplemented with 20 mg/L each of adenine, uracil, histidine, and tryptophan and was used to inoculate medium containing 0.67% yeast nitrogen base without amino acids, 3% glycerol, 2% lactic acid, and supplemented with 20 mg/L each of uracil, histidine, adenine, and tryptophan. After 24 hours growth at 30°C the culture was induced by the addition of galactose to a final concentration of 2%. Samples were collected from 4 to 48 hours after induction for globin analysis by Western Blot.

### 9.6. WESTERN BLOT ANALYSIS OF EXPRESSED GAMMA-GLOBIN

The expressed gamma-globin was quantitated by Western Blot analysis using procedures described in section 6.6., supra. The results indicated that up to 1% of the total yeast protein in 340-5GgVAL was gamma(val)-globin.

### 10. EXAMPLE 5: EXPRESSION OF ALPHA-GLOBIN IN A YEAST EXPRESSION VECTOR CONTAINING A HYBRID PROMOTER AND ADH1 TRANSCRIPTION TERMINATION SEOUENCE

A hybrid promoter was constructed by the fusion of the upstream activating sequence of ADH2 promoter with the downstream promoter elements of the TDH3 promoter (referred to hereafter as the 3'end of the TDH3 promoter or TDH3-3'). Specifically, the ADH2-UAS-TDH3-3' hybrid promoter, alpha-globin gene, and GAL10 terminator were cloned into plasmid pUC19. The resulting plasmid was labeled pUC19-AHαGt. The cassette containing the hybrid promoter+alpha-globin gene+GAL10 terminator was excised and cloned into the yeast shuttle vector, pPM40. Yeast strain Sc1012 was transformed with the resulting plasmid, pNM-R-A-α1 and the proteins expressed were analyzed by Western Blot Analysis.

### 10.1. MATERIALS

The restriction enzymes, Klenow enzyme and T4-DNA ligase were obtained from New England Biolabs (Biolabs), Bethesda Research Laboratories (BRL) or Boehringer Mannheim (BM). All enzymes were used according to the suppliers' specifications. Plasmid DNA was isolated from a one liter culture of the transformed E. coli cells and purified by CsCl gradient centrifugation.

### 10.2. CONSTRUCTION OF pUC19-GHαGt

### 10.2.1. CONSTRUCTION OF PLASMID p19A1

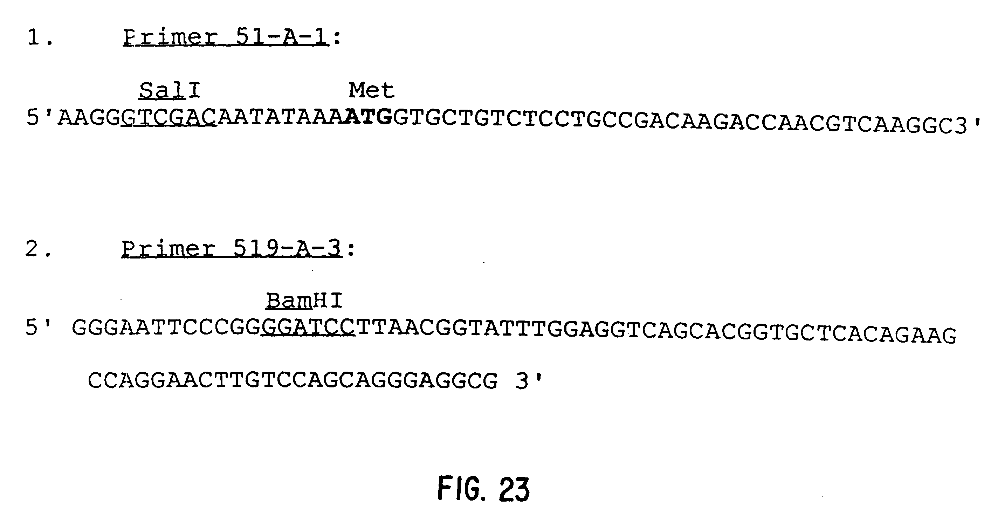

To construct plasmid p19A1, the alpha-globin gene was obtained by Polymerase Chain Reaction (PCR). Template used for the PCR was plasmid pJW101 (Wilson et al., 1978, Nucl, Acids Res. 5: 563-580) and primers used in the reaction were 51-A-1 and 519-A-3 (see Figure 23).

The PCR product was digested with SalI and BamHI restriction enzymes, and the 460 bp fragment was isolated from a 0.6% agarose gel (1X TBE). DNA was electroeluted from the gel slice and was precipitated with ethanol at-20°C. The precipitated DNA was spun in an Eppendorf centrifuge for 15 minutes. The pellet was dried under vacuum. The DNA was suspended in 20°C water. Purified PCR product was ligated to plasmid pUC19 digested with restriction enzymes SalI and BamHI. The ligation reaction was carried out at 15°C according to standard ligation procedures (Maniatis et al., 1982, Molecular Cloning, A Laboratory Manual, Cold Spring Harbor Laboratory, Cold Spring Harbor, New York). Ligation mixture was used to transform E. coli DH5α cells using standard transformation procedures. The cells were spread on plates containing LB-media with 100 mg/l ampicillin. Plates were incubated overnight at 37°C. Twenty-four colonies from ampicillin plates were picked and a 5 ml culture was inoculated with individual transformants. Cultures were grown overnight at 37°C with vigorous shaking. The plasmid DNA was isolated from 1.5 ml culture and DNA was digested with restriction enzyme HindIII. Eight clones showed the expected length of fragments (2900 bp and 200 bp). The resulting plasmid was called p19A1.

### 10.2.2. CONSTRUCTION OF PLASMID p19A1GT

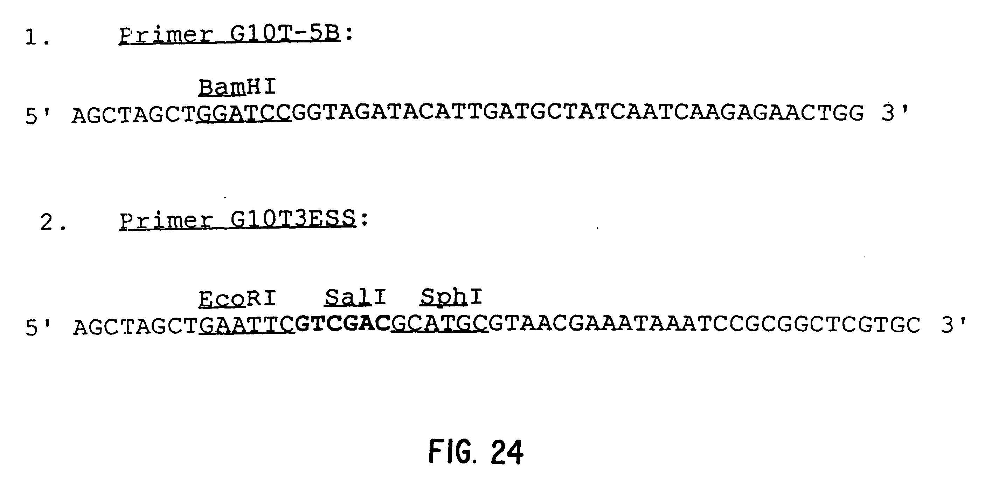

To construct plasmid p19A1GT, the GAL10 terminator was obtained by Polymerase Chain Reaction (PCR). Template used for the PCR was plasmid pCLUI and the primers used in the reaction, G10T-5B, and G10T3ESS are shown in Figure 24.

The PCR product was digested with BamHI and EcoRI restriction enzymes and 450 bp fragment was isolated from a 0.6% agarose gel (1X TBE). DNA was electroeluted from the gel slice and was precipitated with ethanol at-20°C. The precipitated DNA was spun in an Eppendorf Centrifuge for 15 minutes. The pellet was dried under vacuum. The DNA was suspended in 20°C water. Purified PCR product was ligated to plasmid p19A1 digested with restriction enzymes BamHI and EcoRI. The ligation reaction was carried out at 15°C according to standard ligation procedures (Maniatis et al., 1982, Molecular cloning, A Laboratory Manual, Cold Spring Harbor Laboratory, Cold Spring Harbor, New York). Ligation mixture was used to transform E. coli DH5α cells using standard transformation procedures. The cells were spread on plates containing LB-media with 100 mg/l ampicillin. Plates were incubated overnight at 37°C. Twenty-four colonies from ampicillin plates were picked and a 5 ml culture was inoculated with individual transformants. Cultures were grown overnight at 37°C with vigorous shaking. The plasmid DNA was isolated from 1.5 ml culture and DNA was digested with restriction enzyme SalI. Eight clones showed the expected length of fragments (2700 bp and 900 bp). The resulting plasmid was called p19A1GT.

### 10.2.3. CONSTRUCTION OF PLASMID pUC19-GHαGt

PCR was used to synthesize a hybrid promoter fragment, GHαGt, which contains the hybrid promoter, GAL1-10 UAS/TDH3-3' and the first 36 bases of the alpha-globin gene up to and including the StuI site in the gene. The resulting 570 bp PCR fragment was cloned into SmaI cut pUC19 plasmid by blunt end ligation. The transformation was carried out using E. ccli strain 510α.

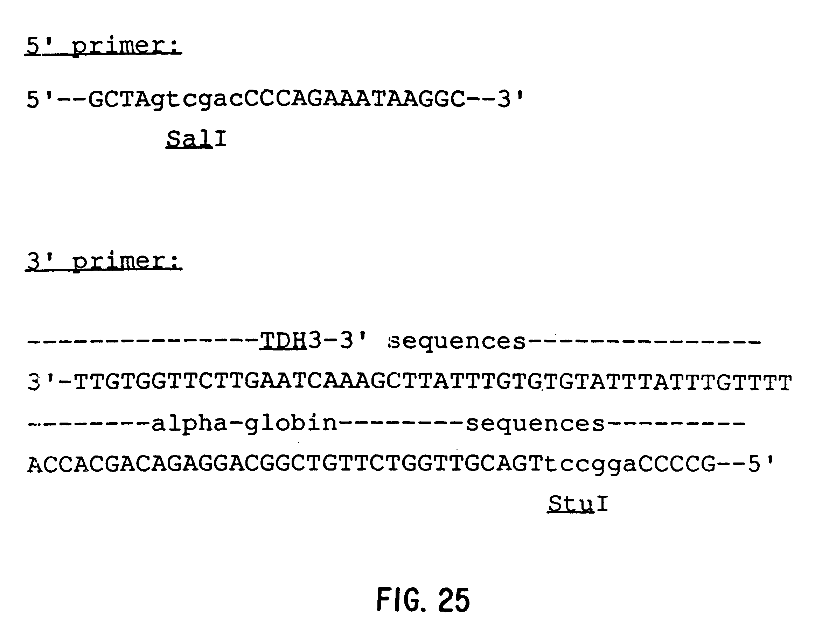

The first restriction site in the alpha-globin coding sequence is StuI which begins 31 bases downstream from the ATG initiation codon. The 3' primer was synthesized to contain sequences complementary to the 3' end of the TDH3-3' promoter and first 36 bases of alpha-globin gene up to the StuI site. The 5' and 3' primers are described in Figure 25. The template used for the PCR was DNA from plasmid pUC19-GHβAt, which contains GAL1-10 UAS/TDH3-3' hybrid promoter.

The 570 bp fragment, containing the hybrid promoter-first 36 bases of alpha-globin, synthesized using the above primers, was cloned into the vector, pUC19 by blunt end ligation. This plasmid was labeled pUC19-GHα'.

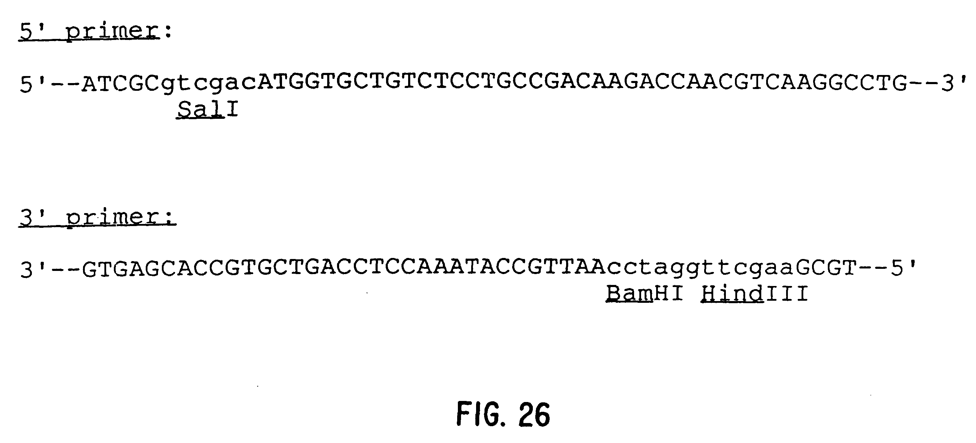

The 3' alpha globin gene fragment containing appropriate cloning sites was made by PCR. Plasmid pUC19-A1 was used as template. The 5' and 3' primers used to synthesize the gene are described in Figure 26.

The resulting 460 bp fragment was cut with BamHI and SalI and cloned into pUC19 cut with BamHI-SalI. The E. coli 510α strain was used for transformation because this strain produces unmethylated DNA. StuI enzyme is inefficient in cutting methylated DNA. The transformants were analyzed by digestion with PvuII and the resulting plasmid containing the appropriate fragment was labeled pUC19-A2.

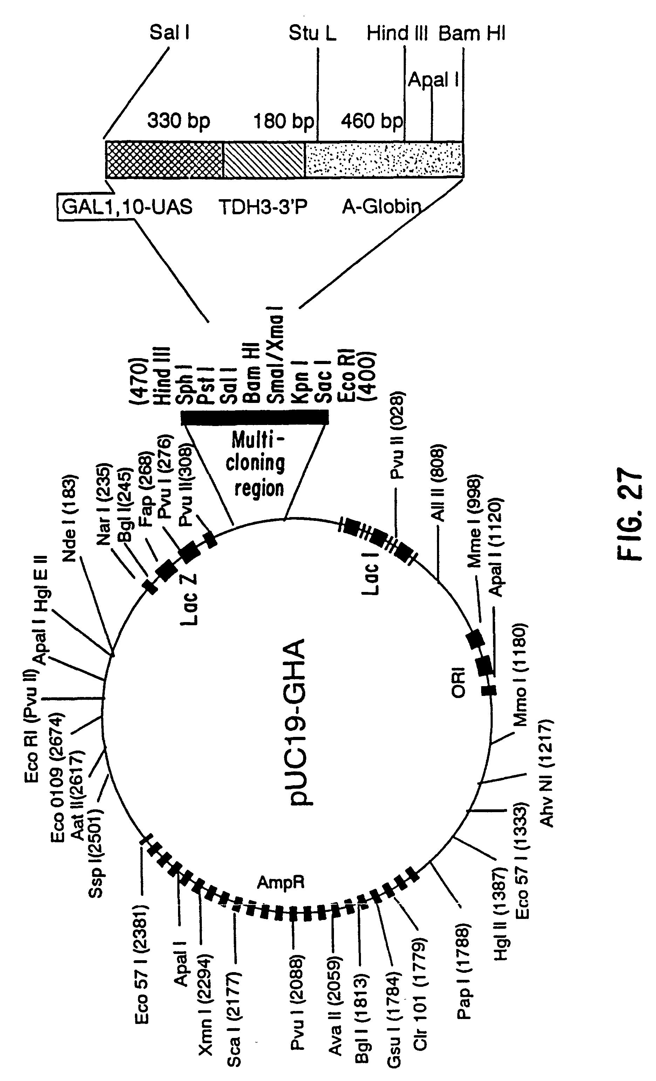

The DNA from plasmids pUC19-A2 and pUC19-GHα' were each cut with StuI-SalI and the resulting linear fragment A and the 580 bp fragment B respectively were isolated by electroelution. Ligation was set between fragments A and B and the ligation mixture was used for transforming E. coli 510α cells. The resulting plasmid containing both the fragments in correct orientation was labeled pUC19-GHA (see Figure 27).

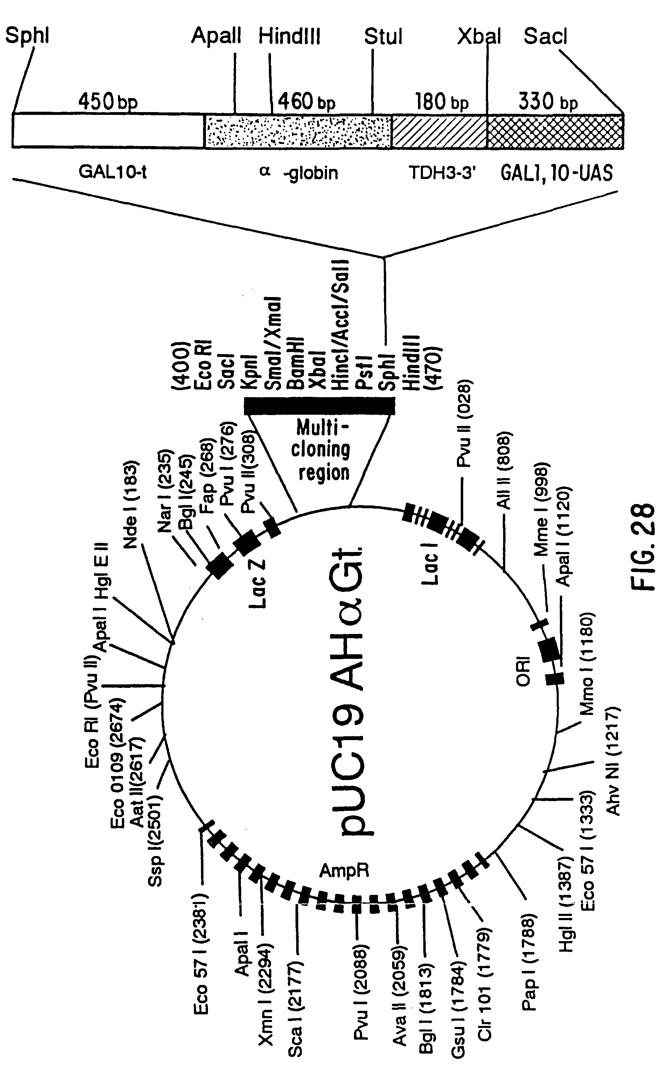

The DNA from vector plasmid pUC19 was cut with SphI. The linear DNA was isolated and dephosphorylated (fragment C). The DNA from plasmid pUC19-GHA was cut with SphI and HindIII and the resulting fragment was isolated by electroelution (fragment D). The DNA from plasmid p19A1GT was cut with SphI and HindIII and the resulting fragment containing part of alpha-globin gene and GAL10 terminator (580 bp) was isolated (fragment E). A three way ligation was set between fragments C, D and E. The ligation mixture was used for transforming E. coli DH5α cells. The plasmid containing both of the inserts in the correct orientation was named pUC19-GHαGt (Figure 28).

### 10.3 CLONING OF ADH-2-UAS AND TDH3-3'/α-GLOBIN GENE/GAL10 INTO pUC19

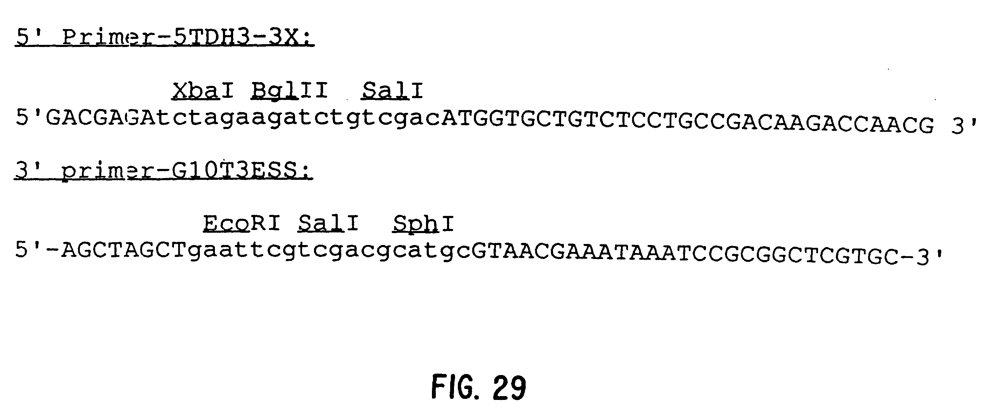

The plasmid pUC19-ADH2-UAS DNA was cut with XbaI/SphI. The linear DNA fragment was isolated (Fragment #1). The DNA fragment containing TDH3-3'/α-globin gene/GAL10 terminator (HαGt) was made by PCR using plasmid pUC19-HαGt as template. The sequence of the primers used to synthesize the DNA fragment by PCR are shown in Figure 29.

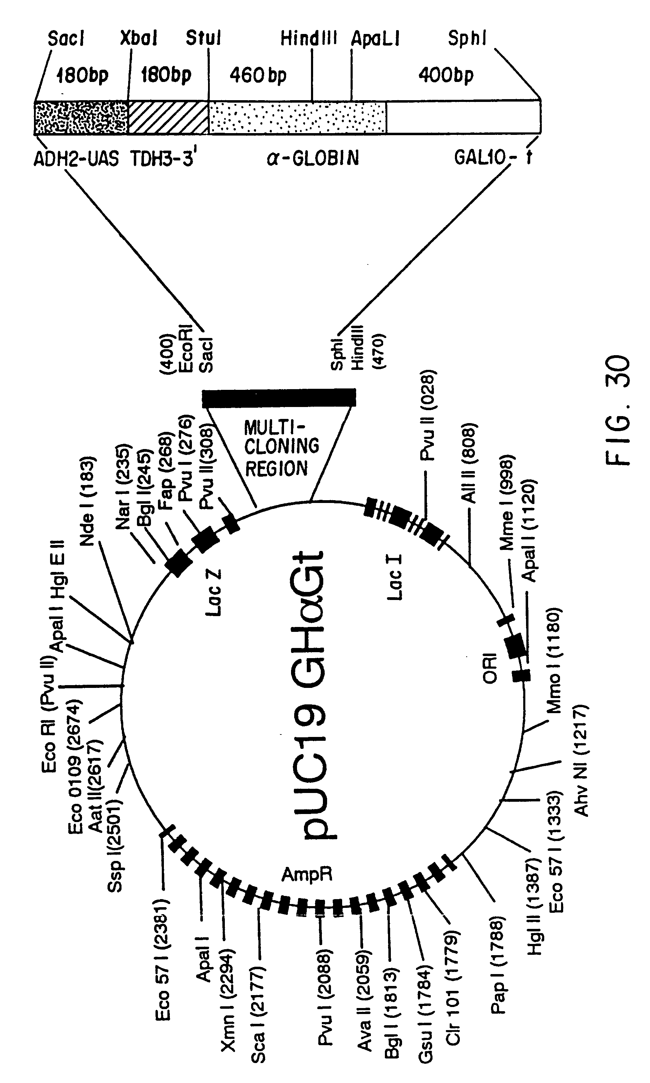

The resulting 1.04 kb fragment, containing TDH3-3'/alpha-globin gene/GAL10 terminator (HαGt) was cut with XbaI/SphI (fragment #2). Ligation was set up between fragment #1 and #2. E. coli DH5α cells were transformed and restriction digests of the DNA from the transformants were analyzed. The plasmid containing the fragments in correct orientation was labeled pUC19-AHαGt. The map of this plasmid is shown in Figure 30.

### 10.4. CLONING OF AHαGt CASSETTE INTO pPM40

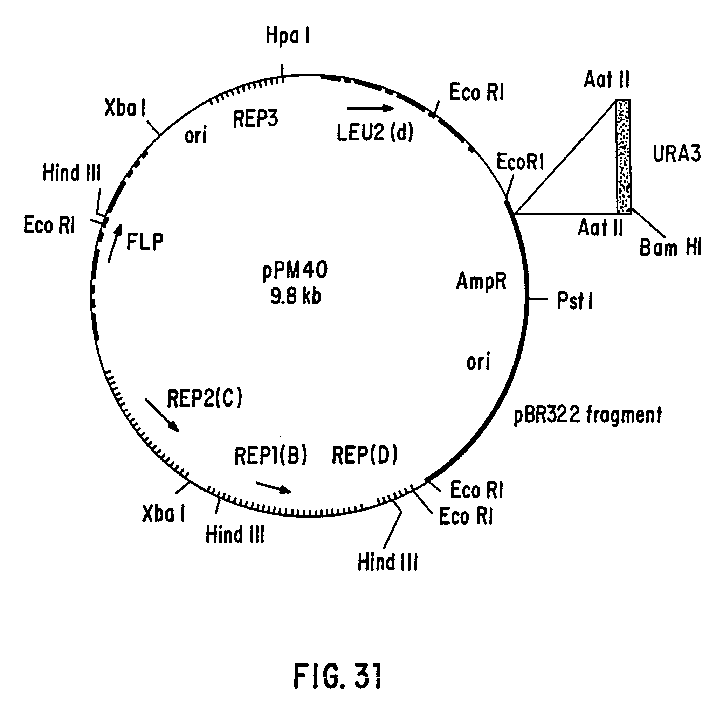

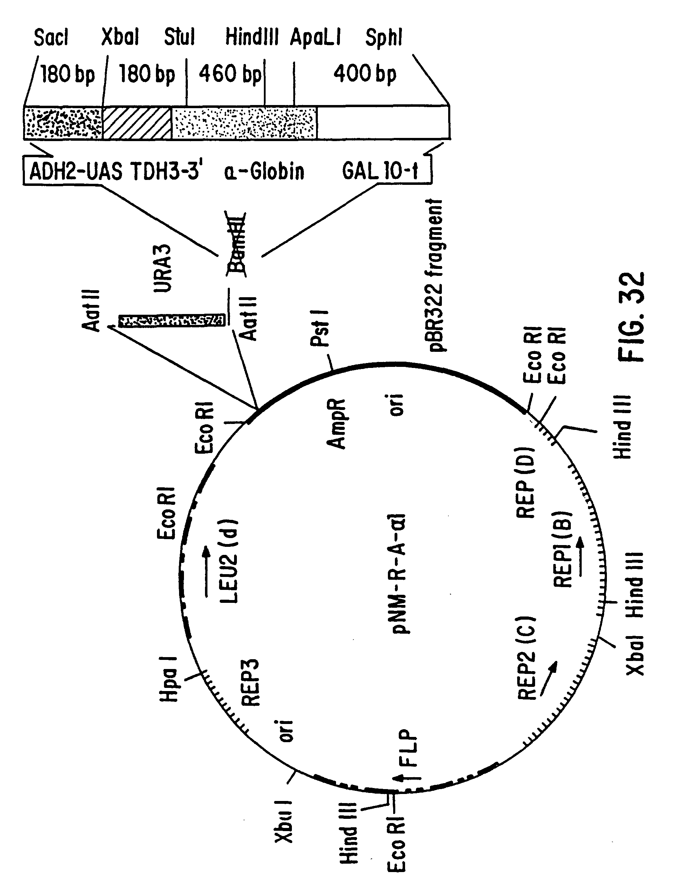

The expression cassette, AHαGt, was excised by cutting the pUC19-AHαGt plasmid DNA with SacI/SphI. The resulting 1.3 kb fragment was cloned into BamHI cut, blunt ended pPM40. The map of pPM40 is shown in Figure 31. The resulting plasmid is called pNM-R-A-α1. The map of this plasmid is shown in Figure 32.

### 10.5.TRANSFORMATION OF YEAST STRAIN Sc1012 WITH pNM-R-A-α1

The yeast strain Sc1012 is a heml mutant which is blocked in 5-aminolevulinic acid synthetase, the first enzyme in the heme biosynthetic pathway. Sc1012 has the following genotype: ade1, ade2, hem1-1, his3, leu2-3, 112, ura3-1. Yeast strain Sc1012 was transformed with plasmid pNM-R-A-α1 using the spheroplast procedure (Rose, M. et al., 1989, Methods in Yeast Genetics, Cold Spring Harbor Laboratory, Cold Spring Harbor, N.Y., pp 112-115). Two changes were made in the above protocol. The amount of YEP added during the regeneration step was reduced from 1% to 0.1%. No YEP broth was added to the regeneration agar.

For the starter culture, cells were grown in 500 ml of minimal media containing 0.67% yeast nitrogen base, 2% glucose, and supplemented with 20 mg/L each of leucine, adenine, and histidine, and 4 mg/L aminolevulinic acid at 30°C in a shake flask to log phase. The cells were then harvested, washed with uracil drop-out media containing 0.67% yeast nitrogen base, 2% glucose, and supplemented with 40 mg/L each of leucine, adenine, and histidine, and 4 mg/L of aminolevulinic acid and used to inoculate 2L of the uracil drop-out medium in a Braun Biostat E fermentor. The pH was maintained at pH 5.5 with a 5% ammonium hydroxide solution. The pO₂ was maintained at 80% of saturation during the growth phase and then adjusted to 10% at glucose depletion. The stirrer speed was set at 500 rpm, and then reduced to 100 rpm at induction. Samples were taken every 2 hours after glucose depletion over a period of fifty hours.

### 10.6. WESTERN BLOT ANALYSIS OF EXPRESSED ALPHA-GLOBIN

The expressed alpha-globin was quantitated by Western Blot analysis using procedures described in section 6.6. supra. The results indicated that up to 0.74% of the total yeast protein expressed in transformed Sc1012 cells was alpha-globin.

### 11. EXAMPLE 6: EXPRESSION OF ALPHA-GLOBIN IN A YEAST EXPRESSION VECTOR CONTAINING A HYBRID PROMOTER AND GAL10 TRANSCRIPTION TERMINATION SEOUENCE

A hybrid promoter was constructed by the fusion of the upstream activating sequence of GAL10 promoter with the downstream promoter elements of the TDH3 promoter (referred to hereafter as the 3'end of the TDH3 promoter or TDH3-3'). Specifically, the GAL10-UAS-TDH3-3' hybrid promoter, alpha-globin gene, and GAL10 terminator were cloned into plasmid pUC19. The resulting plasmid was labeled pUC19-GHαGt. The cassette containing the hybrid promoter+alpha-globin gene+GAL10 terminator was excised and cloned into the yeast shuttle vector, pPM40. Yeast strain Sc1041 cells were transformed with the resulting plasmid, pNM-R-G-α1 and the proteins expressed were analyzed by Western Blot Analysis.

### 11.1. MATERIALS

The restriction enzymes, Klenow enzyme and T4-DNA ligase were obtained from New England Biolabs (Biolabs), Bethesda Research Laboratories (BRL) or Boehringer Mannheim (BM). All enzymes were used according to the suppliers' specifications. Plasmid DNA was isolated from a one liter culture of the transformed E. coli cells and purified by CsCl gradient centrifugation.

### 11.2. CONSTRUCTION OF pUC19-GHαGt

pUC19-GHαGt is constructed using the procedure described in Section 10.2, supra.

### 11.3. CLONING OF GHαGt CASSETTE INTO pPM40

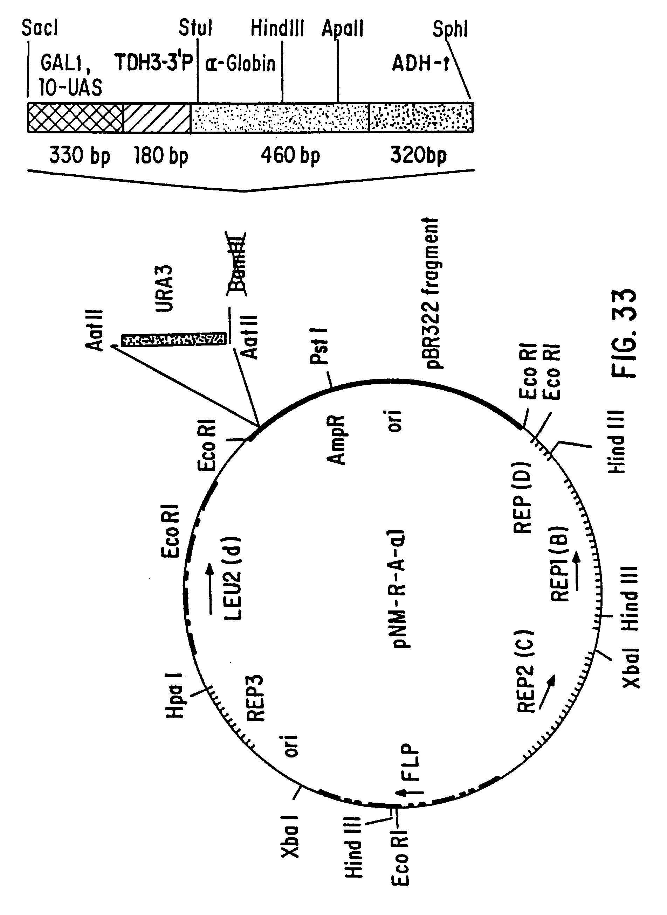

The expression cassette, GHαGt, was excised by cutting the pUC19-GHαGt plasmid DNA with SacI/SphI and blunt ended. The resulting 1.3 kb fragment was cloned into BamHI cut, blunt ended pPM40 (see Figure 31 for a map of pPM40). The resulting plasmid is called pNM-R-G-α1. The map of this plasmid is shown in Figure 33.

### 11.4. TRANSFORMATION OF YEAST STRAIN Sc1041 WITH pNM-R-G-α1

Sc1041 has the following genotype: MATa, leu2, ura3-52, trp(d)63, prb1-112, pep4-3, prc1-407, GAL2⁺. Yeast strain Sc1041 was transformed with plasmid pNM-R-G-α1 using the spheroplast procedure (Rose, M. et al., 1989, Methods in Yeast Genetics, Cold Spring. Harbor Laboratory, Cold Spring Harbor, N.Y., pp 112-115).

For the starter culture, cells were grown in 500 ml of minimal media containing 0.67% yeast nitrogen base, 2% lactic acid, 3% glycerol, and supplemented with 20 mg/L each of tryptophan and leucine at 30°C in a shake flask to log phase. The cells were then harvested, washed with uracil drop-out media containing 0.67% yeast nitrogen base, 2% lactic acid and 3% glycerol, and supplemented with 20 mg/L of tryptophan and used to inoculate 500 ml of the uracil drop-out medium. The culture was incubated with shaking (300 rpm) at 30°C, and grown to mid-log phase before adding galactose to a final concentration of 1%. Samples were collected for analysis after four hours of induction.

### 11.5. WESTERN BLOT ANALYSIS OF EXPRESSED ALPHA-GLOBIN

The expressed alpha-globin was quantitated by Western Blot analysis using procedures described in Section 6.6. supra. Detectable levels of alpha globin were observed.

### 12. EXAMPLE 7: COEXPRESSION OF ALPHA-GLOBIN AND BETA-GLOBIN IN A YEAST BY COTRANSFORMATION

### 12.1. TRANSFORMATION OF YEAST STRAIN Sc1041 WITH pNM-R-G-α1 AND pNML-V-G-1

Sc1041 has the following genotype: MATa, leu2, ura3-52, trp(d)63, prb1-112 pep4-3, prc1-407, GAL2+. Yeast strain Sc1041 was co-transformed with plasmid pNM-R-G-α1 (supra, 11.3) and pNML-V-G-1 (supra, 7.5) using the spheroplast procedure (Rose, M. et al., 1989, Methods ir Yeast Genetics, Cold Spring Harbor Laboratory, Cold Spring Harbor, N.Y., pp. 112-115).

For the starter culture, cells were grown in 500 ml of minimal media containing 0.67% yeast nitrogen base, 2% lactic acid, 3% glycerol, 1% raffinose, and supplemented with 20 mg/L of tryptophan at 30°C in a shake flask to log phase. The cells were then harvested, washed with leucine, uracil drop-out media containing 0.67% yeast nitrogen base, 2% lactic acid and 3% glycerol, and supplemented with 40 mg/L of tryptophan and used to inoculate 1700 ml of the leucine, uracil drop-out medium in a Braun Biostat E fermenter. The pH was maintained at 5.0; the pO₂ was maintained at 30%; the stirrer speed was maintained at 300 rpm. The culture was induced with galactose at the rate of 0.05% per hour. Samples were collected at intervals for sampling.

### 12.2. WESTERN BLOT ANALYSIS OF EXPRESSED GLOBIN

The expressed globins were quantitated by Western Blot analysis using procedures described in Section 6.6. Detectable levels of globin were observed (0.2% soluble protein).

### 12.3. WESTERN BLOT ANALYSIS OF EXPRESSED ALPHA AND BETA GLOBINS

The expressed alpha and beta globins were separated and quantitated using an 18% SDS polyacrylamide gel.

Phosphate-buffered saline (PBS, 0.9 M NaCl, 0.01 M phosphate, pH 7.6) solution (2 ml) was added to thawed yeast samples (0.2 g wet weight) . The samples were centrifuged at 4°C for 10 minutes at 2700 rpm in a Sorvall RT6000B and the supernatant decanted. Cold disruption buffer (50 mM Tris, 5 mM EDTA, 0.5 mM PMSF, pH 8.0) prepared immediately before use (0.2 ml) was added to the pellet, followed by enough ice-cold glass beads to just reach the top surface of the liquid. After vortexing for 30 seconds at maximum speed the samples were placed on ice for 5 minutes. This step was repeated twice. Ice-cold disruption buffer (1 ml) was added to each sample and the homogenate was transferred to an Eppendorf tube. In another Eppendorf tube, 200 µl of homogenate was combined with 200 µl of freshly prepared standard discontinuous 2X sample buffer (Laemli, 1970, Nature 227:680-685) and the sample was boiled for 10 min.

After centrifuging the samples for 10 min., the samples were loaded onto a 20 x 18 cm discontinuous denaturing gel in which the stacking gel was 3% acrylamide and the separating gel was 18% (Laemli, 1970, Nature 227: 680-685). Gels were run at a constant current of 15 mA per gel.

After the electrophoresis was complete and the dye band had reached the bottom of the separating gel, the gels were removed from the electrophoresis unit and the plates were pried apart under running deionized water. The stacking gel was discarded and the lower gel was separated from the plate. The transfer unit was filled with transfer buffer (2L methanol, 30.3 g Tris base, 144 g glycine in a final volume of 10L, pH 8.3) and 2L of the transfer buffer was put into a shallow pan. The transfer sandwich consisting of large pore gauze, 3 M blotting paper, the gel, a piece of nitrocellulose paper precut to just cover the gel, 3M blotting paper, and another piece of large pore gauze was assembled under the buffer in the shallow pan.

Protein was transferred from the gel to the nitrocellulose paper by applying a voltage of 40V for 1.5 hrs. After transfer was complete, the nitrocellulose was removed and placed in a shallow pan with 50 ml of blocking solution [5%(w/v) BSA in PBS]. The nitrocellulose membrane was incubated for 1 hour with agitation, after which the blocking solution was replaced with washing solution [0.1% Tween 20 (v/v) in PBS]. Three washings of 15, 5 and 5 minutes were carried out. The final wash solution was discarded and 25 µl of primary antibody in 25 ml of PBS was added to the pan. After incubation for 2 hours, with agitation, the nitrocellulose was washed three times (1 x 15 and 2 x 5 minutes). The final wash was discarded and 2.5 µl of secondary antibody in 25 ml of PBS added for a 1 hour incubation with agitation. After three washes (1 x 15 and 2 x 5 minutes), 5 µl of streptavidin-HRP (horseradish peroxidase) was added in 25 ml of PBS containing 0.1% Tween 20 (v/v). After a 20 minute incubation with agitation, the membrane was washed three times (1 x 15 and 2 x 5 minutes). The nitrocellulose was then placed in ECL (enhanced chemiluminescent) developing solution that had been prepared immediately prior to use by mixing equal volumes of detection reagent 1 and detection reagent 2 (Amersham). The membrane was incubated for 1 minute with agitation, removed from the developing solution, the excess reagent drained off and the membrane then wrapped in Saran Wrap. The wrapped nitrocellulose was then exposed to X-ray film for an appropriate length of time. After development, the X-ray film was scanned using a laser densitometer and the quantity of globin in each sample estimated by comparison with globin standards run on the same gel.

Alpha and beta globins are separated on this gel by molecular weight. Alpha and beta globin were detected in the protein extracts of the cotransformed yeast.

### 12.4. SORET SPECTRA OF HEMOGLOBIN IN YEAST

The porphyrin ring found in functional hemoglobin is responsible for the molecule's ability to reversibly bind oxygen. In addition, the many double bonds of the ring result in hemoglobin being able to absorb light in the 200 to 600 nm (ultraviolet to visible) range. The exact wavelengths of maximum absorbance (i.e. peaks) are a function of several factors including the exact structure of the ring , the protein with which the ring is associated (e.g. hemoglobin, myoglobin, cytochromes, etc.), differences in the environment near the ring within a given protein (e.g. HbA₀ versus HbAO₂, etc.) and small inorganic molecules bound to the ring (e.g. oxygen, carbon monoxide, nitric oxide, etc.). The hemoglobin absorbance peaks in the 415 to 430 nm range are particularly intense and can be used to monitor the presence of hemoglobin. This region is termed the Soret region and the hemoglobin absorbances here are referred to as Soret bands. The presence of HbA can be confirmed by the shift in the Soret band observed when CO displaces O₂. In this case, the Soret band shifts from 415 to 419 nm.

The yeast cells were suspended in Tris buffer, pH 7.5 at 25°C. The Soret spectrum was scanned initially, followed by exposure to carbon monoxide (3-4 minutes) and reduction with dithionite solution (20 mM in nitrogen saturated buffer). The Soret spectrum indicated absorbance in the region of 416-418 nm which is characteristic of hemoglobin and is not present in control yeast that do not express globins.

### 13. EXAMPLE 8: COEXPRESSION OF ALPHA AND BETA GLOBIN USING A TWIN CASSETTE PLASMID

Two coexpression plasmids were constructed by twin cassette strategy. Two separate expression cassettes one containing human α globin the other β globin genes were cloned at two unique sites in a single yeast vector. The resulting plasmids pBM-V-X1-G-αβ (like orientation) and pBM-V-X2-G-αβ (opposite orientation) carry both α-globin and β-globin genes under the control of two separate (identical) promoters. Yeast Sc1115 cells were transformed with plasmid pBM-V-X2-G-αβ, grown and induced with galactose. The yeast transformed with this twin cassette produced functional HbA.

### 13.1. MATERIALS

The restriction enzymes, Klenow enzyme and T4-DNA ligase were obtained from New England Biolabs (Biolabs), Bethesda Research Laboratories (BRL) or Boehringer Mannheim (BM). All enzymes were used according to the suppliers specifications. Plasmid DNA was isolated from a one liter culture of the transformed cells and purified by CsCl gradient centrifugation.

### 13.2. CLONING OF pBM-V-X1-G-αβ and pBM-V-X2-G-αβ

Construction of the plasmid pNML-V-G-1 was described in Section 7.5., supra. The plasmid pNML-V-G-1 contains the expression cassette, GAL1-10-UAS-TDH3-3' hybrid promoter, β-globin gene and ADH1 terminator (GHβAT) which was cloned in at the BamH1 site of the yeast expression vector, YEp13. The plasmid pUC19-GHαGt (Section 10.2, supra) contains the same hybrid promoter, the α-globin gene and the GAL10 terminator. This expression cassette was labeled GHαGt.

The DNA from plasmid pNML-V-G-1 was digested with PvuII and the linear DNA was isolated by electroelution and dephosphorylated (fragment 1). The DNA from plasmid pUC19-GHαGt was cut with SacI/SphI and the resulting 1.3 kb fragment containing GHαGt cassette was isolated and blunt ended (fragment 2). Ligation was set between fragments 1 and 2. E. coli Sure cells were used for transformation and the transformants were isolated on ampicillin selection plates.

The DNA from 48 transformants was isolated by alkaline lysis and analyzed by digestion with ApaLI enzyme.

The positive clones containing all the fragments were further analyzed by restriction enzyme digestions. The plasmids containing both the expression cassettes in the yeast vector YEp13 were labeled, pBM-V-X1-G-αβ and pBM-V-X2-G-αβ. The X1 refers to the orientation in which both cassettes are transcribed in the same direction and X2 refers to the opposite orientation.

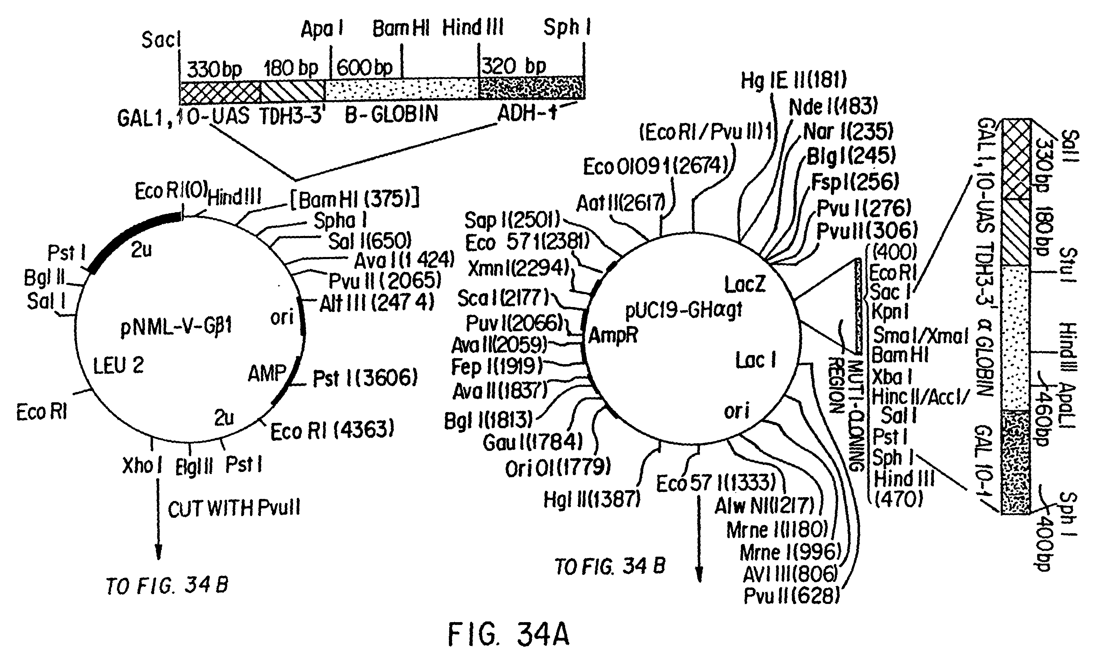

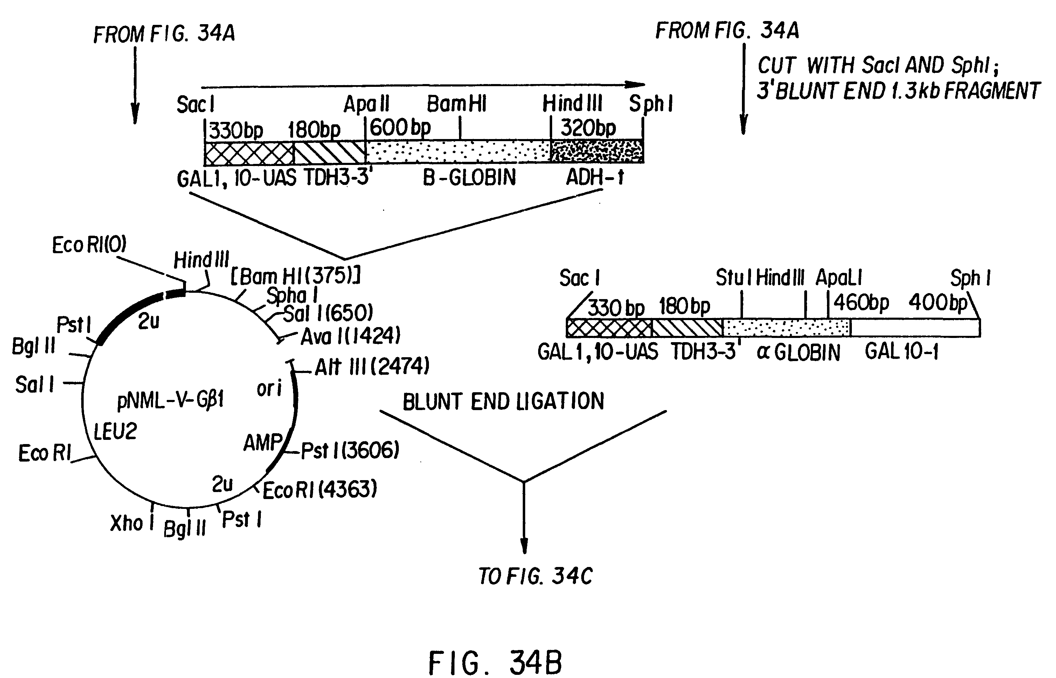

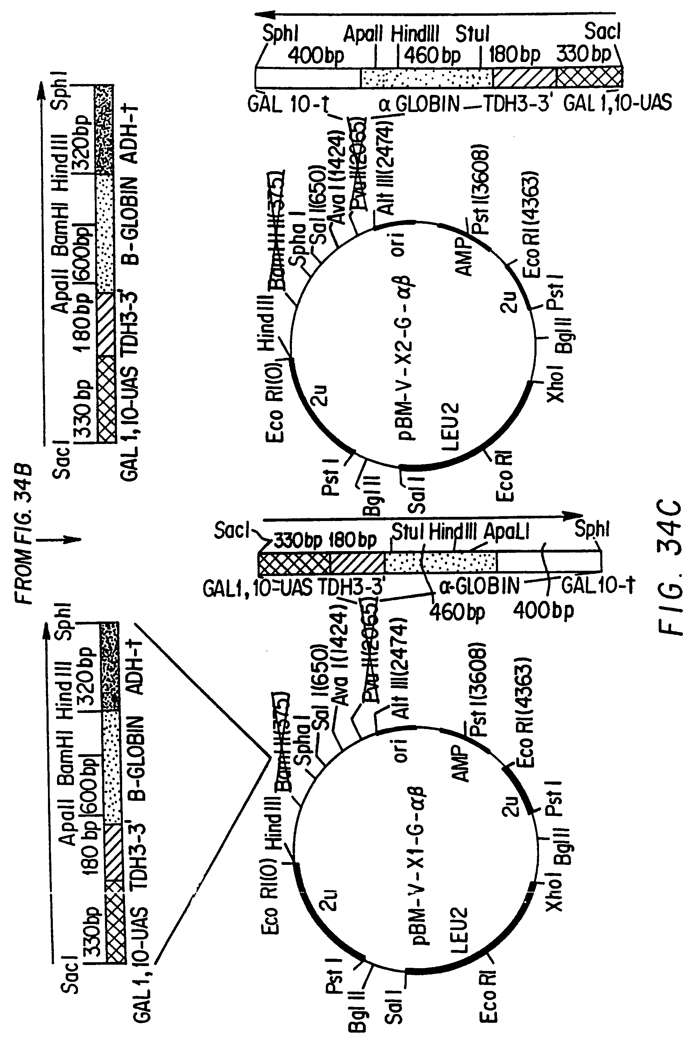

Figure 34 describes the strategy used for cloning GHαGt cassette into a yeast vector carrying GHβAt expression cassette.

### 13.3. TRANSFORMATION OF YEAST STRAIN Sc1115 WITH pBM-V-X1-G-αβ and pBM-V-X2-G-αβ

Sc1115 has the following genotype: MATa, leu2-3,112; his3-115, Can^{R}. Yeast strain Sc1115 was transformed with plasmid pBM-V-X1-G-αβ (1115X1VGa1b1) or pBM-V-X2-G-αβ (1115X2VGa1b1) using the electroporation procedure.

For the starter culture, cells were grown in 500 ml of minimal media containing 0.67% yeast nitrogen base,.2% lactic acid, 3% glycerol, 1% raffinose, and supplemented with 20 mg/L of histidine at 30°C in a shake flask to log phase. The cells were then harvested, washed with leucine drop-out media containing 0.67% yeast nitrogen base, 2% lactic acid and 3% glycerol, and supplemented with 40 mg/L histidine and used to inoculate 1700 ml of the leucine drop-out medium in a Braun Biostat E fermenter. The pH was maintained at 5.0; the pO2 was maintained at 30%; the stirrer speed was maintained at 300 rpm. The culture was induced with galactose at the rate of 0.05% per hour. Samples were collected at intervals for sampling.

### 13.4. WESTERN BLOT ANALYSIS OF EXPRESSED GLOBTN

The expressed globins were quantitated by Western Blot analysis using procedures described in Section 6.6. Detectable levels of globin were observed (1% of total soluble protein).

### 13.5. WESTERN BLOT ANALYSIS OF EXPRESSED ALPHA AND BETA GLOBINS

The expressed alpha and beta globins were separated and quantitated using an 18% SDS polyacrylamide gel (supra, 12.4.). Detectable levels of both alpha and beta globins were observed.

### 13.6. SORET SPECTRA OF HEMOGLOBIN IN YEAST

The Soret spectrum (supra, 12.5) indicated absorbance in the region of 418 nm which is characteristic of hemoglobin and is not present in control yeast that do not express globins. A concentration of 5 µM heme was detected.

### 14. EXAMPLE 9: COEXPRESSION OF ALPHA AND GAMMA_{VAL} GLOBIN USING A TWIN CASSETTE PLASMID

Two coexpression plasmids were constructed by twin cassette strategy. Two separate expression cassettes, one containing human α globin and the other γ(val) globin genes were cloned at two unique sites in a single yeast vector. The resulting plasmids pBM-R-X7-A-αγᵥₐₗ (like orientation) and pBM-R-X8-A-αγᵥₐₗ (opposing orientation) carry both α-globin and γ(val) globin genes under the control of two separate promoters.

### 14.1. MATERIALS

The restriction enzymes, Klenow enzyme and T4-DNA ligase were obtained from New England Biolabs (Biolabs), Bethesda Research Laboratories (BRL) or Boehringer Mannheim (BM). All enzymes were used according to the suppliers specifications. Plasmid DNA was isolated from a one liter culture of the transformed cells and purified by CsCl gradient centrifugation.

### 14.2. CLONING OF PLASMIDS pBM-R-X7-A-αγᵥₐₗ AND pBM-R-X8-A-αγᵥₐₗ

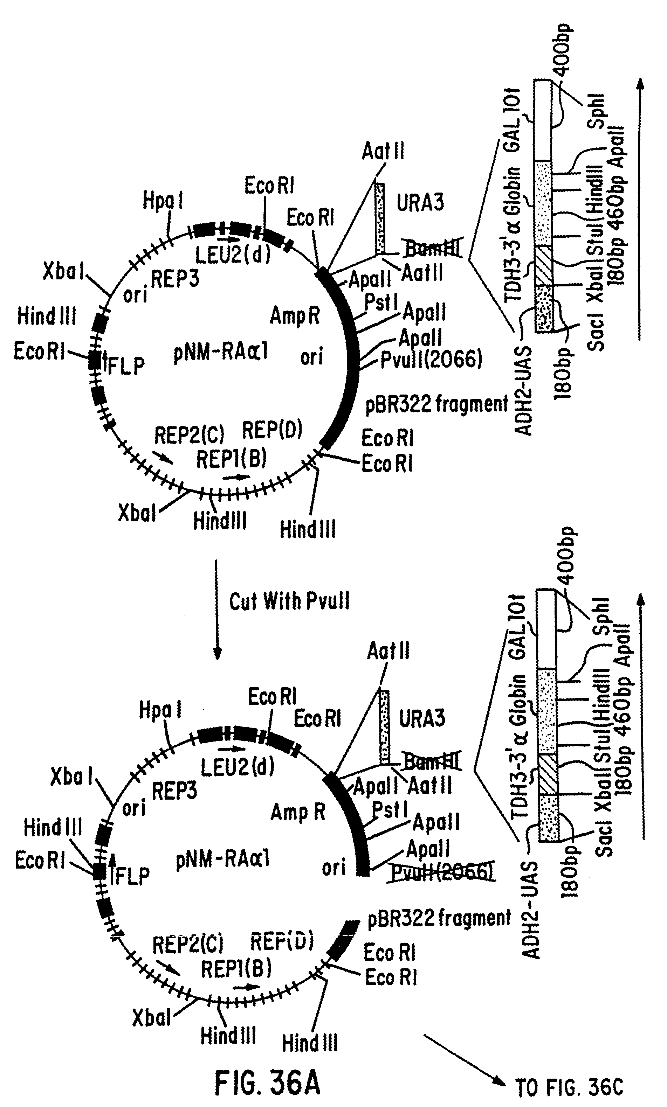

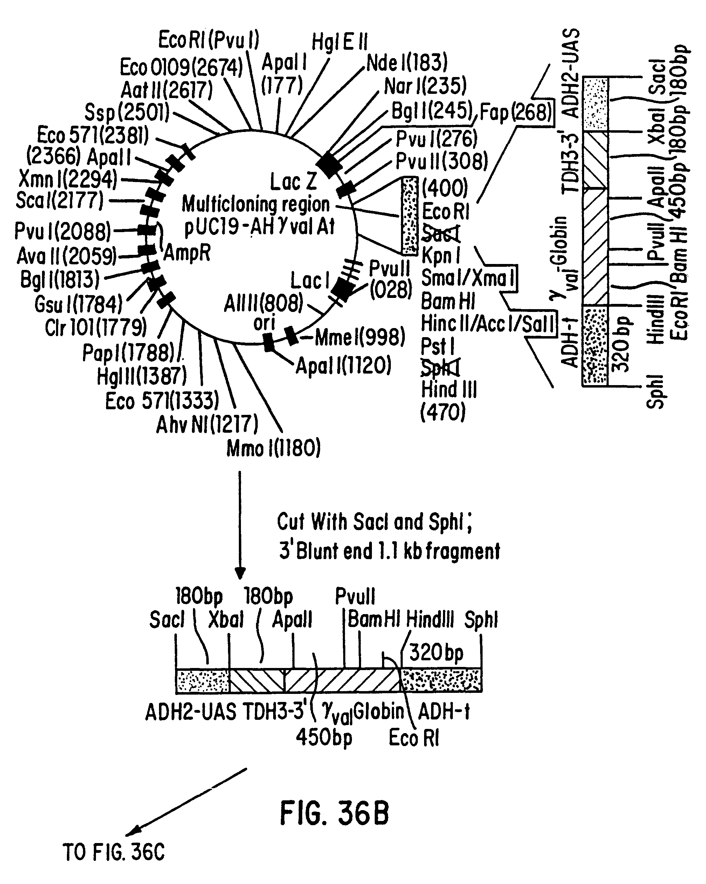

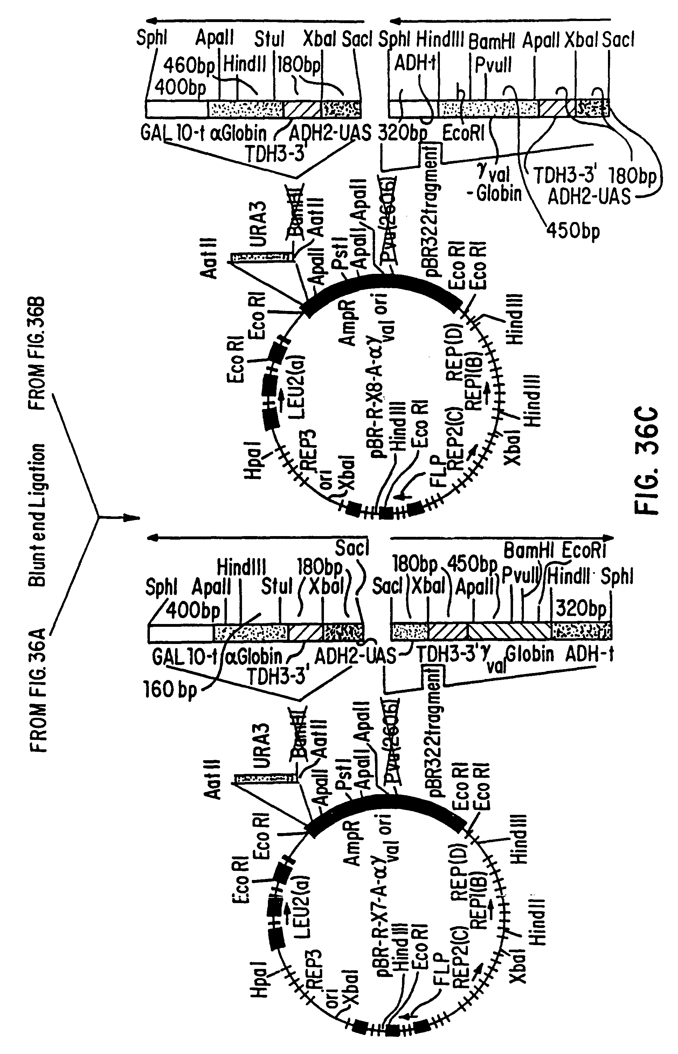

The plasmid pNM-R-A-α1 (Section 9.4., supra) contains the expression cassette, ADH2-UAS-TDH3-3' hybrid promoter, α-globin gene and GAL10 terminator (AHαGt) which was cloned in at the BamHI site of the yeast expression vector, pPM40. Figure 36 describes the strategy used for cloning AHγᵥₐₗAt cassette into yeast expression plasmid pNM-R-A-α1. The resulting twin cassettes were named pBM-R-X7-A-αγᵥₐₗ (like orientation) and pBM-R-X8-A-αγᵥₐₗ (opposite orientation).

### 14.2.1. CLONING OF PLASMID pUC19-AHγᵥₐₗAt

The plasmid pUC19-AHγᵥₐₗt contains the ADH2-UAS-TDH3-3' hybrid promoter, the γᵥₐₗ-globin gene and the ADH terminator. This expression cassette was labeled AHγᵥₐₗAt.

### 14.2.1.1. CONSTRUCTION OF pUC19-AHβAt

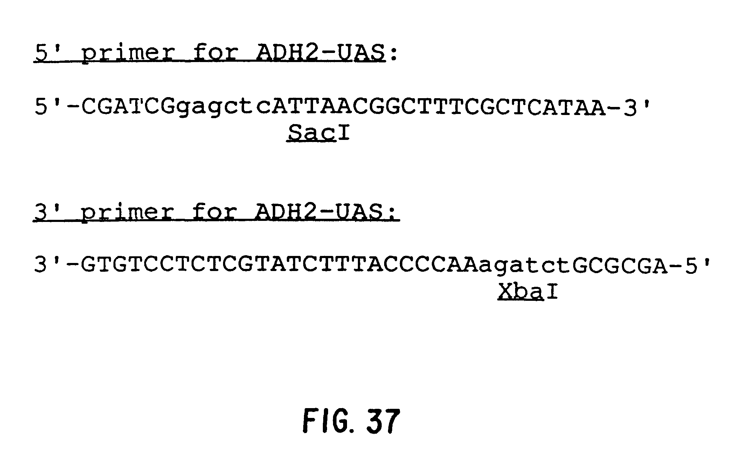

The ADH2-UAS DNA fragment was generated by PCR. Genomic DNA was isolated from yeast strain S173-6B. The 5' and 3' primers used for synthesizing the ADH2-UAS DNA fragment are shown in Figure 37.

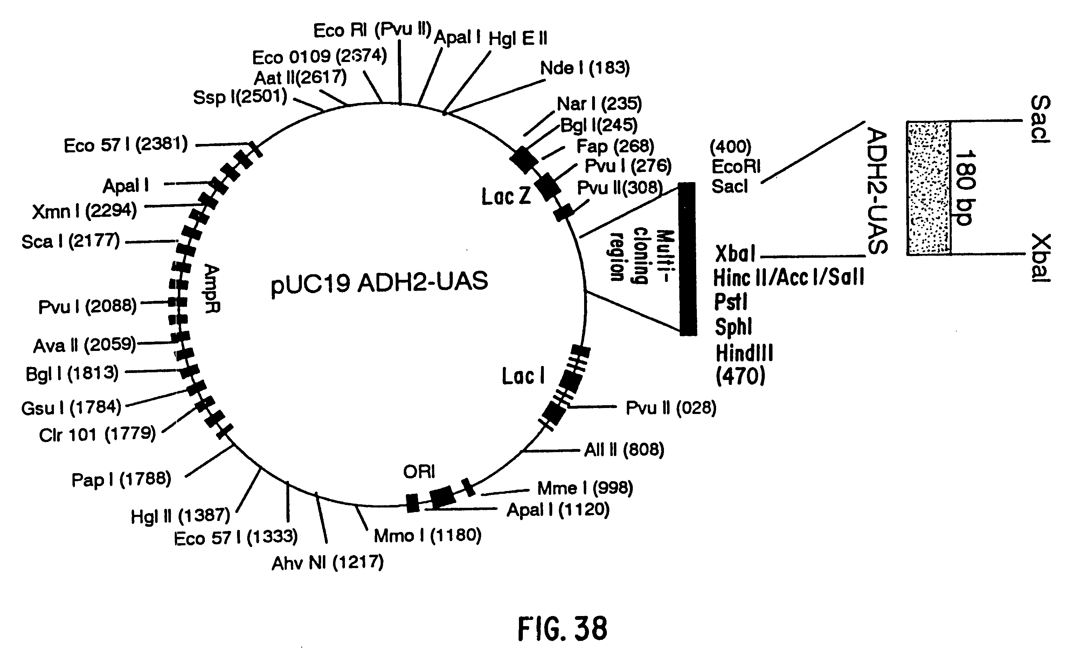

The resulting ADH2-UAS fragment (200 bp) was isolated by electroelution and cut with SacI (5'-end) and XbaI (3'-end) and cloned into pUC19 cut with SacI and XbaI. The ligation mixture was transformed in competent E. coli cells and the DNA was isolated from 24 transformants by alkaline digestion. The DNA samples from the clones were analyzed by appropriate enzymes and the resulting plasmid was labeled pUC19-ADH2-UAS. The map of this plasmid is shown in Figure 38. The DNA from plasmid pUC19-ADH2-UAS was cut with XbaI/SphI. The linear DNA fragment was isolated (Fragment #1).

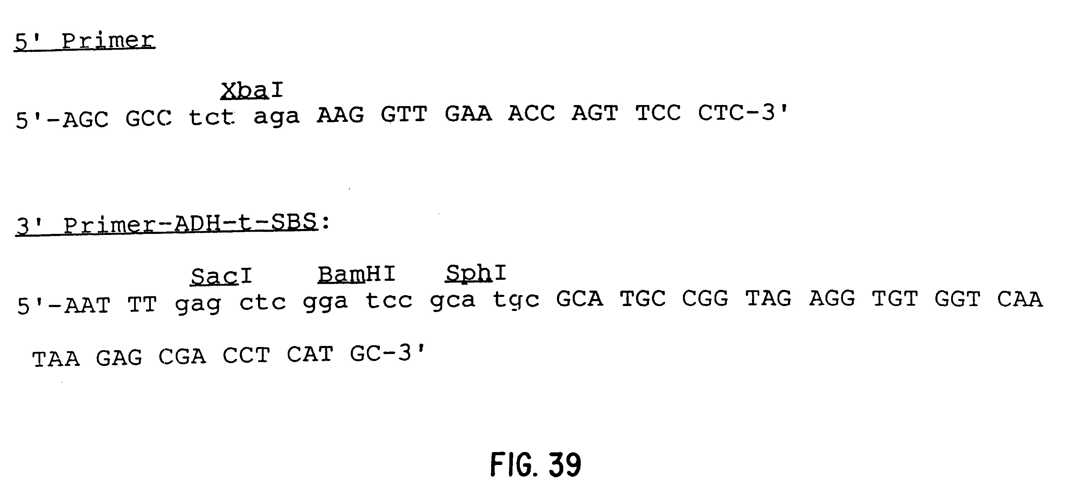

PCR was carried out using DNA from the plasmid, pUC19-GHβAt as a template. The 5' and 3' primers used for synthesizing the sequence are shown in Figure 39. The resulting 1.1 kb fragment was labeled HβAt (fragment #2). The resulting 1.1 kb fragment, containing TDH3-3'-β-globin gene-ADH1 terminator (HβAt) was cut with XbaI/SphI (fragment #2).

Ligation was set between fragment #1 and #2. The E. coli DH5α cells were transformed and the DNA from the transformants was analyzed by appropriate enzymes. The plasmid containing the fragments in correct orientation was labeled pUC19-AHβAt.

### 14.2.1.2. CLONING OF γ(val)-GLOBIN INTO pUC19-AHβAt

Using appropriate primers and DNA from plasmid YEp51TG, γᵥₐₗ-globin containing ADH terminator was synthesized by PCR. The 5' and 3' primers used for synthesizing the sequence are shown in Figure 21. The PCR fragment was cut with ApaLI and SphI (fragment #1). The DNA from pUC19 plasmid was cut with SacI/SphI and linear DNA fragment was isolated (fragment #2). The DNA from pUC19-AHβAt (supra, 14.2.1.1.) was cut with ApaLI and SacI, and the 360 bp fragment containing ADH2-UAS-TDH3-3' hybrid promoter was isolated by electroelution (fragment #3).

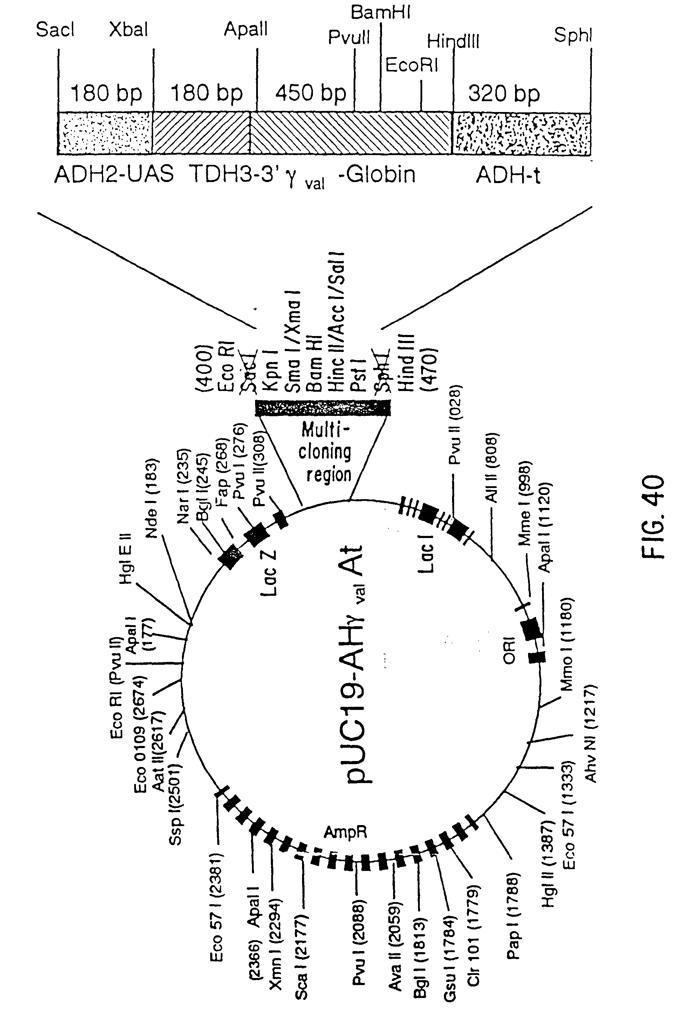

A three way ligation was set between the above three fragments. E. coli DH5α cells were used for transformation. The DNA from 24 transformants was isolated by alkaline digestions and analyzed by restriction digestions. The resulting plasmid containing all fragments in correct orientation was labeled pUC19-AHγᵥₐₗAt; the map of this plasmid is shown in Figure 40.

### 14.2.2. CLONING OF AHγᵥₐₗAt CASSETTE INTO YEAST EXPRESSION PLASMID pNM-R-A-α1

Figure 36 describes the strategy used for cloning AHγᵥₐₗAt cassette into yeast expression plasmid pNM-R-A-α1.

The DNA from plasmid pNM-R-A-α1 (see Section 10, supra) was digested with PvuII and the linear DNA was isolated by electroelution and dephosphorylated (fragment 1). The DNA from plasmid pUC19-AHγᵥₐₗAt was cut with SacI/SphI and the resulting 1.3 kb fragment containing AHγᵥalAt cassette was isolated and blunt ended (fragment 2). Ligation was set between fragments 1 and 2. E. coli Sure cells were used for transformation and the transformants was isolated on ampicillin selection plates. The DNA from 48 transformants was isolated by alkaline lysis and analyzed by digestion with ApaLI enzyme.

The positive clones containing all the fragments were further analyzed by restriction enzyme digestions. The plasmids containing both the expression cassettes in the yeast vector pPM40 (see Figure 31) were labeled pBM-R-X7-A-αγᵥₐₗ (like orientation) and pBM-R-X8-A-αγᵥₐₗ (opposite orientation).

Yeast strain Sc1113 was transformed with plasmid pBM-R-X8-A-αγᵥₐₗ (1113X8VGalgVAL1) using the spheroplast procedure. For the starter culture, cells were grown in 500 ml of minimal media containing 0.67% yeast nitrogen base, 2% lactic acid, 3% glycerol, 1% raffinose, and supplemented with 20 mg/L of histidine at 30°C in a shake flask to log phase. The cells were then harvested, washed with leucine drop-out media containing 0.67% yeast nitrogen base, 2% lactic acid and 3% glycerol, and supplemented with 40 mg/L histidine and used to inoculate 1700 ml of the leucine drop-out medium in a Braun Biostat E fermenter. The pH was adjusted to 6.93. The culture was grown to glucose exhaustion and samples were taken between one and 30 hours after exhaustion for testing.

The expressed globins were quantitated by Western Blot analysis using procedures described in Section 6.6. Globin was detected at a level of 0.03% of soluble protein.

### 15. EXAMPLE 10: EXPRESSION OF VARIANT GLOBINS

The mutant globin genes were cloned into yeast expression vector YEp51NT1. This vector contains GAL10 promoter and ADH terminator sequences. The following mutant genes were cloned into this yeast expression vector:

| | | |
|---|---|---|
| viii. | γ-Porto Alegre | (9 Ala->Cys) |
| ix. | γ-Chico | (66 Lys->Thr) |
| x. | ζ-Titusville | (94 Asp->Asn) |

### 15.1. MATERIATS AND METHODS

Restriction and DNA modifying enzymes were obtained from Boehringer-Mannheim, Bethesda Research Laboratories, Perkin-Elmer or New England Biolabs. All enzymes were used according to the supplier's specifications.

Oligo nucleotides used in the Polymerase Chain Reaction (PCR) were obtained by chemical synthesis on Applied Biosystems 380B DNA synthesizer.

The E. coli strain used for all bacterial transformations was DH5α.

### 15.1.1. DNA FRAGMENT ISOLATION

All DNA fragments were separated on agarose gel (1 x TBE) and isolated by electroelution using Pharmacia Electroeluter.

### 15.1.2. DNA LIGATION AND E. COLI TRANSFORMATION

All DNA ligations were carried out using standard ligation procedures ("Laboratory Cloning: A Laboratory Manual"; Sambrook, J., Fritsch, E. F. and Maniatis, T. eds. Cold Spring Harbor Laboratory Press, 1989, Second Edition pp: 1.631.71) and E. coli transformation was carried out using standard transformation procedures ("Laboratory Cloning: A Laboratory Manual"; Sambrook, J., Fritsch, E. F. and Maniatis, T. eds.; Cold Spring Harbor Laboratory Press 1989, Second Edition pp: 1.74 - 1.84). Transformed cells were plated on LB-media with 100 mg/L ampicillin. Plates were incubated at 37°C overnight. Colonies appearing on these plates were used to inoculate 5.0 ml LB media with 100 mg/L ampicillin and cultures were grown at 37°C overnight.

### 15.1.3. PLASMID DNA ANALYSIS

DNA was isolated from 1.5 ml of the overnight culture using alkaline lysis procedure. Plasmid DNA was analyzed by appropriate restriction enzyme digestion.

### 15.1.4. YEAST TRANSFORMATION

Yeast transformation was done using published procedures (Hinnen, A., Hicks, J. B. and Fink, G. R. (1978) Transformation in Yeast, Proc. Natl. Acad. Sci. U.S.A. 75, 1929-1933).

### 15.2. SYNTHESIS OF OLIGONUCLEOTIDES

Various oligonucleotides were synthesized as a preliminary step in the construction of several beta-globin gene variants. The oligonucleotides to be used in the in vitro mutagenesis procedure with M13 were synthesized and purified. Polyacrylamide gel electrophoresis following kinasing demonstrated that the synthesis was efficient and that the oligonucleotides were ready for use in the M13 system.

Following synthesis and incubation at 65°C, the oligonucleotides were purified using oligonucleotide purification columns (ABI). The purified oligonucleotides were lyophilized and suspended in 100 µl water and the concentration was determined by 0D260.

Approximately 100 ng of the synthetic DNA was used in a kinasing reaction to determine the efficiency of the synthesis. The [γ-³²P]ATP kinased oligonucleotides were analyzed on a 6% acrylamide sequencing gel containing 7M urea. The dye used in this electrophoresis was a mixture of bromphenol blue and xyno-cynol which separate during the procedure, with each dye migrating at different rates. Autoradiography was performed following drying of the gel.

Following autoradiography of the sequencing gel, the results indicated that the synthesis was efficient, as the majority of the radioactivity was incorporated into the larger bands that moved between the two dye fronts. Under the electrophoresis conditions described above, fragments that are approximately 25 bases should migrate with the bromphenol blue dye front, while those of about 90 bases should migrate with the xyno-cynol dye front. The synthetic oligonucleotides ranged in size from 30 to 45 bases which should run between the two dye fronts as was observed.

### 15.3. IN VITRO MUTAGENESIS

The in vitro mutagenesis kit from Bio Rad provides the necessary components for mutagenesis with the M13 system. Included in this kit are two strains of E. coli to be used in the process. E. coli strain CJ236 contains mutations which result in the incorporation of uracil instead of thymine in DNA. E. coli strain MV1190 is a wild type strain that is used to produce the single stranded DNA following mutagenesis.

### 15.3.1. STRAINS

The E. coli strains that were received in the mutagenesis kit were subcultured on appropriate media according to the genetic markers for selection. The constituents of each type of media as well as a suggested protocol for mutagenesis may be found in the brochure that was received with the kit (New England BioLabs, "M13 Cloning and Sequencing System - A Laboratory Manual").

### 15.3.2. TRANSFECTION OF CJ236

CJ236 competent cells for use in transfection were prepared by inoculating 100 ml LB broth containing chloramphenicol with 50 ml of an overnight culture of CJ236. The culture was incubated at 37°C in an air shaker until the OD₆₀₀ reached 0.8. The cells were centrifuged at 3K rpm for 5 minutes, resuspended in 20 ml 50 mM cold CaCl₂, and held on ice for 30 minutes. The cells were centrifuged again and resuspended in 4 ml 50 mM CaCl₂.

The CJ236 competent cells were transfected with M13mp19BHS by adding 1 µl or 5 µl of DNA to 0.3 ml competent cells. The tubes were held on ice for 40 minutes, heat shocked at 42°C for 3 minutes and the contents were added to 4 ml of top agar (50°C) containing chloramphenicol and 300 µl of the overnight culture of CJ236. This top agar was poured onto H-medium plates containing chloramphenicol and incubated overnight at 37°C. The phage was isolated (from those cells which were infected) by touching a toothpick to plaques and suspending in 0.5 ml TE.

### 15.3.3. ISOLATION OF URACIL CONTAINING DNA

Uracil containing DNA was isolated from CJ236 by inoculating 50 ml LB medium containing chloramphenicol with 1.0 ml of an overnight culture of CJ236. The culture was incubated at 37°C with shaking until it reached an OD₆₀₀ of 0.3. At this point, the culture was infected with 50 µl of a -70°C stock culture that was previously infected with phage in order to amplify the production of single stranded DNA. The infected culture was allowed to grow overnight at these conditions. The following day, 30 ml of the culture was centrifuged at 16K rpm for 15 minutes. The supernatant containing the phage particles was transferred to a new tube and centrifuged a second time. The supernatant from this second centrifugation was treated with 150 mg RNase A at room temperature for 30 minutes. Single stranded DNA was precipitated by adding 7.5 ml of PEG solution (3.5 M ammonium acetate, 20% PEG 8000) and held on ice for 30 minutes. The tube was centrifuged and the supernatant was discarded. The pellet was suspended in 200 µl of high salt buffer (300 mM NaCl, 100 mM Tris, pH 8.0, 1 mM EDTA), held on ice for 30 minutes, and centrifuged in a microcentrifuge for 2 minutes. The supernatant was transferred to a new tube.

The phage was titered on CJ236 and MV1190 to determine whether infection was productive. Following confirmation of productive infection, the DNA was extracted with an equal volume of phenol, an equal volume of phenol-chloroform, and an equal volume of ether. The extracted DNA was precipitated with 1/10 volume 7.8 M ammonium acetate and 2.5 volumes ethanol at -20°C overnight. The tube was centrifuged for 15 minutes and the pellet was resuspended in 20 µl TE. This DNA is the single stranded uracil-containing DNA which was used as a template for the synthesis of the mutagenic strand.

### 15.3.4. KINASING OF OLIGONUCLEOTIDES

The purified oligonucleotides were kinased by treating 5 µg of each of the six oligonucleotides with T4 polynucleotide kinase and ATP to ensure efficient ligation of the two ends of the newly synthesized DNA strand.

### 15.3.5. SYNTHESIS OF THE MUTAGENIC STRAND

The synthesis of the mutagenic strand was carried out by adding 0.25 µg (0.1 pM) of the uracil-containing single stranded DNA template and 0.03 µg (3 pM) of each of the synthetic oligonucleotide primers. The primer was annealed to the single stranded template (final reaction volume 10 ml)in 1X annealing buffer (2 mM Tris-HCl, pH 7.4, 0.2 mM MgCl₂, 5 mM NaCl) in a water bath with an initial temperature of 70°C which was allowed to cool to 30°C. The reactions were then placed in an ice water bath and the following components were added to each: 1 µl 10X synthesis buffer (Final concentration = 0.4 mM each dNTP, 0.75 mM ATP, 17.5 mM Tris-HCl, pH 7.4, 3.75 mM MgCl₂, 21.5 mM DTT), 1 µl T4 DNA Ligase (2-5 units), and 1 µl T4 DNA Polymerase (1 unit). The reactions were incubated on ice for 5 minutes in order to stabilize the primer by initiation of DNA synthesis under conditions that favor the binding of the primer to the template. The reactions were then incubated at 25°C for 5 minutes and finally at 37°C for 90 minutes. Following the final incubation, 90 µl of stop buffer (10 mM Tris, pH 8.0, 10 mM EDTA) was added to each reaction and were placed at -20°C until use in the transfection of MV1190.

### 15.3.6. TRANSFECTION OF MV1190 CELLS

MV1190 cells were transfected with the products of the synthesis reactions by adding 3 µl and 9 µl of each reaction to 0.3 ml of- competent cells. The tubes were incubated on ice for 90 minutes, heat shocked at 42°C for 3 minutes, and then placed on ice. 50 and 100 µl of the transfected cells were added to tubes containing 0.3 ml of an overnight culture of MV1190, 50 µl 2% X-gal, 20 µl 100 mM IPTG, and 2.5 ml top agar (55°C). The mixture was vortexed and poured onto H-agar plates. The plates were incubated overnight at 37°C and observed for the formation of plaques the following morning. Those plaques that appeared blue did not contain the insert, while those that appeared clear were the plaques of interest.

### 15.3.7. ANALYSIS OF TRANSFORMANTS BY SEQUENCING

The clear plaques were picked by inserting a sterile Pasteur pipet into the agar and suspending the plug in 3 µl LB broth (24 plaques were chosen from each of the plates containing plaques). 100 ml of an overnight culture of MV1190 was added and the tubes were incubated with shaking overnight at 37°C. Following the incubation period, single stranded DNA was isolated from the cultures and this DNA was used in sequencing reactions.

Dideoxy sequencing was performed to confirm the presence of mutations. The sequencing kit used in this case was obtained from New England Biolabs. Each sequencing reaction was set up using 8 µl of the single stranded DNA to be sequenced, 1 µl of the appropriate primer, and 1 µl 10X sequencing buffer. The primer was annealed to the single stranded template by placing the tubes at 90°C and allowing them to cool to 30°C. 2 µl of the DNA-primer mixture was used in each individual sequencing reaction along with 2 µl of the termination mix (50 µl of the appropriate dNTP's and ddNTP plus 5 µl [α-³²P]dATP) and 2 µl of Klenow enzyme diluted to 0.1 units/ml. The reaction was incubated at room temperature for 15 minutes and 2 µl of a chase mixture was added that consisted of a dNTP mixture containing cold dATP and Klenow enzyme. This reaction was incubated again at room temperature for 15 minutes and 4 µl of dye mix was added to stop the reaction. The samples were denatured by boiling for 2.5 minutes and, placed in an ice water bath, and loaded onto a 6% polyacrylamide sequencing gel containing 7M urea. The gel was run at 55 watts for approximately 4 hours before it was dried under vacuum and placed in an X-ray film cassette for autoradiography.

Other sequencing kits were used to achieve the best results in conjunction with [α³⁵S] dATP. A sequencing kit specifically for use with single stranded DNA was obtained from IBI and a Pharmacia kit was used with T7 DNA polymerase rather than Klenow Enzyme in order to sequence mutants further from the point of primer annealing.

Transfection of MV1190 with the synthesis reaction products resulted in clear plaques on the plates containing Mu66Th, Mu66Th67I and Mu145Cy, Mu40Th, and Mu66Ty DNA. The control which was included consisted of a transfection with template DNA without a primer for synthesis of the second strand. This control revealed some plaques, but fewer than those in which a primer for mutagenesis was used.

### 15.4. CONSTRUCTION OF PLASMID YEp51NT1

Yeast shuttle vector YEp51 was modified to have ADH terminator sequences. The ADH terminator was inserted between the GAL10 promoter and the 2µ present on this vector. Specifically, plasmid YEp51 was digested with restriction enzyme Bcl1. The linearized DNA molecule was treated with Klenow enzyme and dNTPs to make it blunt ended. A double stranded oligonucleotide was ligated to the blunt ended plasmid. This oligonucleotide was obtained from BRL and contained sequences for restriction enzyme NotI. Ligation was carried out overnight at room temperature. DNA from the ligation reaction was precipitated using polyethylene glycol (PEG). This procedure removes all unligated oligonucleotides because only large DNA molecules are precipitated with PEG. After the PEG precipitation, DNA was cleaned by phenol extraction and ethanol precipitation. Pleasmid DNA was digested with NotI and HindIII.

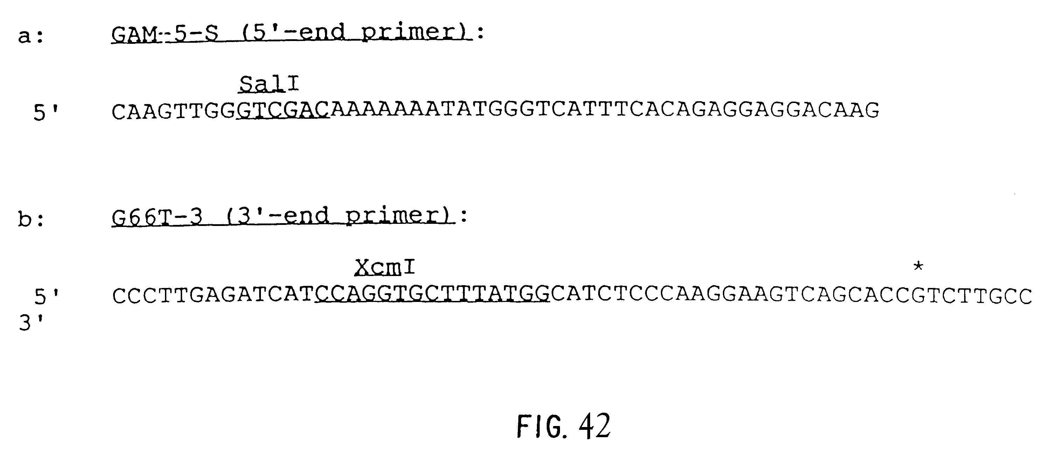

The ADH terminator was obtained from plasmid AAH5 (see Figure 8). Plasmid AAH5 was digested with restriction enzyme BamHI. DNA was blunt-ended with Klenow and dNTPs. Blunt-ended DNA was subjected to phenol extraction and ethanol precipitation. The above-mentioned double-stranded oligonucleotide was ligated to the blunt-ended plasmid. Ligation was carried out overnight at room termperature. DNA from the ligation was precipitated using polyethylene glycol (PEG). After the PEG precipitation, DNA was cleaned by phenol extraction and ethanol precipitation. DNA was then digested with restriction enzyme NotI and HindIII. A 400 bp NotI-HindIII fragment was isolated from a 1.0% agarose gel (1X TBE). DNA was electroeluted from the agarose slice and precipitated with ethanol. This purified DNA fragment was ligated to the above-mentioned plasmid. The ligation mixture was used to transform DH5α-cells. Transformed cells were spread on plates containing LB-media with 100 mg/L ampicillin. Plates were incubated overnight at 37°C. Colonies appearing on these plates were used to inoculate 5.0 ml LB-media containing 100 mg/L ampicillin. Cultures were grown at 37°C overnight. DNA was isolated from 1.5 ml of the overnight culture using the alkaline lysis procedure. Plasmid DNA was digested with restriction enzyme NotI and HindIII. The resulting plasmid was called YEp51NT1 and is shown in Figure 42.

### 15.5. CLONING OF VARIANT GLOBINS

The vector for cloning the mutated β-globin gene(s) was prepared by digesting plasmids YEP51T/G (supra, Section 8.4) or YEp51NT1/γ-Port (infra, 15.5.5.) with SalI and HindIII. The vector for cloning the γ-globin gene was YEp51NT1. This digestion results in two fragments (7300 and 500 bp); the 7300 bp fragment was isolated.

### 15.5.1. CLONING OF PORTO ALEGRE (9 Ala->Cys) γ-GLOBIN GENE

The Porto Alegre γ-globin was created by substituting two bases in the natural γ-globin sequence using PCR. The γ-globin gene was obtained as a 450 bp fragment. The 5' and 3' primers used for synthesizing the sequence are shown in Figure 47.

The mutated γ-globin gene obtained by PCR was digested with SalI and HindIII. This digested DNA fragment (450 bp) was purified by phenol extraction and ethanol precipitation. This purified 450 bp fragment obtained by PCR was ligated to the vector YEp51NT1 cut with SalI and HindIII. DNA ligation, E. coli transformation and DNA isolation was performed as described (supra, 15.1.). DNA isolated from the transformed cells was digested with restriction enzyme PstI. The results obtained from this analysis showed clones that had expected fragments (three fragments when digested with PstI; two fragments from vector without insert). This plasmid was called YEp51NT1/γ-Port.

### 15.5.2. CLONING OF THE CHICO (66 Lys->Thr) γ-GLOBIN GENE

The Chico γ-globin was created by substituting one base in the natural γ-globin gene using PCR. The γ-globin gene was isolated as two fragments. The 5'-end of the gene (SalI-XcmI) was obtained by PCR using plasmid pJW151 as template. The 5' and 3' primers used for synthesizing the sequence are shown in Figure 48.

The mutated fragment of the γ-globin gene was digested with SalI and XcmI. This digested DNA fragment (230 bp) was purified by phenol extraction and ethanol precipitation. The 3'-end of the γ-globin gene was isolated from plasmid YEp51NT1/γ-Port. Plasmid YEp51NT1/γ-Port was digested with restriction enzymes XcmI and HindIII. A 220 bp fragment was isolated. This purified 220 bp fragment along with the fragment obtained by PCR were ligated to the vector YEp51NT1 cut with SalI and HindIII. DNA ligation, E. coli transformation and DNA isolation were performed as described (supra, 15.1.). DNA isolated from the transformed cells was digested with restriction enzyme PstI. The results obtained from this analysis showed that one clone had expected fragments (three fragments when digested with PstI; two fragments from vector without insert). This plasmid was called pNT1/γ-Chi.

### 15.5.3. CLONING OF TITUSVILLE ζ-GLOBIN GENE (94 Asp->Asn).

The 94-Asn ζ-globin was created by substituting one base in the natural ζ-globin gene using PCR. The ζ-globin gene was isolated as two fragments. Both fragments were obtained by PCR. The 5'-end of the gene (SalI-BstEII) was obtained by PCR using plasmid 4p7-7 as template. The 5' and 3' primers used for synthesizing the sequence are shown in Figure 49.

The 3'-end of the gene (BstEII-HindIII) was obtained by PCR using plasmid 4p7-7 as template. The 5' and 3' primers used for synthesizing the sequence are shown in Figure 50.

A mutated fragment of the ζ-globin gene obtained by PCR was digested SalI and BstEII. This digested DNA fragment (330 bp) was purified by phenol extraction and ethanol precipitation. The 3'-end of the ζ-globin gene obtained by PCR was digested with restriction enzymes Bst EII and Hind III. A 100 bp fragment was isolated. Purified fragments (330 and 100 bp) were ligated to the vector YEp51NT1 cut with Sal I and HindIII. DNA ligation, E. coli transformation and DNA isolation were performed as described (supra, 15.1.). DNA isolated from the transformed cells was digested with restriction enzyme HindII. The results obtained from this analysis showed that one clone had expected fragments (five fragments when digested with HindII). This plasmid was called pNT1/Z95An.

### 15.6. EXPRESSION OF VARIANT GLOBINS

### 15.6.1. EXPRESSION OF GAMMA-GLOBIN PORTO ALEGRE IN YEAST

Yeast strain Sc1114 was transformed with plasmid YEp51NT1/γPort using electroporation. For the starter culture,cells were grown in minimal media containing 0.67% yeast nitrogen base, 1% raffinose, and supplemented with 20 mg/L each of adenine, histidine, uracil and tryptophan at 30°C in a shake flask to log phase. The starter culture was used to inoculate 500 ml of media containing 0.67% yeast nitrogen base, 3% glycerol, 2% lactic acid, 1% raffinose, 0.4% Tween-80, and supplemented with 20 mg/L each of adenine, histidine, uracil, and tryptophan. Incubation was at 30°C with shaking. The culture was induced by adding galactose to a final concentration of 2%. At induction, the pH was adjusted to 7.03 with KH₂PO₄ and hemin was added to a final concentration of 40 µg/ml. Samples were collected between two and 27 hours after induction.

The expressed globins were quantitated by Western Blot analysis using procedures described in Section 6.6. All samples contained detectable levels of globin (0.1-1.3% protein).

### 15.6.2. EXPRESSION OF GAMMA-GLOBIN CHICO IN YEAST

Yeast strain Sc340 was transformed with plasmid pNT1/γ-Chi using electroporation. For the starter culture,cells were grown in minimal media containing 0.67% yeast nitrogen base, 1% raffinose, and supplemented with 20 mg/L each of adenine, histidine, uracil and tryptophan at 30°C in a shake flask to log phase. The starter culture was used to inoculate 500 ml of media containing 0.67% yeast nitrogen base, 3% glycerol, 2% lactic acid, 1% raffinose, 0.4% Tween-80, and supplemented with 20 mg/L each of adenine, histidine, uracil, and tryptophan. Incubation was at 30°C with shaking. The culture was induced by adding galactose to a final concentration of 2%. At induction, the pH was adjusted to 7.06 with KH₂PO₄ and hemin was added to a final concentration of 40 µg/ml. Samples were collected between two and eight hours after induction.

The expressed globins were quantitated by Western Blot analysis using procedures described in Section 6.6. Globin was detected.

The whole yeast cell visible carbon monoxide difference spectrum is generated using a procedure adapted from the methods of Springer and Slager (1987, Proc. Natl. Acad. Sci. U.S.A. 84:8961). Approximately 1.2 ml of suspension of yeast with a final O.D. at 600 nm of. about 2.0 is prepared using 0.1 mM PO₄, pH 7.0, as a buffer. The suspension is then reduced with a small amount of sodium dithionite, vortexed, and allowed to sit for one minute. One ml is then removed and placed in a full length small volume cuvette. This suspension is used as the baseline for a scan in a single beam Beckman DU-70 Recording spectrophotomer from 400 to 500 nm. The cuvette is then removed and the suspension is bubbled steadily but not vigorously with oxygen-scrubbed carbon monoxide (CO) for two minutes. The suspension is mixed by gentle rocking for one minute and the spectrum from 400 to 500 nm is scanned. Lastly, the O.D. at 600 nm is measured on the same instrument.

If hemoglobin is present, the difference spectrum will produce a peak around 420 nm and a valley around 435 nm. A single peak at 420 nm does not indicate the presence of hemoglobin.

Functional hemoglobin was detected in this strain by this method.

### 16. EXAMPLE 11: COEXPRESSION OF ALPHA-GLOBIN AND GAMMA-GLOBIN IN YEAST BY COTRANSFORMATION

### 16.1. MATERIALS AND METHODS

Yeast strain Sc340 was used. The yeast cells were grown in a 500 ml YEPD broth with vigorous shaking at 30°c to an O.D. of 1.3-1.5. Cells from a 500 ml culture were collected by centrifugation at 5000 rpm for 5 min. at 4°C. The cells and the rotor were always kept cold. The cells are washed with cold sterile distilled water 2x and collected by centrifugation at 4°C. The cells were resuspended in 20 ml ice cold 1M sorbitol and mixed by pipetting. The cells were collected and resuspended in a final volume of 0.5-1 ml ice cold sorbitol (1M).

BioRad (Richmond, CA) Gene Pulser with Pulse Controller was used for electroporation. The 0.2-cm cuvettes were obtained from Bio Rad. 40 µl of yeast cells were transferred to a sterile Eppendorf tube. DNA (1-100 ng) in 5 ul TE was added to the cells. The mixture was incubated on ice for 5 min., transferred to a 0.2 cm cuvette and pulsed at 1.5 kV, 25 uF, 200 ohms for 5 msec. 250 ul cold 1M sorbitol was immediately added to the cuvette, mix the contents were gently mixed and the cells were plated on appropriate plates.

### 16.2. TRANSFORMATION OF YEAST STRAIN Sc340 WITH pNM-R-G-α1 AND YEp51T/G

Yeast strain Sc340 was transformed with plasmids pNM-R-G-α1 (supra 11.3) and YEp51T/G (supra 8.4.) using electroporation.

### 16.3. WESTERN BLOT ANALYSIS OF EXPRESSED GLOBIN

The expressed globins were quantitated by Western Blot analysis using procedures described in Section 6.6 (0.4% soluble protein).

### 16.4. DETECTION OF HEMOGLOBIN IN YEAST BY CARBON MONOXIDE DIFFERENCE SPECTRUM

The whole yeast cell visible carbon monoxide difference spectrum is generated using a procedure adapted from the methods of Springer and Slager (1987, Proc. Natl. Acad. Sci. U.S.A. 84:8961). Approximately 1.2 ml of suspension of yeast with a final O.D. at 600 nm of about 2.0 is prepared using 0.1 mM PO₄, pH 7.0, as a buffer. The suspension is then reduced with a small amount of sodium dithionite vortexed, and allowed to sit for one minute. One ml is then removed and placed in a full length small volume cuvette. This suspension is used as the baseline for a scan in a single beam Beckman DU-70 Recording spectrophotomer from 400 to 500 nm. The cuvette is then removed and the suspension is bubbled steadily but not vigorously with oxygen-scrubbed carbon monoxide (CO) for two minutes. The suspension is mixed by gentle rocking for one minute and the spectrum from 400 to 500 nm is scanned. Lastly, the O.D. at 600 nm is measured on the same instrument.

If hemoglobin is present, the difference spectrum will produce a peak around 420 nm and a valley around 435 nm. A single peak at 420 nm does not indicate the presence of hemoglobin.

Functional hemoglobin was detected in this strain by this method (10 µM heme).

### 17. EXAMPLE 12: COEXPRESSION OF ALPHA-GLOBIN AND GAMMAᵥₐₗ-GLOBIN IN A YEAST BY COTRANSFORMATION

### 17.1. MATERIALS AND METHODS.

Yeast strains Sc1113 was used. The yeast cells were grown in a 500 ml YEPD broth with vigorous shaking at 30°C to an O.D. of 1.3-1.5. Cells from a 500 ml culture were collected by centrifugation at 5000 rpm for 5 min. at 4°C. The cells and the rotor were always kept cold. The cells are washed with cold sterile distilled water 2x and collected by centrifugation at 4°C. The cells were resuspended in 20 ml ice cold 1M sorbitol and mixed by pipetting. The cells were collected and resuspended in a final volume of 0.5-1 ml ice cold sorbitol (1M).

BioRad (Richmond, CA) Gene Pulser with Pulse Controller was used for electroporation. The 0.2-cm cuvettes were obtained from Bio Rad. 40 µl of yeast cells were transferred to a sterile Eppendorf tube. DNA (1-100 ng) in 5 ul TE was added to the cells. The mixture was incubated on ice for 5 min., transferred to a 0.2 cm cuvette and pulsed at 1.5 kV, 25 uF, 200 ohms for 5 msec. 250 ul cold 1M sorbitol was immediately added to the cuvette, mix the contents were gently mixed and the cells were plated on appropriate plates.

### 17.2. TRANSFORMATION OF YEAST STRAINS Sc1113 AND Sc340 WITH pNM-R-G-α1 AND pNM-5-G-γᵥₐₗ1

Yeast strain Sc1113 was transformed with plasmids pNM-R-G-α1 (supra, 11.3) and pNM-5-G-γᵥₐₗ1 (supra, 9.4) using electroporation.

### 17.3. WESTERN BLOT ANALYSIS OF EXPRESSED GLOBIN

The expressed globins were quantitated by Western Blot analysis using procedures described in Section 6.6 (0.1% soluble protein).

### 17.4. SORET SPECTRA OF HEMOGLOBIN IN YEAST

The Soret spectrum indicated absorbance in the region of 418 nm which is characteristic of hemoglobin and is not present in control yeast that do not express globins.

### 18. EXAMPLE 13 COEXPRESSION OF ALPHA-GLOBIN AND GAMMA-GLOBIN PORTO ALEGRE IN A YEAST BY COTRANSFORMATION

### 18.1. MATERIALS AND METHODS

Yeast strain Sc340 was used. The yeast cells were grown in a 500 ml YEPD broth with vigorous shaking at 30°C to an O.D. of 1.3-1.5. Cells from a 500 ml culture were collected by centrifugation at 5000 rpm for 5 min. at 4°C. The cells and the rotor were always kept cold. The cells are washed with cold sterile distilled water 2x and collected by centrifugation at 4°C. The cells were resuspended in 20 ml ice cold 1M sorbitol and mixed by pipetting. The cells were collected and resuspended in a final volume of 0.5-1 ml ice cold sorbitol (1M).

BioRad (Richmond, CA) Gene Pulser with Pulse Controller was used for electroporation. The 0.2-cm cuvettes were obtained from Bio Rad. 40 µl of yeast cells were transferred to a sterile Eppendorf tube. DNA (1-100 ng) in 5 ul TE was added to the cells. The mixture was incubated on ice for 5 min., transferred to a 0.2 cm cuvette and pulsed at 1.5 kV, 25 uF, 200 ohms for 5 msec. 250 ul cold 1M sorbitol was immediately added to the cuvette, mix the contents were gently mixed and the cells were plated on appropriate plates.

### 18.2. TRANSFORMATION OF YEAST STRAINS Sc340 WITH pNM-R-G-α1 AND YEpNT1/γ-PORT

Yeast strain Sc340 was transformed with plasmids pNM-R-G-α1 (supra 11.3) and YEp51NT1/γ-PORT (supra, 15.5.1.) using electroporation.

### 18.3. WESTERN BLOT ANALYSIS OF EXPRESSED GLOBIN

The expressed globins were quantitated by Western Blot analysis using procedures described in Section 6.6.

### 18.4. DETECTION OF HEMOGLOBIN IN YEAST BY CARBON MONOXIDE DIFFERENCE SPECTRUM

The whole yeast cell visible carbon monoxide difference spectrum is generated using a procedure adapted from the methods of Springer and Slager (1987, Proc. Natl. Acad. Sci. U.S.A. 84:8961). Approximately 1.2 ml of suspension of yeast with a final O.D. at 600 nm of about 2.0 is prepared using 0.1 mM PO₄, pH 7.0, as a buffer. The suspension is then reduced with a small amount of sodium dithionite vortexed, and allowed to sit for one minute. One ml is then removed and placed in a full length small volume cuvette. This suspension is used as the baseline for a scan in a single beam Beckman DU-70 Recording spectrophotomer from 400 to 500 nm. The cuvette is then removed and the suspension is bubbled steadily but not vigorously with oxygen-scrubbed carbon monoxide (CO) for two minutes. The suspension is mixed by gentle rocking for one minute and the spectrum from 400 to 500 nm is scanned. Lastly, the O.D. at 600 nm is measured on the same instrument.

If hemoglobin is present, the difference spectrum will produce a peak around 420 nm and a valley around 435 nm. A single peak at 420 nm does not indicate the presence of hemoglobin.

Functional hemoglobin was detected in this strain by this method.

### 19. EXAMPLE 14: COEXPRESSION OF ZETA-GLOBIN TITUSVILLE AND GAMMA-GLOBIN IN YEAST BY COTRANSFORMATION

### 19.1. TRANSFORMATION OF YEAST STRAIN Sc1115 WITH pNT1/Z95An AND YEp51T/G

Yeast strain Sc1115 was transformed with plasmids YEp51T/G (supra 8.4.) and pNT1/Z95An (supra, 15.5.3.) using electroporation. For the starter culture, cells were grown in minimal media containing 0.67% yeast nitrogen base, 1% raffinose, and supplemented with 20 mg/L each of adenine, histidine, uracil and tryptophan at 30°C in a shake flask to log phase. The starter culture was used to inoculate 500 ml of media containing 0.67% yeast nitrogen base, 3% glycerol, 2% lactic acid, 1% raffinose, 0.4% Tween-80, and supplemented with 20 mg/L each of adenine, histidine, uracil, and tryptophan. Incubation was at 30°C with shaking. The culture was induced by adding galactose to a final concentration of 2%. At induction, the pH was adjusted to 7.06 with KH₂PO₄ and hemin was added to a final concentration of 40 µg/ml. Samples were collected between two and 30 hours after induction.

The expressed globins were quantitated by Western Blot analysis using procedures described in Section 6.6.

### 19.2. WESTERN BLOT ANALYSIS OF EXPRESSED GLOBIN

The expressed globins were quantitated by Western Blot analysis using procedures described in Section 6.6. Globin was detected at 0.2% of soluble protein.

### 19.3. DETECTION OF HEMOGLOBIN IN YEAST BY CARBON MONOXIDE DIFFERENCE SPECTRUM

The whole yeast cell visible carbon monoxide difference spectrum is generated using a procedure adapted from the methods of Springer and Slager (1987, Proc. Natl. Acad. Sci. U.S.A. 84:8961). Approximately 1.2 ml of suspension of yeast with a final O.D. at 600 nm of about 2.0 is prepared using 0.1 mM PO₄, pH 7.0, as a buffer. The suspension is then reduced with a small amount of sodium dithionite vortexed, and allowed to sit for one minute. One ml is then removed and placed in a full length small volume cuvette. This suspension is used as the baseline for a scan in a single beam Beckman DU-70 Recording spectrophotomer from 400 to 500 nm. The cuvette is then removed and the suspension is bubbled steadily but not vigorously with oxygen-scrubbed carbon monoxide (C)) for two minutes. The suspension is mixed by gentle rocking for one minute and the spectrum from 400 to 500 nm is scanned. Lastly, the O.D. at 600 nm is measured on the same instrument.

If hemoglobin is present, the difference spectrum will produce a peak around 420 nm and a valley around 435 nm. A single peak at 420 nm does not indicate the presence of hemoglobin.

Functional hemoglobin was detected in this strain by this method.

### 20. DEPOSIT OF MICROORGANISMS

The following yeast strains of the species Saccharomyces cereviseae carrying the listed plasmids were deposited with the Agricultural Research Culture Collection (NRRL), Peoria, IL.

| Accession | | | |
|---|---|---|---|
| Yeast strain | Plasmid | Number | Date of Deposit |
| 340g2P | YEpWB51WB/Port | Y-18640 | April 2, 1990 |
| 340VGTB | pNML-V-G-1 | Y-18641 | April 2, 1990 |
| 340g2G | YEp51T/G | Y-18695 | August 7, 1990 |
| 3405GgVAL | pNM-5-G-γᵥₐₗl | Y-18735 | October 16, 1990 |
| Sc1012-R-A-al | pNM-R-A-α1 | Y-18694 | August 7, 1990 |
| 1041GabCot1 | pNM-R-G-α1 | | March 27, 1991 |
| | pNML-V-G-1 | | |
| 1115X1VGab | pBM-V-X1-G-αβ | | March 27, 1991 |
| 340GagCot6 | pNM-R-G-α1 | | March 27, 1991 |
| | YEp51T/G | | |
| 1113GagVALCot5 | pNM-R-G-α1 | | March 27, 1991 |
| | pNM-5-G-γᵥₐₗ1 | | |
| 340g2GC | pNTl/γ-Chico | | March 27, 1991 |
| 1114g2GP | YEp51NT1/γ-PORT | | March 27, 1991 |
| 340g2ATi | pNT1/αTit | | March 27, 1991 |
| 340g2A104S | pNT1/α104S | | March 27, 1991 |
| 340g2BMo | pNT1/β-Mot | | March 27, 1991 |
| 340g2BTaS | pNT1/β-TaLiS | | March 29, 1991 |
| 340g2GPRA | pNM-R-G-α1 | | March 29, 1991 |
| | YEp51NT1/γ-PORT | | |
| 1090g2BChRA1 | pNM-R-G-α1 | | March 29, 1991 |
| | pNT1/γ-Chico | | |
| 1090g2BRa | pNT1/β-Ran | | March 29, 1991 |
| 340g2BMS | pNT1/β-Miss | | March 29, 1991 |
| 1113X8agVAL1 | pBM-V-X8-Gαγᵥₐₗ | | April 6, 1991 |
| 1041gaTPORTCot7 | pNM-R-G-α1 | | April 6, 1991 |
| | YEpWB51T/Port | | |
| 1115g2GTZTi | pNT1/Z95 | | April 6, 1991 |
| | YEp51T/G | | |

## Claims

1. A composition comprising a variant hemoglobin, in which the variant hemoglobin comprises:
(a) a variant globin chain or heme-binding fragment thereof expressed in a yeast, which is substantially homologous to:
i) a mammalian fetal gamma-globin chain or heme-binding fragment thereof
(b) an alpha-like globin chain or heme-binding fragment thereof expressed in a yeast, wherein the alpha-like globin chain is a mammalian embryonic zeta-globin chain; and
(c) heme; and wherein
the variant hemoglobin
(d) has the ability to bind to oxygen at a low oxygen affinity;
(e) has the ability to autopolymerize;
(f) is stable in alkali; or
(g) does not dissociate under physiological conditions;
and which composition
is free of erythrocyte membrane components and E. coli endotoxins,
wherein the substantially homologous variant globin chain or heme-binding fragment thereof is a variant in which a cDNA sequence encoding the variant globin chain or heme-binding fragment thereof has the ability to hybridize to a cDNA sequence encoding the mammalian fetal gamma-globin chain or heme-binding fragment thereof, under stringent conditions comprising 0.1X SSC and a temperature of about 65°C.

2. The composition of claim 1, in which; the variant hemoglobin has the ability to bind to oxygen at a low oxygen affinity.

3. The composition of claim 2, in which the variant globin chain or heme-binding fragment thereof comprises a threonine at the gamma-66 position.

4. The composition of claim 2, in which the variant globin chain or heme-binding fragment thereof comprises a valine at the gamma-1 position.

5. The composition of claim 1, in which the variant hemoglobin has the ability to autopolymerize.

6. The composition of claim 5, in which the variant globin chain or heme-binding fragment thereof comprises a cysteine at the gamma-9 position.

7. A composition comprising a variant hemoglobin, in which the variant hemoglobin comprises:
(a) a variant globin chain or heme-binding fragment thereof expressed in a yeast, which is substantially homologous to:
i) a mammalian embryonic zeta-globin chain or heme-binding fragment thereof,
(b) a beta-like globin chain or heme-binding fragment thereof expressed in a yeast, wherein the beta-like globin chain is a mammalian fetal gamma-globin chain, and
(c) heme; and wherein
the variant hemoglobin
(d) has the ability to bind to oxygen at a low oxygen affinity; or
(e) is stable in alkali;
and which composition is free of erythrocyte membrane components and E. coli endotoxins,
wherein the substantially homologous variant globin chain or heme-binding fragment thereof is a variant in which a cDNA sequence encoding the variant globin chain or heme-binding fragment thereof has the ability to hybridize to a cDNA sequence encoding the mammalian embryonic zeta-globin chain or heme-binding fragment thereof under stringent conditions comprising 0.1X SSC and a temperature of about 65°C.

8. The composition of claim 7, in which the variant hemoglobin has the ability to bind oxygen at a low oxygen affinity.

9. The composition of claim 8, in which the variant globin chain or heme-binding fragment thereof comprises an asparagine at the zeta-94 position.

10. A recombinant DNA vector capable of expressing in a yeast cell a globin chain I and a globin chain II, which vector comprises:
(a) a cDNA sequence I encoding the globin chain I;
(b) a yeast transcriptional promoter upstream of the sequence I which regulates the transcription of the sequence I;
(c) a cDNA sequence II encoding the globin chain II;
(d) a yeast transcriptional promoter upstream of the sequence II which regulates the transcription of the sequence II;
(e) a sequence encoding a yeast selectable marker or functionally active portion thereof; and
(f) a yeast replication origin or functionally active portion thereof;
wherein the globin chain I is
(i) a mammalian embryonic zeta-globin chain or heme-binding fragment thereof or a variant zeta globin chain or heme-binding fragment thereof which is substantially homologous to the zeta globin chain or heme-binding fragment thereof, wherein the substantially homologous variant zeta globin chain or heme-binding fragment thereof is a variant in which a DNA sequence encoding the variant zeta globin chain or heme-binding fragment thereof has the ability to hybridize to a DNA sequence encoding the zeta globin chain or heme-binding fragment thereof under stringent conditions comprising 0.1X SSC and a temperature of about 65°C; and
wherein the globin chain II is
(ii) a mammalian fetal gamma-globin chain, or heme-binding fragment thereof or a variant gamma globin chain or heme-binding fragment thereof which is substantially homologous to the gamma globin chain or heme-binding fragment thereof, wherein the substantially homologous variant gamma globin chain or heme-binding fragment thereof is a variant in which a DNA sequence encoding the variant gamma globin chain or heme-binding fragment thereof has the ability to hybridize to a DNA sequence encoding the gamma globin chain or heme-binding fragment thereof under stringent conditions comprising 0.1X SSC and a temperature of about 65°C.

11. The recombinant DNA vector of claim 10 which additionally comprises:
(g) a transcription termination sequence located downstream from the cDNA sequence I which terminates the transcription of the cDNA sequence I.

12. The recombinant DNA vector of claim 10 which additionally comprises:
(h) a yeast transcriptional promoter upstream of the cDNA sequence II which regulates the transcription of the cDNA sequence II.

13. The recombinant DNA vector of any one of claims 12, which additionally comprises:
(i) a transcription termination sequence located downstream from the cDNA sequence II which terminates the transcription of the cDNA sequence II.

14. The recombinant DNA vector of any one of claims 10-13, in which the yeast transcriptional promoter is a yeast inducible promoter.

15. The recombinant DNA vector of claim 14 in which the yeast inducible promoter is a unidirectional promoter.

16. The recombinant DNA vector of claim 14, in which the yeast inducible promoter is a bi-directional promoter.

17. The recombinant DNA vector of any one of claims 10-13, in which the yeast transcriptional promoter is a yeast constitutive promoter.

18. The recombinant DNA vector of any one of claims 10-13, in which the yeast transcriptional promoter comprises: (i) a sequence containing a transcriptional regulatory region of an inducible promoter; and (ii) a sequence containing a transcription initiation region of a constitutive promoter, wherein the sequence containing the transcriptional regulatory region of an inducible promoter is located upstream from the sequence containing the transcription initiation region of a constitutive promoter.

19. The recombinant DNA vector of claim 18, in which the transcriptional regulatory region is an upstream activating sequence of the yeast inducible promoter.

20. The recombinant DNA vector of any one of claims 10-13, in which the yeast selectable marker is a LEU2 gene.

21. The recombinant DNA vector of any one of claims 10-13, in which the yeast selectable marker is a leu2d gene.

22. The recombinant DNA vector of any one of claims 10-13, in which the yeast selectable marker is a URA3

23. The recombinant DNA vector of any one of claims 10-13, in which the yeast replication origin is a yeast 2µ plasmid replication system.

24. The recombinant DNA vector of any one of claims 10-13, in which the yeast replication origin is an autonomous replicating sequence.

25. A yeast cell containing the recombinant DNA vector of any one of claims 10 to 24.

26. A yeast cell containing a recombinant DNA vector I capable of expressing a globin chain I in the yeast cell, and a recombinant DNA vector II capable of expressing a globin chain II in the yeast cell wherein globin chains I and II are as defined in Claim 10, wherein
(a) the recombinant DNA vector I expresses the globin chain I in the yeast cell and comprises:
(i) a yeast transcription promoter I;
(ii) a cDNA sequence I which encodes the globin chain I and is located downstream from the promoter I, wherein the promoter I regulates the transcription of the sequence I;
(iii) a yeast selectable marker I or functionally active portion thereof;
(iv) a yeast replication origin I or functionally active portion thereof; and
(v) a transcription termination sequence I located downstream from the sequence I; and
(b) the recombinant DNA vector II expresses the globin II chain in the yeast cell and comprises:
(i) a yeast transcription promoter II;
(ii) a cDNA sequence II which encodes the globin chain II, and is located downstream from the promoter II, wherein the promoter II regulates the transcription of the sequence II;
(iii) a yeast selectable marker II or functionally active portion thereof;
(iv) a yeast replication origin II or functionally active portion thereof; and
(v) a transcription termination sequence II located downstream from the sequence II;

27. The yeast cell of claim 26 in which
(a) the globin chain I is a variant alpha-like globin chain which is substantially homologous to a human embryonic zeta-globin chain and comprises an asparagine at the zeta-94 position; the promoter I is a GAL10 promoter; the DNA sequence encoding the variant alpha-like globin chain has the ability to hybridize to a DNA sequence encoding the human embryonic zeta-globin chain under stringent conditions comprising 0.1X SSC and a temperature of about 65°C; the selectable marker I is a LEU2 gene; the replication origin I is a 2µ plasmid replication system; and the termination sequence I comprises a transcription termination region of an alcohol dehydrogenase I gene; and
(b) the globin chain II is a human fetal gamma-globin chain; the promoter II is a GAL10 promoter; the selectable marker II is a LEU2 gene; the replication origin is a 2µ plasmid replication system; and the termination sequence II comprises a transcription termination region of an alcohol dehydrogenase I gene.

28. The yeast cell of claim 27 which is a *Saccharomyces cerevisiae*.

29. A method for producing a globin chain I and globin chain II in a yeast cell comprising:
culturing the yeast cell, which yeast cell contains the recombinant DNA vector of any one of claims 10-24, in an appropriate medium such that the globin chain I and globin chain II are expressed.

30. A method for producing a hemoglobin comprising a globin chain I and a globin chain II, wherein the globin chain I and globin chain II are as defined in claim 10 which method comprises the steps of:
(a) culturing a yeast cell I such that the globin chain I is expressed, which yeast cell I contains a recombinant DNA vector comprising:
(i) a sequence I encoding the globin chain I,
(ii) a yeast transcriptional promoter located upstream of the sequence I which regulates the transcription of the sequence I,
(iii) a sequence encoding a yeast selectable marker or functionally active portion thereof, and
(iv) a yeast replication origin or functionally active portion thereof;
(b) culturing a yeast cell II such that the globin chain II is expressed, which yeast cell II contains a recombinant DNA vector comprising:
(i) a sequence II encoding the globin chain II,
(ii) a yeast transcriptional promoter located upstream of the sequence II which regulates the transcription of the sequence II,
(iii) a sequence encoding a yeast selectable marker or functionally active portion thereof, and
(iv) a yeast replication origin or functionally active portion thereof;
(c) isolating the globin chain I from the yeast cell I;
(d) isolating the globin chain II from the yeast cell II; and
(e) combining the isolated globin chain I and the isolated globin chain II, with a source of heme to form the hemoglobin.

31. A method for producing a hemoglobin comprising a globin chain I and a globin chain II wherein the globin chains I and II are as defined in claim 10 which method comprises the steps of:
(a) culturing a yeast cell such that the globin chain I and globin chain II are expressed, which yeast cell contains a recombinant DNA vector comprising:
(i) a cDNA sequence I encoding the globin chain I,
(ii) a cDNA sequence II encoding the globin chain II,
(iii) a yeast transcriptional promoter located upstream of the sequence I which regulates the transcription of the sequence I,
(iv) a yeast transcriptional promoter located upstream of the sequence II which regulates the transcription of the sequence II,
(v) a sequence encoding a yeast selectable marker or functionally active portion thereof, and
(vi) a yeast replication origin or functionally active portion thereof;
(b) isolating the globin chain I and globin chain II from the yeast cell; and
(c) combining the globin chain I and globin chain II with a source of heme to form the hemoglobin.

32. A method for producing in a yeast cell a hemoglobin comprising a globin chain I and a globin chain II wherein the globin chain I and globin chain II are as defined in claim 10, which method comprises:
culturing the yeast cell in an appropriate medium such that the globin chain I and globin chain II are expressed and assembled together with heme in the yeast cell to form the hemoglobin, which yeast cell contains a recombinant DNA vector comprising:
(i) a cDNA sequence I encoding the globin chain I,
(ii) a cDNA sequence II encoding the globin chain II,
(iii) a yeast transcriptional promoter located upstream of the sequence I which regulates the transcription of the sequence I,
(iv) a transcriptional promoter located upstream of the sequence II which regulates the transcription of the sequence II,
(v) a sequence encoding a yeast selectable marker or functionally active portion thereof, and
(vi) a yeast replication origin or functionally active portion thereof.

33. A method for producing in a yeast cell a hemoglobin comprising a globin chain I and a globin chain II, wherein the global chains I and II are as defined in claim 10 which method comprises:
culturing the yeast cell in an appropriate medium such that the globin chain I and globin chain II are expressed and assembled together with heme in the yeast cell to form the hemoglobin, which yeast cell contains a recombinant DNA vector I and a recombinant DNA vector II, which recombinant DNA vector I comprises:
(i) a cDNA sequence I encoding the globin chain I;
(ii) a yeast transcriptional promoter located upstream of the sequence I which regulates the transcription of the sequence I;
(iii) a sequence encoding a yeast selectable marker or functionally active portion thereof; and
(iv) a yeast replication origin or functionally active portion thereof; and
which recombinant DNA vector II comprises:
(v) a cDNA sequence II encoding a globin chain II;
(vi) a yeast transcriptional promoter located upstream of the sequence II which regulates the transcription of the sequence II;
(vii) a sequence encoding a yeast selectable marker or functionally active portion thereof; and
(viii) a yeast replication origin or functionally active portion thereof.

34. A method for producing a hemoglobin comprising a globin chain I and a globin chain II wherein the globin chains I and II are as defined in claim 10, which method comprises the steps of:
(a) culturing a yeast cell in an appropriate medium such that the globin chain I and globin chain II are expressed, which yeast cell contains a recombinant DNA vector I and a recombinant DNA vector II, which recombinant DNA vector I comprises:
(i) a cDNA sequence I encoding the globin chain I,
(ii) a yeast transcriptional promoter located upstream of the sequence I which regulates the transcription of the sequence I,
(iii) a sequence encoding a yeast selectable marker or functionally active portion thereof, and
(iv) a yeast replication origin or functionally active portion thereof; and
which recombinant DNA vector II comprises:
(v) a cDNA sequence II encoding the globin chain II,
(vi) a yeast transcriptional promoter located upstream of sequence II which regulates the transcription of the sequence II,
(vii) a sequence encoding a yeast selectable marker or functionally active portion thereof, and
(viii) a yeast replication origin or functionally active portion thereof;
(b) isolating the globin chain I and globin chain II; and
(c) combining the globin chain I and globin chain II with a source of heme to form the hemoglobin.

35. The composition of any one of claims 1-9 or produced according to the method of any one of claims 30 to 34 for its use for supplementing the oxygen-carrying capacity of a patient's blood.

## Patentansprüche

1. Zusammensetzung, die eine Hämoglobin-Abart umfaßt, bei der die Hämoglobin-Abart aufweist:
(a) eine Abart der Globinkette oder des Häm-bindenden Fragments davon, die in einer Hefe exprimiert wurden, die im wesentlichen homolog ist zu:
(i) einer fötalen Säugetier-Gamma-Globinkette oder dem Häm-bindenden Fragment davon.
(b) eine Alpha-artige Globinkette oder das Häm-bindende Fragment davon, exprimiert in einer Hefe, wobei die Alpha-artige Globinkette eine embryonale Säugetier-Zeta-Globinkette ist; und
(c) Häm; und worin die Hämoglobin-Abart
(d) die Fähigkeit aufweist, Sauerstoff mit einer niedrigen Sauerstoffaffinität zu binden;
(e) die Fähigkeit zur Autopolymerisation aufweist;
(f) in Alkali stabil ist; oder
(g) unter physiologischen Bedingungen nicht dissoziiert,
und wobei die Zusammensetzung
frei von Erythrozytenmembran-Komponenten und E. coli-Endotoxinen ist,
wobei die im wesentlichen homologe Abart der Globinkette oder des Häm-bindenden Fragments davon eine Abart ist, bei der eine cDNA-Sequenz, die für die Abart der Globinkette oder des Häm-bindenden Fragments davon codiert, die Fähigkeit hat, mit einer cDNA-Sequenz, die für die fötale Säugetier-Gamma-Globinkette oder das Häm-bindende Fragment davon codiert, unter stringenten Bedingungen, die 0,1 X SSC und eine Temperatur von etwa 65°C umfassen, zu hybridisieren.

2. Zusammensetzung nach Anspruch 1, bei der die Hämoglobin-Abart die Fähigkeit aufweist, Sauerstoff bei einer niedrigen Sauerstoffaffinität zu binden.

3. Zusammensetzung nach Anspruch 2, bei dem die Abart der Globinkette oder des Häm-bindenden Fragments davon ein Threonin in der Gamma-66-Stellung aufweist.

4. Zusammensetzung nach Anspruch 2, bei dem die Abart der Globinkette oder des Häm-bindenden Fragments davon ein Valin in der Gamma-1-Stellung aufweist.

5. Zusammensetzung nach Anspruch 1, bei der die Hämoglobin-Abart die Fähigkeit zur Autopolymerisation aufweist.

6. Zusammensetzung nach Anspruch 5, bei der die Abart der Globinkette oder des Häm-bindenden Fragments davon ein Cystein in der Gamma-9-Stellung aufweist.

7. Zusammensetzung, die eine Abart des Hämoglobins umfaßt, wobei die Abart des Hämoglobins aufweist:
(a) eine Abart der Globinkette oder des Häm-bindenden Fragments davon, exprimiert in einer Hefe, die im wesentlichen homolog ist zu:
(i) einer embryonalen Säugetier-Zeta-Globin-Kette oder dem Häm-bindenden Fragment davon,
(b) eine Beta-artige Globinkette oder das Häm-bindende Fragment davon, exprimiert in einer Hefe, wobei die Beta-artige Globinkette eine fötale Säugetier-Gamma-Globinkette ist; und
(c) Häm; und worin die Hämoglobin-Abart
(d) die Fähigkeit aufweist, Sauerstoff mit einer niedrigen Sauerstoffaffinität zu binden; oder
(e) in Alkali stabil ist;
und wobei die Zusammensetzung frei von Erythrozytenmembran-Komponenten und E. coli-Endotoxinen ist,
wobei die im wesentlichen homologe Abart der Globinkette oder des Häm-bindenden Fragments davon eine Abart ist, bei der eine cDNA-Sequenz, die für die Abart der Globinkette oder des Häm-bindenden Fragments davon codiert, die Fähigkeit aufweist, an eine cDNA-Sequenz, die für die embryonale Säugetier-Zeta-Globinkette oder das Häm-bindende Fragment davon codiert, unter stringenten Bedingungen, die 0,1 X SSC und eine Temperatur von etwa 65°C umfassen, zu hybridisieren.

8. Zusammensetzung nach Anspruch 7, bei der die Hämoglobin-Abart die Fähigkeit aufweist, Sauerstoff mit einer niedrigen Sauerstoffaffinität zu binden.

9. Zusammensetzung nach Anspruch 8, bei der die Abart der Globinkette oder des Häm-bindenden Fragments davon ein Asparagin in der Zeta-94-Stellung aufweist.

10. Rekombinanter DNA-Vektor, der in der Lage ist, in einer Hefezelle eine Globinkette I und eine Globinkette II zur Expression zu bringen, wobei der Vektor aufweist:
(a) eine cDNA-Sequenz I, die für die Globinkette I codiert;
(b) einen Hefe-Transkriptionspromotor stromauf der Sequenz I, der die Transkription der Sequenz I reguliert;
(c) eine cDNA-Sequenz II, die für die Globinkette II codiert;
(d) einen Hefe-Transkriptionspromotor stromauf der Sequenz II, der die Transkription der Sequenz II reguliert;
(i) eine Sequenz, die für einen Hefe-Selektiermarker oder einen funktional aktiven Abschnitt davon codiert; und
(f) einen Hefe-Replikationsursprung oder einen funktional aktiven Teil davon;
wobei die Globinkette I eine embryonale Säugetier-Zeta-Globinkette oder das Häm-bindende Fragment davon oder eine Abart der Beta-Globinkette oder des Häm-bindenden Fragments davon ist, die im wesentlichen homolog zu der Beta-Globinkette oder dem Häm-bindenden Fragment davon ist, wobei die im wesentlichen homologe Abart der Beta-Globinkette oder des Häm-bindenden Fragments davon eine Abart ist, bei der eine DNA-Sequenz, die für die Abart der Beta-Globinkette oder des Häm-bindenden Fragments davon codiert, die Fähigkeit hat, mit einer DNA-Sequenz, die für die Beta-Globinkette oder das Häm-bindende Fragment davon codiert, unter stringenten Bedingungen, die 0,1 X SSC und eine Temperatur von etwa 65°C umfassen, zu hybridisieren; und
wobei die Globinkette II ist:
(ii) eine fötale Säugetier-Gamma-Globinkette oder das Häm-bindende Fragment davon oder eine Abart der Gamma-Globinkette oder des Häm-bindenden Fragments davon, die im wesentlichen homolog zu der Gamma-Globinkette oder dem Häm-bindenden Fragment davon ist, wobei die im wesentlichen homologe Abart der Gamma-Globinkette oder des Häm-bindenden Fragments davon eine Abart ist, bei der eine DNA-Sequenz, die für die Abart der Gamma-Globinkette oder des Häm-bindenden Fragments davon codiert, die Fähigkeit aufweist, mit einer DNA-Sequenz, die für die Gamma-Globinkette oder das Häm-bindende Fragment davon codiert, unter stringenten Bedingungen, die 0,1 X SSC und eine Temperatur von etwa 65°C umfassen, zu hybridisieren.

11. Rekombinanter DNA-Vektor von Anspruch 10, der zusätzlich aufweist:
(g) eine Transkriptions-Terminations-Sequenz, die stromab der cDNA-Sequenz I angeordnet ist, die die Transkription der cDNA-Sequenz I terminiert.

12. Rekombinanter DNA-Vektor von Anspruch 10, der zusätzlich aufweist:
(h) eine Hefe-Transkriptions-Promotor stromauf der cDNA-Sequenz II, der die Transkription der cDNA-Sequenz II reguliert.

13. Rekombinanter DNA-Vektor nach Anspruch 12, der zusätzlich aufweist:
(i) eine Transkriptions-Terminations-Sequenz, die stromab der cDNA-Sequenz II angeordnet ist, die die Transkription der cDNA-Sequenz II terminiert.

14. Rekombinanter DNA-Vektor nach irgendeinem der Ansprüche 10 bis 13, bei dem der Hefe-Transkriptions-Promotor ein induzierbarer Hefe-Promotor ist.

15. Rekombinanter DNA-Vektor nach Anspruch 14, bei dem der induzierbare Hefe-Promotor ein unidirektioneller Promotor ist.

16. Rekombinanter DNA-Vektor nach Anspruch 14, bei dem der induzierbare Hefe-Promotor ein bidirektioneller Promotor ist.

17. Rekombinanter DNA-Vektor nach irgendeinem der Ansprüche 10 bis 13, bei dem der Hefe-Transkriptions-Promotor ein konstitutiver Hefe-Promotor ist.

18. Rekombinanter DNA-Vektor nach irgendeinem der Ansprüche 10 bis 13, bei dem der Hefe-Transkriptions-Promotor aufweist: (i) eine Sequenz, die einen Transkriptions-Regelabschnitt eines induzierbaren Promotors enthält; und (ii) eine Sequenz, die einen Transkriptions-Initiationsabschnitt eines konstitutiven Promotors enthält, wobei die Sequenz, die den Transkriptions-Regelabschnitt eines induzierbaren Promotors enthält, stromauf der Sequenz angeordnet ist, die den Transkriptions-Initiationsabschnitt eines konstitutiven Promotors enthält.

19. Rekombinanter DNA-Vektor nach Anspruch 18, bei dem der Transkriptions-Regelabschnitt eine Stromauf-Aktivierungssequenz des induzierbaren Hefe-Promotors ist.

20. Rekombinanter DNA-Vektor nach irgendeinem der Ansprüche 10 bis 13, bei dem der Hefe-selektierbare Marker ein LEU2-Gen ist.

21. Rekombinanter DNA-Vektor nach irgendeinem der Ansprüche 10 bis 13, bei dem der Hefe-selektierbare Marker ein LEU2d-Gen ist.

22. Rekombinanter DNA-Vektor nach irgendeinem der Ansprüche 10 bis 13, bei dem der Hefe-selektierbare Marker ein URA3-Gen ist.

23. Rekombinanter DNA-Vektor nach irgendeinem der Ansprüche 10 bis 13, bei dem der Hefe-Replikationsursprung ein Hefe-2µ-Plasmid-Replikationssystem ist.

24. Rekombinanter DNA-Vektor nach irgendeinem der Ansprüche 10 bis 13, bei dem der Hefe-Replikationsursprung eine autonome Replikationssequenz ist.

25. Hefezelle, die den rekombinanten DNA-Vektor nach irgendeinem der Ansprüche 10 bis 24 enthält.

26. Hefezelle, die einen rekombinanten DNA-Vektor I, der in der Lage ist, die Expression einer Globinkette I in der Hefezelle zu bewirken, und einen rekombinanten DNA-Vektor II, der in der Lage ist, die Expression einer Globinkette II in der Hefezelle zu bewirken, enthält, wobei die Globinketten I und II wie in Anspruch 10 definiert sind, wobei
(a) der rekombinante DNA-Vektor I die Expression der Globinkette I in der Hefezelle bewirkt und aufweist:
(i) einen Hefe-Transkriptions-Promotor I;
(ii) eine cDNA-Sequenz I, die für die Globinkette I codiert und stromab des Promotors I angeordnet ist, wobei der Promotor I die Transkription der Sequenz I reguliert,
(iii) einen Hefe-selektierbaren Marker I oder einen funktional aktiven Abschnitt davon;
(iv) einen Hefe-Replikationsursprung I oder einen funktional aktiven Abschnitt davon; und
(v) eine Transkriptions-Terminations-Sequenz I, die stromab der Sequenz I angeordnet ist; und
(b) der rekombinante DNA-Vektor II die Expression der Globin II-Kette in der Hefezelle bewirkt und aufweist:
(i) einen Hefe-Transkriptions-Promotor II;
(ii) eine cDNA-Sequenz II, die für die Globinkette II codiert und stromab des Promotors II angeordnet ist, wobei der Promotor II die Transkription der Sequenz II reguliert,
(iii) einen Hefe-selektierbaren Marker II oder einen funktional aktiven Abschnitt davon;
(iv) einen Hefe-Replikationsursprung II oder einen funktional aktiven Abschnitt davon; und
(v) eine Transkriptions-Terminations-Sequenz II, die stromab der Sequenz II angeordnet ist.

27. Hefezelle nach Anspruch 26, bei der
(a) die Globinkette I eine Abart der Alpha-artigen Globinkette ist, die im wesentlichen homolog zu einer embryonalen humanen Zeta-Globinkette ist und ein Asparagin in der Zeta-94-Position aufweist; der Promotor I ein GAL10-Promotor ist; die DNA-Sequenz, die für die Abart der Alpha-artigen Globinkette codiert, die Fähigkeit hat, an eine DNA-Sequenz, die für die embryonale humane Zeta-Globinkette codiert, unter stringenten Bedingungen, die 0,1 X SSC und eine Temperatur von etwa 65°C umfassen, zu hybridisieren; der selektierbare Marker I ein LEU2-Gen ist; der Replikationsursprung I ein 2µ-Plasmid-Replikationssystem ist; und die Terminations-Sequenz I einen Transkriptions-Terminationsabschnitt eines Alkoholdehydrogenase I-Gens umfaßt;
(b) die Globinkette II eine fötale humane Gamma-Globinkette ist; der Promotor II ein GAL10-Promotor ist; der selektierbare Marker II ein LEU2-Gen ist; der Replikationsursprung ein 2µ-Plasmid-Replikationssystem ist; und die Terminations-Sequenz II einen Transkriptions-Terminationsabschnitt eines Alkoholdehydrogenase I-Gens umfaßt.

28. Hefezelle nach Anspruch 27, die eine Saccharomyces cerevisiae ist.

29. Verfahren zur Herstellung einer Globinkette I und einer Globinkette II in einer Hefezelle, das umfaßt:
Kultivieren der Hefezelle, wobei die Hefezelle den rekombinanten DNA-Vektor nach irgendeinem der Ansprüche 10 bis 24 enthält, in einem geeigneten Medium, so daß die Globinkette I und die Globinkette II exprimiert werden.

30. Verfahren zur Erzeugung eines Hämoglobins, das eine Globinkette I und eine Globinkette II aufweist, wobei die Globinketten I und II wie in Anspruch 10 definiert sind, wobei das Verfahren die Schritte umfaßt:
(a) Kultivieren einer Hefezelle I so, daß die Globinkette I exprimiert wird, wobei die Hefezelle I einen rekombinanten DNA-Vektor enthält, der aufweist:
(i) eine Sequenz I, die für die Globinkette I codiert,
(ii) einen Hefe-Transkriptions-Promotor, der stromauf der Sequenz I angeordnet ist, der die Transkription der Sequenz I reguliert,
(iii) eine Sequenz, die für einen Hefe-selektierbaren Marker oder einen funktional aktiven Abschnitt davon codiert, und
(iv) einen Hefe-Replikationsursprung oder einen funktional aktiven Abschnitt davon;
(b) Kultivieren einer Hefezelle II so, daß die Globinkette II exprimiert wird, wobei die Hefezelle II einen rekombinanten DNA-Vektor enthält, der aufweist:
(i) eine Sequenz II, die für die Globinkette II codiert,
(ii) einen Hefe-Transkriptions-Promotor, der stromauf der Sequenz II angeordnet ist, der die Transkription der Sequenz II reguliert,
(iii) eine Sequenz, die für einen Hefe-selektierbaren Marker oder einen funktional aktiven Abschnitt davon codiert, und
(iv) einen Hefe-Replikationsursprung oder einen funktional aktiven Abschnitt davon;
(c) Isolieren der Globinkette aus der Hefezelle I;
(d) Isolieren der Globinkette aus der Hefezelle II; und
(e) Kombinieren der isolierten Globinkette I und der isolierten Globinkette II mit einer Häm-Quelle, um das Hämoglobin zu bilden.

31. Verfahren zur Herstellung eines Hämoglobins, das eine Globinkette I und eine Globinkette II umfaßt, wobei die Globinketten I und II wie in Anspruch 10 definiert sind, wobei das Verfahren die Schritte umfaßt:
(a) Kultivieren einer Hefezelle auf eine solche Weise, daß die Globinkette I und die Globinkette II exprimiert werden, wobei die Hefezelle einen rekombinanten DNA-Vektor enthält, der aufweist:
(i) eine cDNA-Sequenz I, die für die Globinkette I codiert,
(ii) eine cDNA-Sequenz II, die für die Globinkette II codiert,
(iii) einen Hefe-Transkriptions-Promotor, der stromauf der Sequenz I angeordnet ist, der die Transkription der Sequenz I reguliert,
(iv) einen Hefe-Transkriptions-Promotor, der stromauf der Sequenz II angeordnet ist, der die Transkription der Sequenz II reguliert,
(v) eine Sequenz, die für einen Hefe-selektierbaren Marker oder einen funktional aktiven Abschnitt davon codiert, und
(vi) einen Hefe-Replikationsursprung oder einen funktional aktiven Abschnitt davon;
(b) Isolieren der Globinkette I und der Globinkette II aus der Hefezelle; und
(c) Kombinieren der Globinkette I und der Globinkette II mit einer Häm-Quelle, um das Hämoglobin zu bilden.

32. Verfahren zur Herstellung eines Hämoglobins, das eine Globinkette I und eine Globinkette II aufweist, in einer Hefezelle, wobei die Globinketten I und II wie in Anspruch 10 definiert sind, wobei das Verfahren umfaßt:
(a) Kultivieren der Hefezelle in einem geeigneten Medium, so daß die Globinkette I und die Globinkette II exprimiert und mit Häm in der Hefezelle assoziiert werden, um das Hämoglobin zu bilden, wobei die Hefezelle einen rekombinanten DNA-Vektor enthält, der aufweist:
(i) eine cDNA-Sequenz I, die für die Globinkette I codiert,
(ii) eine cDNA-Sequenz II, die für die Globinkette II codiert,
(iii) einen Hefe-Transkriptions-Promotor, der stromauf der Sequenz I angeordnet ist, der die Transkription der Sequenz I reguliert,
(iv) einen Transkriptions-Promotor, der stromauf der Sequenz II angeordnet ist, der die Transkription der Sequenz II reguliert,
(v) eine Sequenz, die für einen Hefe-selektierbaren Marker oder einen funktional aktiven Abschnitt davon codiert, und
(vi) einen Hefe-Replikationsursprung oder einen funktional aktiven Abschnitt davon.

33. Verfahren zur Herstellung eines Hämoglobins, das eine Globinkette I und eine Globinkette II aufweist, in einer Hefe, wobei die Globinketten I und II wie in Anspruch 10 definiert sind, wobei das Verfahren umfaßt:
Kultivieren der Hefezelle in einem geeigneten Medium, so daß die Globinkette I und die Globinkette II exprimiert und in der Hefezelle unter Bildung des Hämoglobins mit Häm assoziiert werden, wobei die Hefezelle einen rekombinanten DNA-Vektor I und einen rekombinanten DNA-Vektor II enthält, wobei der rekombinante DNA-Vektor I aufweist:
(i) eine cDNA-Sequenz I, die für die Globinkette I codiert,
(ii) einen Hefe-Transkriptions-Promotor, der stromauf der Sequenz I angeordnet ist, der die Transkription der Sequenz I reguliert;
(iii) eine Sequenz, die für einen Hefe-selektierbaren Marker oder einen funktional aktiven Abschnitt davon codiert; und
(iv) einen Hefe-Replikationsursprung oder einen funktional aktiven Abschnitt davon; und
wobei der rekombinante DNA-Vektor II aufweist:
(v) eine cDNA-Sequenz II, die für eine Globinkette II codiert;
(vi) einen Hefe-Transkriptions-Promotor, der stromauf der Sequenz II angeordnet ist, der die Transkription der Sequenz II reguliert;
(vii) eine Sequenz, die für einen Hefe-selektierbaren Marker oder einen funktional aktiven Abschnitt davon codiert; und
(viii) einen Hefe-Replikationsursprung oder einen funktional aktiven Abschnitt davon.

34. Verfahren zur Herstellung eines Hämoglobins, das eine Globinkette I und eine Globinkette II aufweist, wobei die Globinketten I und II wie in Anspruch 10 definiert sind, wobei das Verfahren die Schritte umfaßt:
(a) Kultivieren einer Hefezelle in einem geeigneten Medium, so daß die Globinkette I und die Globinkette II exprimiert werden, wobei die Hefezelle einen rekombinanten DNA-Vektor I und einen rekombinanten DNA-Vektor II enthält, wobei der rekombinante DNA-Vektor I aufweist:
(i) eine cDNA-Sequenz I, die für die Globinkette I codiert,
(ii) einen Hefe-Transkriptions-Promotor, der stromauf der Sequenz I angeordnet ist, der die Transkription der Sequenz I reguliert,
(iii) eine Sequenz, die für einen Hefe-selektierbaren Marker oder einen funktional aktiven Abschnitt davon codiert, und
(iv) einen Hefe-Replikationsursprung oder einen funktional aktiven Abschnitt davon; und
wobei der rekombinante DNA-Vektor II aufweist:
(v) eine cDNA-Sequenz II, die für die Globinkette II codiert;
(vi) einen Hefe-Transkriptions-Promotor, der stromauf der Sequenz II angeordnet ist, der die Transkription der Sequenz II reguliert,
(vii) eine Sequenz, die für einen Hefe-selektierbaren Marker oder einen funktional aktiven Abschnitt davon codiert, und
(viii) einen Hefe-Replikationsursprung oder einen funktional aktiven Abschnitt davon;
(b) Isolieren der Globinkette I und der Globinkette II; und
(c) Kombinieren der Globinkette I und der Globinkette II mit einer Häm-Quelle, um das Hämoglobin zu bilden.

35. Zusammensetzung nach irgendeinem der Ansprüche 1 bis 9 oder hergestellt nach dem Verfahren nach irgendeinem der Ansprüche 30 bis 34 für ihre Verwendung zur Ergänzung der Sauerstofftransportkapazität des Bluts eines Patienten.

## Revendications

1. Composition comprenant un variant d'hémoglobine,
dans laquelle le variant d'hémoglobine comprend:
(a) une chaîne de variant de globine ou un fragment de celle-ci se fixant à l'hème exprimé dans une levure, qui est substantiellement homologue à:
i) une chaîne de gamma-globine foetale de mammifère ou un fragment de celle-ci se fixant à l'hème.
une chaîne de globine de type alpha ou un fragment de celle-ci se fixant à l'hème exprimé dans une levure, où la chaîne de globine de type alpha est une chaîne de zéta-globine d'embryon de mammifère; et
un hème;
dans laquelle le variant d'hémoglobine
(d) est capable de se lier à l'oxygène à une affinité pour l'oxygène faible;
(e) est capable de s'autopolymériser;
(f) est stable dans un alcali; ou
(g) ne se dissocie pas dans des conditions physiologiques;
ladite composition étant dépourvue de composants de membrane d'érythrocytes et d'endotoxines de E.Coli,
et la chaîne substantiellement homologue de variant de globine ou un fragment de celle-ci se fixant à l'hème, est un variant dans lequel une séquence d'ADNc codant pour la chaîne de variant de globine ou un fragment de celle-ci se fixant à l'hème, est capable de s'hybrider à une séquence d'ADNc codant pour la chaîne de gamma-globine foetal de mammifère ou un fragment de celle-ci se fixant à l'hème, dans des conditions sévères comprenant 0,1X SSC et une température d'environ 65°C.

2. Composition suivant la revendication 1, dans laquelle le variant d'hémoglobine est capable de se lier à l'oxygène à une affinité pour l'oxygène faible.

3. Composition suivant la revendication 2, dans laquelle la chaîne de variant de globine ou un fragment de celle-ci se fixant à l'hème comprend une thréonine en position gamma-66.

4. Composition suivant la revendication 2, dans laquelle la chaîne de variant de globine ou un fragment de celle-ci se fixant à l'hème comprend une valine en position gamma-1.

5. Composition suivant la revendication I, dans laquelle le variant d'hémoglobine est capable de s'autopolymériser.

6. Composition suivant la revendication 5, dans laquelle la chaîne de variant de globine ou un fragment de celle-ci se fixant à l'hème comprend une cystéine en position gamma-9.

7. Composition comprenant un variant d'hémoglobine,
dans laquelle le variant d'hémoglobine comprend:
(a) une chaîne de variant de globine ou un fragment de celle-ci se fixant à l'hème exprimé dans une levure, qui est substantiellement homologue à:
i) une chaîne de zéta-globine d'embryon de mammifère ou un fragment de celle-ci se fixant à l'hème.
(b) une chaîne de globine de type bêta ou un fragment de celle-ci se fixant à l'hème exprimé dans une levure, où la chaîne de globine de type bêta est une chaîne de gamma-globine de foetus de mammifère; et
un hème;
dans laquelle le variant d'hémoglobine:
(d) est capable de se lier à l'oxygène à une affinité pour l'oxygène faible; ou
(e) est stable dans un alcali;
ladite composition étant dépourvue de composants de membrane d'érythrocytes et d'endotoxines de E.Coli,
et la chaîne substantiellement homologue de variant de globine ou un fragment de celle-ci se fixant à l'hème est un variant dans lequel une séquence d'ADNc codant pour la chaîne de variant de globine ou un fragment de celle-ci se fixant à l'hème, est capable de s'hybrider à une séquence d'ADNc codant pour la chaîne de zéta-globine d'embryon de mammifère ou un fragment de celle-ci se fixant à l'hème, dans des conditions sévères comprenant 0,1X SSC et une température d'environ 65°C.

8. Composition suivant la revendication 7, dans laquelle le variant d'hémoglobine est capable de se lier à l'oxygène à une affinité pour l'oxygène faible.

9. Composition suivant la revendication 8, dans laquelle la chaîne de variant de globine ou un fragment de celle-ci se fixant à l'hème comprend une asparagine en position zéta-94.

10. Vecteur d'ADN recombinant capable d'exprimer dans une cellule de levure, une chaîne I de globine et une chaîne II de globine, lequel vecteur comprend:
(a) une séquence I d'ADNc codant pour la chaîne I de globine;
(b) un promoteur transcriptionnel de levure situé en amont de la séquence I qui régule la transcription de la séquence I;
(c) une séquence II d'ADNc codant pour la chaîne II de globine;
(d) un promoteur transcriptionnel de levure situé en amont de la séquence II qui régule la transcription de la séquence II;
(e) une séquence codant pour un marqueur de levure pouvant être sélectionné ou une partie fonctionnellement active de celui-ci; et
(f) une origine de réplication de levure ou une partie fonctionnellement active de celle-ci;
où la chaîne I de globine est:
(i) une chaîne de zéta-globine d'embryon de mammifère ou un fragment de celle-ci se fixant à l'hème ou une chaîne de variant de zéta-globine ou un fragment de celle-ci se fixant à l'hème qui est substantiellement homologue à la chaîne de zéta-globine ou à un fragment de celle-ci se fixant à l'hème, où la chaîne substantiellement homologue de variant de zéta-globine ou un fragment de celle-ci se fixant à l'hème est un variant dans lequel une séquence d'ADN codant pour la chaîne de variant de zéta-globine ou un fragment de celle-ci se fixant à l'hème, est capable de s'hybrider à une séquence d'ADN codant pour la chaîne de zéra-globine ou un fragment de celle-ci se fixant à l'hème, dans des conditions sévères comprenant 0,1 X SSC et une température d'environ 65°C; et
où la chaîne II de globine est:
(ii) une chaîne de bêta-globine de foetus de mammifère ou un fragment de celle-ci se fixant à l'hème ou une chaîne de variant de gamma-globine ou un fragment de celle-ci se fixant à l'hème qui est substantiellement homologue à la chaîne de gamma-globine ou à un fragment de celle-ci se fixant à l'hème, où la chaîne substantiellement homologue de variant de gamma-globine ou un fragment de celle-ci se fixant à l'hème est un variant dans lequel une séquence d'ADN codant pour la chaîne de variant de gamma-globine ou un fragment de celle-ci se fixant à l'hème, est capable de s'hybrider à une séquence d'ADN codant pour la chaîne de gamma-globine ou un fragment de celle-ci se fixant à l'hème, dans des conditions sévères comprenant 0,1 X SSC et une température d'environ 65°C.

11. Vecteur d'ADN recombinant suivant la revendication 10, qui comprend de plus:
(g) une séquence de terminaison de transcription située en aval de la séquence I d'ADNc qui termine la transcription de la séquence I d'ADNc.

12. Vecteur d'ADN recombinant suivant la revendication 10, qui comprend de plus:
(h) un promoteur transcriptionnel de levure situé en amont de la séquence II d'ADNc qui régule la transcription de la séquence II d'ADNc.

13. Vecteur d'ADN recombinant suivant la revendication 12, qui comprend de plus:
(i) une séquence de terminaison de transcription située en aval de la séquence II d'ADNc qui termine la transcription de la séquence II d'ADNc.

14. Vecteur d'ADN recombinant suivant l'une quelconque des revendications 10 à 13, dans lequel le promoteur transcriptionnel de levure est un promoteur de levure pouvant être induit.

15. Vecteur d'ADN recombinant suivant la revendication 14, dans lequel le promoteur de levure pouvant être induit est un promoteur unidirectionnel.

16. Vecteur d'ADN recombinant suivant la revendication 14, dans lequel le promoteur de levure pouvant être induit est un promoteur bidirectionnel.

17. Vecteur d'ADN recombinant suivant l'une quelconque des revendications 10 à 13, dans lequel le promoteur transcriptionnel de levure est un promoteur constitutif de levure.

18. Vecteur d'ADN recombinant suivant l'une quelconque des revendications 10 à 13, dans lequel le promoteur transcriptionnel de levure comprend:
une séquence contenant une région de régulation transcriptionnelle d'un promoteur pouvant être induit; et
une séquence contenant une région d'initiation de transcription d'un promoteur constitutif, la séquence contenant la région de régulation transcriptionnelle d'un promoteur pouvant être induit étant située en amont de la séquence contenant la région d'initiation de transcription d'un promoteur constitutif.

19. Vecteur d'ADN recombinant suivant la revendication 18, dans lequel la région de régulation transcriptionnelle est une séquence d'activation située en amont du promoteur de levure pouvant être induit.

20. Vecteur d'ADN recombinant suivant l'une quelconque des revendications 10 à 13, dans lequel le marqueur de levure pouvant être sélectionné est un gène LEU2.

21. Vecteur d'ADN recombinant suivant l'une quelconque des revendications 10 à 13, dans lequel le marqueur de levure pouvant être sélectionné est un gène leu2d.

22. Vecteur d'ADN recombinant suivant l'une quelconque des revendications 10 à 13, dans lequel le marqueur de levure pouvant être sélectionné est un gène URA3.

23. Vecteur d'ADN recombinant suivant l'une quelconque des revendications 10 à 13, dans lequel l'origine de réplication de la levure est un système de réplication de levure de plasmide 2µ.

24. Vecteur d'ADN recombinant suivant l'une quelconque des revendications 10 à 13, dans lequel l'origine de réplication de la levure est une séquence de réplication autonome.

25. Cellule de levure contenant le vecteur d'ADN recombinant suivant l'une quelconque des revendications 10 à 24.

26. Cellule de levure contenant un vecteur I d'ADN recombinant, capable d'exprimer une chaîne I de globine dans la cellule de levure, et un vecteur II d'ADN recombinant capable d'exprimer une chaîne II de globine dans la cellule de levure, les chaînes I et II de globine étant telles que définies dans la revendication 10, dans laquelle cellule:
(a) le vecteur I d'ADN recombinant exprime la chaîne I de globine dans la cellule de levure et comprend:
(i) un promoteur I de transcription de levure;
(ii) une séquence I d'ADNc qui code pour la chaîne I de globine et est située en aval du promoteur I, où le promoteur I régule la transcription de la séquence I;
(iii) un marqueur I de levure pouvant être sélectionné ou une partie fonctionnellement active de celui-ci;
(iv) une origine I de réplication de levure ou une partie fonctionnellement active de celle-ci;
(v) une séquence I de terminaison de transcription située en aval de la séquence I; et
(b) le vecteur II d'ADN recombinant exprime la chaîne II de globine dans la cellule de levure et comprend:
(i) un promoteur II de transcription de levure;
(ii) une séquence II d'ADNc qui code pour la chaîne II de globine et est située en aval du promoteur II, où le promoteur II régule la transcription de la séquence II;
(iii) un marqueur II de levure pouvant être sélectionné ou une partie fonctionnellement active de celui-ci;
(iv) une origine II de réplication de levure ou une partie fonctionnellement active de celle-ci;
(v) une séquence II de terminaison de transcription située en aval de la séquence II.

27. Cellule de levure suivant la revendication 26, dans laquelle:
la chaîne I de globine est une chaîne de variant de globine de type alpha qui est substantiellement homologue à une chaîne de zéta-globine d'embryon humain et comprend une asparagine en position zéta-94; le promoteur I est un promoteur GAL10; la séquence d'ADN codant pour la chaîne de variant de globine de type alpha est capable de s'hybrider à une séquence d'ADN codant pour la chaîne de zéta-globine d'embryon humain dans des conditions sévères comprenant 0,1 X SSC et une température d'environ 65°C; le marqueur I pouvant être sélectionné est un gène LEU2; l'origine I de réplication est un système de réplication de plasmide 2µ; et la séquence I de terminaison comprend une région de terminaison de transcription d'un gène d'alcool déshydrogénase I; et
la chaîne II de globine est une chaîne de gamma-globine de foetus humain; le promoteur II est un promoteur GAL10; le marqueur II pouvant être sélectionné est un gène LEU2; l'origine de réplication est un système de réplication de plasmide 2µ; et la séquence II de terminaison comprend une région de terminaison de transcription d'un gène d'alcool déshydrogénase I.

28. Cellule de levure suivant la revendication 27, qui est une *Saccharomyces cerevisiae*.

29. Procédé pour la production d'une chaîne I de globine et d'une chaîne II de globine dans une cellule de levure, qui comprend la culture de la cellule de levure dans un milieu approprié tel que la chaîne I de globine et la chaîne II de globine soient exprimées, ladite cellule de levure contenant le vecteur d'ADN recombinant suivant l'une quelconque des revendications 10 à 24,.

30. Procédé pour la production d'une hémoglobine comprenant une chaîne I de globine et une chaîne II de globine, où la chaîne I de globine et la chaîne II de globine sont telles que définies dans la revendication 10, lequel procédé comprend les étapes de:
(a) culture d'une cellule de levure I de façon à ce que la chaîne I de globine soit exprimée, laquelle cellule de levure I contient un vecteur d'ADN recombinant comprenant:
(i) une séquence I codant pour la chaîne I de globine,
(ii) un promoteur transcriptionnel de levure situé en amont de la séquence I qui régule la transcription de la séquence I,
(iii) une séquence codant pour un marqueur de levure pouvant être sélectionné ou une partie fonctionnellement active de celui-ci, et
(iv) une origine de réplication de levure ou une partie fonctionnellement active de celle-ci;
(b) culture d'une cellule de levure II de façon à ce que la chaîne II de globine soit exprimée, laquelle cellule de levure II contient un vecteur d'ADN recombinant comprenant:
(i) une séquence II codant pour la chaîne II de globine,
(ii) un promoteur transcriptionnel de levure situé en amont de la séquence II qui régule la transcription de la séquence II,
(iii) une séquence codant pour un marqueur de levure pouvant être sélectionné ou une partie fonctionnellement active de celui-ci, et
(iv) une origine de réplication de levure ou une partie fonctionnellement active de celle-ci;
(c) isolement de la chaîne I de globine de la cellule de levure I;
(d) isolement de la chaîne II de globine de la cellule de levure II; et
(e) combinaison de la chaîne I de globine isolée et de la chaîne II de globine isolée, avec une source d'hème pour former l'hémoglobine.

31. Procédé pour la production d'une hémoglobine comprenant une chaîne I de globine et une chaîne II de globine, où la chaîne I de globine et la chaîne II de globine sont telles que définies dans la revendication 10, lequel procédé comprend les étapes de:
(a) culture d'une cellule de levure de façon à ce que la chaîne I de globine et la chaîne II de globine soient exprimées, laquelle cellule de levure contient un vecteur d'ADN recombinant qui comprend:
(i) une séquence I d'ADNc codant pour la chaîne I de globine,
(ii) une séquence II d'ADNc codant pour la chaîne II de globine,
(iii) un promoteur transcriptionnel de levure situé en amont de la séquence I qui régule la transcription de la séquence I,
(iv) un promoteur transcriptionnel de levure situé en amont de la séquence II qui régule la transcription de la séquence II,
(v) une séquence codant pour un marqueur de levure pouvant être sélectionné ou une partie fonctionnellement active de celui-ci, et
(vi) une origine de réplication de levure ou une partie fonctionnellement active de celle-ci;
(b) isolement de la chaîne I de globine et de la chaîne II de globine de la cellule de levure; et
(c) combinaison de la chaîne I de globine et de la chaîne II de globine, avec une source d'hème pour former l'hémoglobine.

32. Procédé pour la production dans une cellule de levure d'une hémoglobine comprenant une chaîne I de globine et une chaîne II de globine, où la chaîne I de globine et la chaîne II de globine sont telles que définies dans la revendication 10, lequel procédé comprend:
la culture d'une cellule de levure dans un milieu approprié de façon à ce que la chaîne I de globine et la chaîne II de globine soient exprimées et assemblées ensemble avec l'hème, dans la cellule de levure pour former l'hémoglobine, laquelle cellule de levure contient un vecteur d'ADN recombinant qui comprend:
(i) une séquence I d'ADNc codant pour la chaîne I de globine,
(ii) une séquence II d'ADNc codant pour la chaîne II de globine,
(iii) un promoteur transcriptionnel de levure situé en amont de la séquence I qui régule la transcription de la séquence I,
(iv) un promoteur transcriptionnel de levure situé en amont de la séquence II qui régule la transcription de la séquence II,
(v) une séquence codant pour un marqueur de levure pouvant être sélectionné ou une partie fonctionnellement active de celui-ci, et
(vi) une origine de réplication de levure ou une partie fonctionnellement active de celle-ci.

33. Procédé pour la production dans une cellule de levure d'une hémoglobine comprenant une chaîne I de globine et une chaîne II de globine, la chaîne I de globine et la chaîne II de globine étant telles que définies dans la revendication 10, lequel procédé comprend:
la culture d'une cellule de levure dans un milieu approprié de façon à ce que la chaîne I de globine et la chaîne II de globine soient exprimées et assemblées ensemble avec l'hème dans la cellule de levure pour former l'hémoglobine, laquelle cellule de levure contient un vecteur I d'ADN recombinant et un vecteur II d'ADN recombinant, lequel vecteur I d'ADN recombinant comprend:
(i) une séquence I d'ADNc codant pour la chaîne I de globine,
(ii) un promoteur transcriptionnel de levure situé en amont de la séquence I qui régule la transcription de la séquence I,
(iii) une séquence codant pour un marqueur de levure pouvant être sélectionné ou une partie fonctionnellement active de celui-ci, et
(iv) une origine de réplication de levure ou une partie fonctionnellement active de celle-ci, et
lequel vecteur II d'ADN recombinant comprend:
(v) une séquence II d'ADNc codant pour la chaîne II de globine,
(vi) un promoteur transcriptionnel de levure situé en amont de la séquence II qui régule la transcription de la séquence II,
(vii) une séquence codant pour un marqueur de levure pouvant être sélectionné ou une partie fonctionnellement active de celui-ci, et
(viii) une origine de réplication de levure ou une partie fonctionnellement active de celle-ci.

34. Procédé pour la production d'une hémoglobine comprenant une chaîne I de globine et une chaîne II de globine, la chaîne I de globine et la chaîne II de globine étant telles que définies dans la revendication 10, lequel procédé comprend les étapes de:
(a) culture d'une cellule de levure dans un milieu approprié de façon à ce que la chaîne I de globine et la chaîne II de globine soient exprimées, laquelle cellule de levure contient un vecteur I d'ADN recombinant et un vecteur II d'ADN recombinant, lequel vecteur I d'ADN recombinant comprend:
(i) une séquence I d'ADNc codant pour la chaîne I de globine,
(ii) un promoteur transcriptionnel de levure situé en amont de la séquence I qui régule la transcription de la séquence I,
(iii) une séquence codant pour un marqueur de levure pouvant être sélectionné ou une partie fonctionnellement active de celui-ci, et
(iv) une origine de réplication de levure ou une partie fonctionnellement active de celle-ci, et
lequel vecteur II d'ADN recombinant comprend:
(v) une séquence II d'ADNc codant pour la chaîne II de globine,
(vi) un promoteur transcriptionnel de levure situé en amont de la séquence II qui régule la transcription de la séquence II,
(vii) une séquence codant pour un marqueur de levure pouvant être sélectionné ou une partie fonctionnellement active de celui-ci, et
(viii) une origine de réplication de levure ou une partie fonctionnellement active de celle-ci.
(b) isolement de la chaîne I de globine et de la chaîne II de globine; et
(c) combinaison de la chaîne I de globine et de la chaîne II de globine avec une source d'hème pour former l'hémoglobine.

35. La composition suivant l'une quelconque des revendications 1 à 9, ou produite selon le procédé suivant l'une quelconque des revendications 30 à 34 pour être utilisée pour augmenter la capacité de transport d'oxygène du sang d'un patient.
